# EUROPEAN PATENT APPLICATION

(11) **EP 1 074 618 A2**
(43) Date of publication of application: **07.02.2001**
(21) Application number: 00118520.6
(22) Date of filing: 19.04.1993
(51) Int. Cl.: C12N 15/12, C07K 14/705, C07K 16/28

(54) **Cadherin materials and methods**

(30) Priority: 17.04.1992 US 872643
(62) Divisional of application: 93910658.9
(71) Applicant: DOHENY EYE INSTITUTE, Los Angeles, CA 90033 (US)
(72) Inventor: SUZUKI, Shintaro, Torrance, CA 90505 (US)
(74) Representative: Brown, John David

(57) **Abstract**

DNA sequences encoding novel cadherins, designated cadherins-4 through -12, are disclosed along with methods and materials for the recombinant production of the same. Antibody substances specific for the novel cadherins and cadherin peptides are disclosed as useful for modulating the natural binding and/or regulatory activities of the cadherins.

## Description

This application is a continuation-in-part of U.S. Patent Application Serial No. 07/872,643 filed on April 17, 1992.

### FIELD OF THE INVENTION

The present invention relates, in general, to materials and methods relevant to cell-cell adhesion. More particularly, the invention relates to novel Ca²⁺-dependent cell adhesion proteins, referred to as cadherins, and to polynucleotide sequences encoding the cadherins. The invention also relates to methods for inhibiting binding of the cadherins to their natural ligands/antiligands.

### BACKGROUND

*In vivo*, cell-cell adhesion plays an important role in a wide range of events including morphogenesis and organ formation, leukocyte extravasion, tumor metastasis and invasion, and the formation of cell junctions. Additionally, cell-cell adhesion is crucial for the maintenance of tissue integrity, e.g., of the intestinal epithelial barrier, of the blood brain barrier and of cardiac muscle.

Intercellular adhesion is mediated by specific cell adhesion molecules. Cell adhesion molecules have been classified into at least three superfamilies including the immunoglobulin (Ig) superfamily, the integrin superfamily and the cadherin superfamily. All cell types that form solid tissues express some members of the cadherin superfamily suggesting that cadherins are involved in selective adhesion of most cell types.

Cadherins have been generally described as glycosylated integral membrane proteins that have an N-terminal extracellular domain that determines binding specificity (the N-terminal 113 amino acids appear to be directly involved in binding), a hydrophobic membrane-spanning domain and a C-terminal cytoplasmic domain (highly conserved among the members of the superfamily) that interacts with the cytoskeleton through catenins and other cytoskeleton-associated proteins. Some cadherins lack a cytoplasmic domain, however, and appear to function in cell-cell adhesion by a different mechanism than cadherins that have a cytoplasmic domain. The cytoplasmic domain is required for the binding function of the extracellular domain in cadherins that do have a cytoplasmic domain. Binding between members of the cadherin family expressed on different cells is mainly homophilic (i.e., a member of the cadherin family binds to cadhering of its own or a closely related subclass) and Ca²⁺-dependent. For recent reviews on cadherins, see Takeichi, *Annu. Rev. Biochem., 59*:237-252 (1990) and Takeichi, *Science, 251,* 1451-1455 (1991).

The first cadherins to be described (E-cadherin in mouse epithelial cells, L-CAM in avian liver, uvomorulin in the mouse blastocyst, and CAM 120/80 in human epithelial cells) were identified by their involvement in Ca²⁺-dependent cell adhesion and by their unique immunological characteristics and tissue localization. With the later immunological identification of N-cadherin, which was found to have a different tissue distribution from E-cadherin, it became apparent that a new family of Ca²⁺-dependent cell-cell adhesion molecules had been discovered.

The molecular cloning of the genes encoding mouse E- [see Nagafuchi *et al., Nature, 329*: 341-343 (1987)], chicken N- [Hatta *et al*., *J.Cell Biol*., *106*: 873-881 (1988)], and mouse P-[Nose *et al*., *EMBO J. 6*; 3655-3661 (1987)] cadherins provided structural evidence that the cadherins comprised a family of cell adhesion molecules. Cloning of chicken L-CAM [Gallin *et al*., *Proc. Natl. Acad. Sci. USA, 84*: 2808-2812 (1987)] and mouse uvomorulin [Ringwald *et al*., *EMBO I.*, *6*; 3647-3653 (1987)] revealed that they were identical to E-cadherin. Comparisons of the amino acid sequences of E-, N-, and P-cadherins showed a level of amino similarity of about 45%-58% among the three subclasses. Liaw *et al.*, *EMBO J*., *9*: 2701-2708 (1990) describes the use of PCR with degenerate oligonucleotides based on one conserved region of E-, N-and P-cadherins to isolate N- and P-cadherin from a bovine microvascular endodthelial cell cDNA. The Liaw et al., *supra,* results implied that there were only E-, N-, and P-cadherins because no new cadherins were identified. Also in 1990, it was imported in Heimark *et al*., *J. Cell Biol., 110*: 1745-1756 (1990) that an antibody generated to bovine aortic endothelial cells recognized an intercellular junctional molecule designated V-cadherin which had a similar molecular weight to known cadherins and was able to inhibit Ca²⁺-dependent cell endothelial cell adhesion. The article did not disclose any sequence information for the protein recognized by the antibody.

No further cadherin genes were described until the identification of eight of the novel cadherins claimed herein was reported in Suzuki *et al., Cell Regulation, 2*: 261-270 (1991). Subsequently, several other cadherins were described including chicken R-cadherin [Inuzuka *et al., Neuron, 7*: 69-79 (1991)], mouse M-cadherin [Donalies *et al., Proc. Natl. Acad. Sci. USA, 88*: 8024-8028 (1991)], chicken B-cadherin [Napolitano *et al., J. Cell. Biol., 113*: 893-905 (1991)], and T-cadherin [chicken in Ranscht *et al., Neuron, 7*: 391-402 (1991) and chicken and human in Patent Cooperation Treaty (PCT) International Publication No. WO 92/08731 published on May 29, 1992].

The determination of the tissue expression of the various cadherins reveals that each subclass of cadherins has a unique tissue distribution pattern. For example, E-cadherin is found in epithelial tissues while N-cadherin is found in nonepithelial tissues such as neural and muscle tissue. The unique expression pattern of the different cadherins is particularly significant when the role each subclass of cadherins may play *in vivo* in normal events (e.g., the maintenance of the intestinal epithelial barrier) and in abnormal events (e.g., tumor metastatis or inflammation) is considered. Supression of cadherin function has been implicated in the progression of various cancers. See Shimoyama *et al., Cancer Res., 52*: 5770-5774 (1992). Different subclasses or combinations of subclasses of cadherins are likely to be responsible for different cell-cell adhesion events in which therapeutic detection and/or intervention may be desirable. Studies have also suggested that cadherins may have some regulatory activity in addition to adhesive activity. Matsunaga *et al, Nature, 334,* 62-64 (1988) reports that N-cadherin has neurite outgrowth promoting activity and Mahoney *et al., Cell, 67,* 853-868 (1991) reports that the Drosophila *fat* tumor supressor gene, another member of the cadherin superfamily, appear to regulate cell growth. Expression of the cytoplasmic domain of N-cadherin without its extracellular domain has been shown in Kintner *et al., Cell, 69*: 229-236 (1992) to disrupt embryonic cell adhesion and in Fugimori *et al., Mol. Biol. Cell, 4*: 37-47 (1993) to disrupt epithial cell adhesion. Thus, therapeutic intervention in the regulatory activities of cadherins expressed in specific tissues may also be desirable.

There thus continues to exist a need in the art for the identification and characterization of additional cadherins participating in cell-cell adhesion and/or regulatory events. Moreover, to the extent that cadherins might form the basis for the development of therapeutic and diagnostic agents, it is essential that the genes encoding the proteins be cloned. Information about the DNA sequences and amino acid sequences encoding the cadherins would provide for the large scale production of the proteins and for the identification of the cells/tissues naturally producing the proteins, and would permit the preparation of antibody substances or other novel binding molecules specifically reactive with the cadherins that may be useful in modulating the natural ligand/antiligand binding reactions in which the cadherins are involved.

### SUMMARY OF THE INVENTION

The present invention provides materials and methods that are relevant to cell-cell adhesion. In one of its aspects, the present invention provides purified and isolated polynucleotide sequences (e.g., DNA and RNA, both sense and antisense strands) encoding novel cadherins, cadherin-4 through -12. Preferred polynucleotide sequences of the invention include genomic and cDNA sequences as well as wholly or partially synthesized DNA sequences, and biological replicas thereof (i.e., copies of purified and isolated DNA sequences made *in vivo* or *in vitro* using biological reagents). Biologically active vectors comprising the polynucleotide sequences are also contemplated.

The scientific value of the information contributed through the disclosures of the DNA and amino acid sequences of the present invention is manifest. For example, knowledge of the sequence of a cDNA encoding a cadherin makes possible the isolation by DNA/DNA hybridization of genomic DNA sequences that encode the protein and that specify cadherin-specific expression regulating sequences such as promoters, enhancers and the like. DNA/DNA hybridization procedures utilizing the DNA sequences of the present invention also allow the isolation of DNAs encoding heterologous species proteins homologous to the rat and human cadherins specifically illustrated herein.

According to another aspect of the invention, host cells, especially eucaryotic and procaryotic cells, are stably transformed or transfected with the polynucleotide sequences of the invention in a manner allowing the expression of cadherin polypeptides in the cells. Host cells expressing cadherin polypeptide products, when grown in a suitable culture medium, are particularly useful for the large scale production of cadherin polypeptides, fragments and variants; thereby enabling the isolation of the desired polypeptide products from the cells or from the medium in which the cells are grown.

The novel cadherin proteins, fragments and variants of the invention may be obtained as isolates from natural tissue sources, but are preferably produced by recombinant procedures involving the host cells of the invention. The products may be obtained in fully or partially glycosylated, partially or wholly de-glycosylated or non-glycosylated forms, depending on the host cell selected or recombinant production and/or post-isolation processing.

Cadherin variants according to the invention may comprise polypeptide analogs wherein one or more of the specified (i.e., naturally encoded) amino acids is deleted or replaced or wherein one or more nonspecified amino acids are added: (1) without loss, and preferably with enhancement, of one or more of the biological activities or immunological characteristics specific for a cadherin; or (2) with specific disablement of a particular ligand/antiligand binding function of a cadherin.

Also contemplated by the present invention are antibody substances [e.g., monoclonal and polyclonal antibodies, chimeric and humanized antibodies, and antibody domains including Fab, Fab' and F(ab')₂, single chain antibodies, and Fv or single variable domains] and other binding proteins or peptides specifically react with cadherins of the invention. Antibody substances can be developed using isolated natural, recombinant or synthetic cadherin polypeptide products or host cells expressing such products on their surfaces. The antibody substances may be utilized for purifying polypeptides of the invention, for determining the tissue expression of the polypeptides and as antagonists of the ligand/antiligand binding activities of the cadherins. Specifically illustrating antibody substances of the invention are the monoclonal antibodies produced by the hybridomas designated 30Q8A, 30Q4H, 45A5G, 30S2F and 45C6A which were all deposited with the American Type Culture Collection (ATCC), 12301 Parklawn Drive, Rockvilie, Maryland 20852 on April 6, 1993 and were respectively assigned ATCC Deposit Nos. HB11316, HB11317, HB11318, HB11319 and HB11320. Also illustrating antibody substances of the invention is the monoclonal antibody produced by the hybridoma designated 30T11G which was deposited with the ATCC on April 8, 1993 and was assigned ATCC Deposit No. HB11324.

The DNA and amino acid sequence information provided by the present invention makes possible the systematic analysis of the structure and function of the cadherins described herein and definition of those molecules with which the cadherins will interact on extracellular and intracellular levels. The idiotypes of anti-cadherin monoclonal antibodies of the invention are representative of such molecules and may mimic natural binding proteins (peptides and polypeptides) through which the intercellular and intracellular activities of cadherins are modulated. Alternately, they may represent new classes of modulators of cadherin activities. Anti-idiotypic antibodies, in turn, may represent new classes of biologically active cadherin equivalents.

Methods for modulating cadherin activity may involve contacting a cadherin with an antibody (or antibody fragment), another polypeptide or peptide ligand (including peptides derived from cadherins or other proteins, or a novel peptide), or a small molecule ligand that specifically binds to a portion (extracellular or cytoplasmic) of the cadherin.

Numerous aspects and advantages of the present invention will be apparent upon consideration of the following detailed description thereof, reference being made to the drawing wherein:

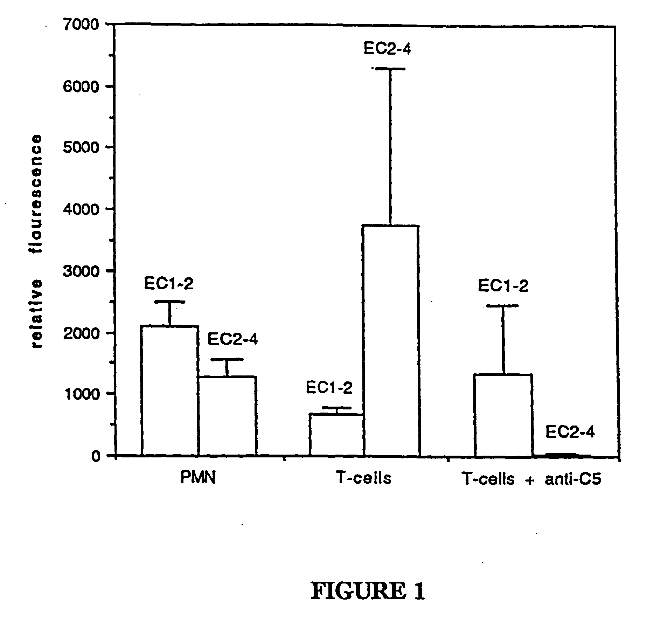

FIGURE 1 is a bar graph illustrating the binding of polymorphonuclear neutrophils and T cells to fusion proteins comprising extracellular subdomains of cadherin-5.

### DETAILED DESCRIPTION

The present invention is illustrated by the following examples wherein Example 1 describes the isolation of cDNA sequences encoding rat cadherins-4 through -11 and -13; Example 2 describes the isolation of cDNA sequences encoding the human homologs of rat cadherins-4, -5, -6, -8, -10, -11 and -13 and the isolation of a human cadherin not identified in rat, cadherin-12; Example 3 characterizes the relationship of cadherins of the invention to previously identified cadherins in terms of amino acid sequence and structure. The generation of polyclonal and monoclonal antibodies specific for cadherins of the invention is described in Example 4. Example 5 describes the construction of expression constructs comprising cadherin-4, -5 and -8 sequences, transfection of mammalian cells with the constructs and results of cell-cell adhesion assays performed with the transfected cells. Example 6 presents the results of assays for cadherin mRNA and protein expression in various mammalian tissues, cells and cell lines. The results of *in vitro* transendothelial migration assays involving cadherin-5 and assays of neutrophil and T-cell binding to cadherin-5 fusion protein are described in Example 7. Example 8 describes expression of cadherin-5 in the blood-brain barrier and Example 9 describes cadherin-5 peptides that are capable of increasing endothelim permeability. Example 10 describes the association of the cytoplasmic domain of cadherin-5 with plakoglobin. The disclosures of Suzuki *et al., Cell Regulation, supra*; Suzuki *et al., J. Cell. Biol*., 115, Abstract 72a (1991); Suzuki *et al*., *Cell. Struc. Funct., 16,* 605 (1991); and Tanihara *et al., Invest. Ophthalmol. Vis. Sci.,* 32, 1013 (1991) are incorporated by reference herein for purposes of illustrating the background of the invention.

### Example 1

Partial cDNA clones encoding nine novel cadherins were isolated from rat brain and retina by PCR. Eight of the novel rat cadherin cDNAs were isolated using degenerate PCR primers based on highly conserved regions of the cytoplasmic domain of known cadherins and one was isolated using degenerate PCR primers based on moderately conserved regions of the extracellular domain of known cadherins.

### A. Preparation of Rat cDNA

Total RNAs were prepared from rat brain by the guanidium isothiocyanate/cesium chloride method described in Maniatis *et al.,* pp. 196 in *Molecular Cloning: A Laboratory Manual,* Cold Spring Harbor, New York: Cold Spring Harbor Laboratory (1982). Brain poly(A)⁺ RNAs were then isolated using an Invitrogen (San Diego, CA) FastTrack kit. Rat retina poly(A)⁺ RNA was purchased from Clonetech (Palo Alto, CA). cDNA was synthesized from the poly(A)⁺ RNA of both rat brain and retina using a cDNA synthesis kit (Boehringer Mannheim Corporation, Indianopolis, IN).

### B. Design and Synthesis of PCR Primers Corresponding to Cadherin Cytoplasmic Domain

A first pair of degenerate oligonucleotide primers, listed below in IUPAC nomenclature, was designed to correspond to highly conserved sequences in the cytoplasmic domain of mouse N-, E-, and P-cadherins. Underlined sequences at the end of each oligonucleotide indicate an *Eco*R1 site added to the primers to facilitate cloning of the fragments generated by PCR.
Degenerate Primer 1
   **TAPPYD** (SEQ ID NO: 1)
   5' GAATTCACNGCNCCNCCNTAYGA 3' (SEQ ID NO: 2)
Degenerate Primer 2
   **FKKLAD** (SEQ ID NO: 3)
   3' AARTTYTTYRANCGNCTCTTAAG 5' (SEQ ID NO: 4)
The degenerate oligonucleotides were synthesized using the Applied Biosystems model 380B DNA synthesizer (Foster City, CA).

### C. Design and Synthesis of PCR Primers Corresponding to Cadherin Extracellular Domain

A second pair of degenerate oligonucleotide primers, listed below in IUPAC nomenclature, was designed to correspond to moderately conserved sequences in the third subdomain of the extracellular domain of mouse N-, E-, and P-cadherins. The extracellullar domains of the mouse N-, E- and P-cadherins have been characterized as having five internal subdomains, some of which may be involved in cadherin interaction with Ca²⁺. Underlined sequences at the end of each oligonucleotide indicate an *Eco*R1 site added to the primers, to facilitate cloning of the fragments generated by PCR.
Degenerate Primer 3
   **K(P/G)(L/I/V)D(F/Y)E** (SEQ ID NO: 5)
   5' GAATTCAARSSNNTNGAYTWYGA 3' (SEQ ID NO: 6)
Degerenate Primer 4
   **(N/D)E(A/P)PXF** (SEQ ID NO: 7)
   3' TRCTYSGNGGNNNNAARCTTAAG 5' (SEQ ID NO: 8)

### D. Cloning of cDNA Encoding Eight Novel Rat Cadherins

PCR amplification reactions of rat brain and retina cDNA were carried out either with degenerate primers 1 and 2 or with degenerate primers 3 and 4 under conditions essentially the same as those described in Saiki *et al., Science, 239*, 487-491 (1988). Briefly, 100 ng of brain or retina first strand cDNA was used as template for amplification by Taq DNA polymerase (International Bioltechnology, New Haven, CT) using 10 µg of each primer set per reaction. PCR reactions were initiated by adding 2 units of Taq DNA polymerase to the reaction solution, after which 35 PCR reaction cycles were carried out. Reaction cycles consisted of denaturation performed at 94°C for 1.5 minutes, oligonucleotide annealing at 45°C for 2 minutes, and elongation at 72°C for 3 minutes. The resulting PCR fragments were separated by agarose gel electrophoresis, and DNA bands of the expected size were extracted from the gel and digested with *Eco*R1. The fragments were then cloned into the M13 vector (Boehringer Mannheim Corp., Indianapolis, IN) and *E. coli* JM101 cells were transformed with the resulting constructs. Individual clones were then isolated and sequenced. Sequencing of the DNAs was carried out using a sequenase kit (United States Biochemicals, Cleveland, OH) and the resulting DNA and deduced amino acid, sequences of the clones were compared to sequences of known cadherins using the Microgenie program (Beckman, Fullerton, CA).

Ten representative cDNA clones encoding cadherins were identified from the PCR reaction based on degenerate primers 1 and 2. Two clones corresponded to rat N-, and E-cadherins, but eight clones encoded previously undescribed cadherins, and were designated cadherins-4 through -11. The DNA and deduced amino acid sequences of the eight rat cytoplasmic domain cDNA clones are respectively set out in SEQ ID NOs: 9 and 10 (cadherin-4), SEQ ID NOs: 11 and 12 (cadherin-5), SEQ ID NOs: 13 and 14 (cadherin-6), SEQ ID NOs: 15 and 16 (cadherin-7), SEQ ID NOs: 17 and 18 (cadherin-8), SEQ ID NOs: 19 and 20 (cadherin-9), SEQ ID NOs: 21 and 22 (cadherin-10) and SEQ ID NOs: 23 and 24 (cadherin-11).

An additional novel cadherin was identified from the PCR reaction based on degenerate primers 3 and 4, and it was designated cadherin-13. The DNA and deduced amino acid sequences of the rat cadherin-13 fragment are respectively set out in SEQ ID NOs: 25 and 26.

The PCR reaction based on degenerate primers 3 and 4 also amplified sequences which were later determined to be fragments of the extracellular domains of rat cadherins-4, -5, -6, -8, -9, -10, -11 and -13. The DNA and amino acid sequences of these extracellular fragments are respectively set out in SEQ ID NOs: 27 and 28 (cadherin-4), SEQ ID NOs: 29 and 30 (cadherin-6), SEQ ID NOs: 31 and 32 (cadherin-8), SEQ ID NOs: 33 and 34 (cadherin-9), SEQ ID NOs: 35 and 36 (cadherin-10), SEQ ID NOs: 37 and 38 (cadherin-11), SEQ ID NOs: 39 and 40 (cadherin-13).

Larger cadherin-8 and -10 cDNAs were isolated from a rat brain cDNA library made in Uni-ZAP vector (Stratagene, La Jolla, CA) using labelled cadherin-8 extracellular domain PCR fragment (SEQ ID NO: 17) or cadherin-10 extracellular domain fragment (SEQ ID NO: 21) as probes. Two types of cadherin-8 cDNA clones were isolated. The first type encodes a full length cadherin, but the second type encodes a truncated protein the sequence of which diverges from the first type of cadherin-8 clone near the N-terminus of the fifth extracellular subdomain (EC5). The truncated clone contains a short stretch of unique sequence in the N-terminus of EC5 but lacks the remainder of EC5, the transmembrane domain and the cytoplasmic domain. DNA and deduced amino acid sequences of the full length clone are respectively set out in SEQ ID NOs: 41 and 42 and the DNA and deducted amino acid sequences of the truncated cadherin-8 clone are set out in SEQ ID NOs: 43 and 44. The cadherin-10 cDNA clone that was isolated has an open reading frame which begins at a region corrsponding to the middle of the first extracellular domain (EC1) of previously identified cadherins. The DNA and deduced amino acid sequences of the cadherin-10 clone are set out in SEQ ID NOs: 45 and 46.

### Example 2

Full length cDNAs encoding human homologs of rat cadherins-4, -8, -11 and -13 and partial cDNAs encoding human homologs of rat cadherins-6 and -10 were isolated from a human fetal brain cDNA library (λZapII vector, Stratagene). A full length cDNA encoding a human homolog of rat cadherin-5 was isolated from a human placental cDNA library (λgt11 vector, Dr. Millan, La Jolla Cancer Research Foundation, La Jolla, CA).

Probes for screening the human fetal brain and placental cDNA libraries were amplified by PCR from human brain cDNA (Dr. Taketani, Kansain Medical University, Moriguchi, Osaka, Japan) using the primers described in Example 1B-C. Probes consisting of human cadherin-4, -5, -6, -8, -10 and -11 sequences were generated using degenerate primers 1 and 2 and probes consisting of human cadherin-13 sequence were generated using degenerate primers 3 and 4. Amplification of the human fetal brain cDNA with degenerate primers 3 and 4 also generated a PCR fragment encoding a cadherin not isolated from rat, designated cadherin-12.

PCR fragments encoding human cadherins-4, -5, -6, -8, -10, -11, -12 and -13 were labeled with ³²P and used to probe the human fetal brain and placental cDNA libraries according to the plaque hybridization method described in Ausubel et al., Eds., *Current Protocols in Molecular Biology,* Sections 6.1.1 to 6.1.4 and 6.2.1 to 6.2.3, John Wiley & Sons, New York (1987). Positives were plaque-purified and inserts were cut out using an *in vivo* excision method. The inserts were then subcloned into the M13 vector (Boehringer Mannheim) for sequencing.

Inserts consisting of full length cDNAs encoding human homologs of rat cadherins-4, -8, -11, -12 (putative) and -13 and partial cDNAs encoding human homologs of rat cadherins-6 and -10 were identified in clones from the human fetal brain cDNA library and a full length cDNA encoding a human homolog of rat cadherin-5 was identified in a clone from the human placental cDNA library. The DNA and deduced amino acid sequences of the human homologs are respectively set out in SEQ ID NOs: 47 and 48 (cadherin-4), SEQ ID NOs: 49 and 50 (cadherin-5), SEQ ID NOs: 51 and 52 (cadherin-6), SEQ ID NOs: 53 and 54 (cadherin-8), SEQ ID NOs: 55 and 56 (cadherin-10), SEQ ID NOs: 57 and 58 (cadherin-11), SEQ ID NOs: 59 and 60 (cadherin-12), and SEQ ID NOs: 61 and 62 (cadherin-13).

### Example 3

Comparison of the full-length sequences of the novel human cadherins described in Examples 1 and 2 with sequences of previously described cadherins and cadherin-related proteins provides support for the proposal that cadherins can be divided into at least three subgroups based on amino acid sequence identity and/or domain structure. Identity values for one possible alignment of the sequences of the extracellular domains of selected human cadherins are presented in Table 1 below.

**Table 1**

| | N | E | P | 4 | 5 | 8 | 11 | 12 | 13 |
|---|---|---|---|---|---|---|---|---|---|
| N | 100 | 45 | 45 | 68 | 30 | 34 | 35 | 33 | 46 |
| E | 45 | 100 | 53 | 41 | 29 | 30 | 29 | 31 | 37 |
| P | 45 | 53 | 100 | 29 | 30 | 29 | 31 | 31 | 38 |
| 4 | 68 | 41 | 41 | 100 | 29 | 33 | 34 | 33 | 44 |
| 5 | 30 | 29 | 30 | 29 | 100 | 40 | 41 | 39 | 32 |
| 8 | 34 | 30 | 29 | 33 | 40 | 100 | 66 | 58 | 32 |
| 11 | 35 | 29 | 31 | 34 | 41 | 66 | 100 | 58 | 31 |
| 12 | 33 | 31 | 31 | 33 | 39 | 58 | 58 | 100 | 33 |
| 13 | 46 | 37 | 38 | 44 | 32 | 32 | 31 | 33 | 100 |

Based on such sequence alignments and on the fact that certain combinations of cadherin sequences seem to have conserved stretches of amino acids when aligned, one subgroup of cadherins may include E-cadherin, N-cadherin, P-cadherin and cadherin-4, while a second subgroup may include cadherin-5, cadherin-8, cadherin-11 and cadherin-12. Cadherins-6, -7, -9 and -10 may also be included with the second subgroup based on their partial amino acid sequences disclosed herein. The amino acid sequence of cadherin-4 exhibits especially high amino acid sequence identity with that of R-cadherin (92%), indicating that cadherin-4 may be the human homolog of chicken R-cadherin. All cadherins in these two subgroups have a similar structure. Following an initiation codon, each has a signal sequence, prosequence, proteolytic cleavage site of precursor protein, an extracellular domain (which comprises five subdomains EC1-5), a transmembrane sequence and a cytoplasmic domain. For cadherin-5, these sequences/domains appear to correspond to about the following amino acid positions of SEQ ID NO: 50: 1-24 (signal sequence), 25-43 (prosequence), 44-147 (EC1), 148-254 (EC2), 255-368 (EC3), 369-475(EC4), 476-589 (EC5), 590-616 (transmembrane sequence) and 617-780 (cytoplasmic domain).

Cadherin-13, T-cadherin and V-cadherin may be representative of a third subgroup of cadherins. Cadherin-13 consists of a cadherin-like extracellular domain, but has no domains that would correspond to the typical transmembrane or cytoplasmic domains of other cadherins. Even though about 10% of the clones obtained by PCR using degenerate primers 3 and 4 were cadherin-13 clones, none of the clones included sequences corresponding to a cytoplasmic domain. An attempt to isolate a cDNA that contained this region by PCR using a primer corresponding to the most C-terminal region of cadherin-13 available and a mixed oligonucleotide primer corresponding to a well-conserved amino acid sequence of the cytoplasmic domain of cadherins failed to generate any product with the anticipated molecular weight. A similar protein, T-cadherin, has been identified in chicken which also lacks the typical cadherin cytoplasmic domain. The amino acid sequence identity between the two molecules is about 80%. Cadherin-13 may be the human homologue of chicken T-cadherin or may be a closely related molecule. Human cadherin-13 and avian T-cadherin may also both be closely related to V-cadherin. A 29-amino acid amino terminal sequence of bovine V-cadherin is similar to the start of the precursor region of cadherin-13 (93%) and T-cadherin (79%). V-cadherin is a 135 KD protein which appears to be restricted in tissue distribution to endothelium. In constrast, mature T-cadherin has a molecular weight of 95 KD and shows a wide tissue distribution. Both V-cadherin and T-cadherin are linked to the cell membrane through phosphoinositiol.

### Example 4

Polyclonal and/or monoclonal antibodies specific for cadherins of the invention were generated.

### A. Generation of Polyclonal Antibodies

Bacterial fusion proteins consisting of maltose binding protein fused to portions of cadherin extracellular subdomains (either human cadherin-4, -5 or -11, or rat cadherin-8) were generated and subsequently used for the generation of polyclonal antibodies.

A cDNA fragment corresponding to a 40 KD portion of the extracellular domain of human cadherin-5 (nucleotides 535 to 1527 of SEQ ID NO: 49) was synthesized by PCR from the full-length human cadherin-5 cDNA described in Example 2. The fragment was subcloned into the multicloning site (*Eco*R1-*Xba*I) of the pMAL-RI plasmid vector [New England Biolabs Inc. (NEB), Beverly, MA]. The resulting construct encodes maltose binding protein fused to the extracellular domain of cadherin-5. Constructs encoding maltose binding protein fused to the three N-terminal subdomains of human cadherin-4, rat cadherin-8 and human cadherin-11 were generated by similar methods.

*E. coli* NM522 cells (Stratagene) were then transformed with one of the fusion protein constructs and grown in quantity. After disruption of *E. coli* cells, the individual fusion proteins were purified by affinity column chromatography using amylose resin (NEB) according to the instructions of the manufacturer. When subjected to SDS-PAGE, the purified fusion proteins each showed essentially one band of the expected size.

A total of five hundred µg of a fusion protein in Freund's complete adjuvant was injected into rabbits at four subcutaneous sites. Subsequent injections were carried out at three week intervals using 100 µg of the fusion protein in Freund's incomplete adjuvant also at four subcutaneous sites. The resulting polyclonal sera generated from immunization of rabbits with cadherin-4, -5 or -8 fusion protein were collected and tested for specificity on L cells transfected with the appropriate cadherin sequence (see Example 5). Polyclonal serum generated from immunization of rabbits with cadherin-11 was also collected.

Immunoblotting of various cell types showed that the The anti-cadherin-4 polyclonal serum reacts with protein of about 130 KD in L cells transfected with full length cadherin-4 cDNA and in rat brain. Cadherin-5-specific serum reacts with a protein of about 135 KD in L cells transfected with a full length cadherin-5 DNA and with a protein of about 135 KD in human umbilical vein endothelial cells (HUVEcs). The serum does not react with MDCK cells that expressed high levels of E-cadherin. In bovine aortic endothelial cells, the anti-cadherin-5 serum reacts with a protein of about 120 KD. Additionally, the anti-cadherin-5 serum reacts with a protein which has the same molecular weight in rat brain enclothelial cells in culture. The cadherin-8 polyclonal antibody detected a strong band of about 90 KD and a weak band of about 130 KD in rat brain.

### B. Generation of Monoclonal Antibodies Specific for Human Cadherin-5

Monoclonal antibodies to cadherin-5 were prepared using bacterial fusion proteins containing subdomains of the extracellular domain of human cadherin-5 as immunogens. The fusion proteins prepared included maltose binding protein and the extracellular subdomains 1-2 (EC1-2) or extracellular subdomains 2-4 (EC2-4) of cadherin-5 in the bacterial expression vector pMAL (NEB). The two fusion proteins were expressed in bacteria and purified on amylose-sepharose as described in foregoing section on generation of polyclonal antibodies. The purified fusion proteins were used separately to immunize mice at two subcutaneous sites (100 µg of fusion protein per mouse in Freund's complete adjuvant). The mice then were subcutaneously immunized with Freund's incomplete adjuvant.

The spleen from each mouse was removed sterility and treated in the same manner. Briefly, a single-cell suspension was formed by grinding the spleen between the frosted ends of two glass microscope slides submerged in serum free RPMI 1640 supplemented with 2 mM L-glutamine, 1 mM sodium pyruvate, 100 units/ml penicillin and 100 mg/ml streptomycin (RPMI) (Gibco, Canada). The cell suspension was filtered through a sterile 70-mesh cell strainer, and washed twice by centrifuging at 200 g for 5 minutes and resuspending the pellet in 20 ml serum free RPMI. Thymocytes taken from 3 naive Balb/c mice were prepared in a similar manner. NS-1 myeloma cells, kept in log phase in RPMI with 11% fetal bovine serum (FBS) (Hyclone Laboratories, Inc., Logan, UT) for three days prior to fusion, were centrifuged at 200 g for 5 minutes, and the pellet was washed twice as described for the mouse spleen cells.

After washing, the spleen cells and myeloma cells were brought to a final volume of 10 ml in serum free RPMI, and 10 µl of that final volume was diluted 1:100 in serum free RPMI. Twenty µl of each dilution was removed, mixed with 20 µl 0.4% trypan blue stain in 0.85% saline, loaded onto a hemacytometer and counted. Two x 10⁸ spleen cells were combined with 4 x 10⁷ NS-1 cells, centrifuged and the supernatant was aspirated. The cell pellets were dislodged by tapping the tube and 2 ml of 37°C PEG 1500 (50% in 75 mM Hepes, pH 8.0) (Boehringer Mannheim) was added with stirring over the course of 1 minute, followed by adding 14 ml of serum free RPMI over 7 minutes. An additional 16 ml RPMI was added and the cells were centrifuged at 200 g for 10 minutes. After discarding the supernatant, the pellet was resuspended in 200 ml RPMI containing 15% FBS, 100 mM sodium hypoxanthine, 0.4 mM aminopterin, 16 mM thymidine (HAT) (Gibco), 25 units/ml IL-6 (Boehringer Mannheim) and 1.5 x 10⁶ thymocytes/ml (plating medium). The suspension was dispensed into ten 96-well flat bottom tissue culture plates at 200 ml/well. Cells in plates were fed on days 2, 4, and 6 days post-fusion by aspirating approximately 100 ml from each well with an 18 G needle, and adding 100 ml/well plating medium described above except containing 10 units/ml IL-6 and lacking thymocytes.

Fusions 30 (from a mouse immunized with EC2-4) and 45 (from a mouse immunized with EC1-2) were screened initially by antibody capture ELISA, testing for presence of mouse IgG. Secondary screening of fusions 30 and 45 consisted of assays using plates coated with a monolayer of fixed endothelial cells for ELISAs. HUVEcs, Lewis rat brain endothelial cells (LeBCE), and bovine aortic endothelial cells (BAE) were allowed to grow in 96-well flat bottom tissue culture microtiter plates until the bottom of well was completely covered with a monolayer of cells. Plates were washed twice with 100 µl/well of Ca²⁺/Mg²⁺ free PBS (CMF-PBS) and aspirated completely. Cells were then fixed with 100 µl/well of 3% ρ-Formaldehyde, 1% Sucrose in CMF-PBS at room temperature for 30 minutes. Cells were then permeablized with approximately 250 µl/well of CSK buffer (0.5% Triton 100, 100mM NaCl, 10mM PIPES, 2mM MgCl) and incubated at room temperature for 30 minutes. Plates were blocked with 250 µl/well of 2% BSA in 1X CMF-PBS (blocking solution) and incubated at 37°C for 60 minutes. Blocking solution was aspirated and 50 to 100 µl/well of supernatant from fusion plates was added. Plates were incubated at room temperature for 60 minutes and then were washed one time with 250 µl/well of 0.5% BSA in CMF-PBS (wash solution 1) and two times with 250 µl/well of CMF-PBS (wash solution 2). One hundred fifty µl of horseradish peroxidase conjugated goat anti-mouse IgG(fc) (Jackson ImmunoResearch, West Grove, PA) diluted 1:3500 in PBST was added and plates were incubated at room temperature for 60 minutes. Plates were washed as before and 150 µl substrate consisting of 1mg/ml o-phenylene diamine (Sigma) and 0.1 ml/ml 30% H₂O₂ in 100mM Citrate, pH 4.5 was added. The color reaction was stopped after 30 minutes with the addition of 50 µl of 15% H₂SO₄. A₄₉₀ was read on a plate reader (Dynatech). About 20 positive wells were identified for each fusion and were subsequently cloned.

Hybridomas were screened in cloning steps in an ELISA assay by testing for reactivity of monoclonals to the cadherin-5 EC2-4 fusion protein and excluding maltose binding protein reactive monoclonals. Immulon 4 plates (Dynatech, Cambridge, MA) were coated at 4°C with 50 µl/well fusion protein diluted to 0.1 µg/well (for fusion protein) and to 0.2 µg/well (for maltose binding protein alone) in 50mM carbonate buffer, pH 9.6. Plates were washed 3 times with PBS, 0.05% Tween 20 (PBST) and 50 µl hybridoma culture supernatant was added. After incubation at 37°C for 30 minutes, and washing as above, 50 µl of horseradish peroxidase conjugated goat anti-mouse IgG(fc) (Jackson ImmunoReseach, West Grove, PA) diluted 1:3500 in PBST was added. Plates were incubated at 37°C for 30 minutes and washed 4 times with PBST. One hundred µl substrate consisting of 1 mg/ml o-phenylene diamine (Sigma Chemical Co., St. Louis, MO) and 0.1 µl 30% H₂O₂ in 100 mM citrate, pH 4.5 was added. The color reaction was stopped after 5 minutes with the addition of 50 µl of 15% H₂SO₄. Absorbance at 490 nm was determined using a plate reader.

The hybridomas designated 30Q8A (ATCC HB11316), 30Q4H (ATCC HB11317), 45A5G (HB11318), 30S2F (HB11319), 45C6A (HB11320), 30T11G (ATCC HB11324), 30M8G, 30O6E and 30R1A] were identified as reactive with endothelial cells and with the cadherin-5 EC2-4 fusion protein. The hybridomas were cloned twice by limiting dilution and grown in ascites. The monoclonal antibodies produced by the hybridomas were isotyped in an ELISA assay. The results of the assay are presented in Table 2 below.

### C. Subdomain Specificity of C5 Specific Monoclonal Antibodies

To determine if the hybridomas produced monoclonal antibodies reactive with unique epitopes of the extracellular domain of C5, the monoclonal antibodies were purified, biotinylaled, and tested in a cross competition ELISA. Immulon IV 96-well plates were coated with either EC1-2 or EC2-4 cadherin-5 fusion protein at 0.2 µg/ml in 50 µl 50mM NaCO₃, pH 9.6 overnight at 4°C. The wells were aspirated and washed three times with PBS/0.05% Tween 20. The plate was then blocked with 50 µl/well PBS, 2% BSA (Sigma) for 30 minutes at 37°C. Monoclonal antibodies were purified from hybridoma supernatants over a protein A-Sepharose column and the eluted antibody was dialyzed against 0.1M NaCO₃ pH 8.2. One mg/ml of antibody was reacted with 60 µl of a 1 mg/ml stock solution in DMSO of NHS-biotin (Pierce Chemical Co., Rockford, IL) for 1 hour at room temperature and the reaction was stopped by dialysis overnight at 4°C against CMF/PBS. The biotinylated antibodies in PBS/0.05% Tween 20 were then added as primary antibody (50 µl/well) to a plate coated with fusion protein and incubated for 30 minutes at 37°C. The plate was then aspirated and washed three times with PBS/0.05% Tween 20. Peroxidase-conjugated strepavidin in PBS/Tween was added 50 µl/well and incubated for 30 minutes at 37°C. The plate was aspirated and washed three times in PBS/0.05% Tween 20, and o-phenylenediamine in 100mM citrate buffer and hydrogen peroxide was added at 100 µl/well. The plate was developed at room temperature for 5-15 minutes. The reaction was stopped with 50 µl/well 15% sulfuric acid and the plate was read on a plate reader. Results of the assay are presented in Table 2 below.

To confirm subdomain specificity, the cadherin-5 fusion proteins EC1-2 and EC2-4 were run on SDS-PAGE (10%) and immunoblotted with the cadherin-5 specific monoclonal antibodies.

Table 2 below set outs the domain specificity and isotype of the cadherin-5 specific monoclonal antibodies.

**Table 2**

| Monoclonal Antibody | C5 Subdomain | Isotype |
|---|---|---|
| 30Q4H | 2 | IgG_{2b} |
| 45A5G | 2 | IgG₁ |
| 45C6A | 2 | IgG₁ |
| 30S2F | 3-4 | IgG₁ |
| 30Q8A | 3-4 | IgG_{2b} |
| 30T11G | 3-4 | IgG₁ |

Competition assays were carried out as described above for assays for binding to cadherin-5 EC2-4 fusion protein except that unlabelled primary cadherin-5 specific monoclonal antibodies (or mouse IgG) were added 30 minutes prior to addition of biotinylated cadherin-5 specific monoclonal antibodies. Monoclonal antibodies produced by the hybridomas 30M8G, 30O6E and 30RIA compete for a site that is near or identical to the binding site of the antibody produced by hybridoma 30Q4H.

### Example 5

Human cadherins-4 and -5 and rat cadherin -8 were expressed in mouse fibroblast L cells (ATCC CCL1.3) which do not normally express cadherins.

### A. Construction of Expression Vectors

The cDNA sequences encoding human cadherins-4 and -5 which are described in Example 2 and the cDNA sequence encoding rat cadherin-8 which is described in Example 1 were subcloned into the multicloning site of expression vector pRC/RSV (Invitrogen).

Cadherin-4 DNA sequences were isolated by an *in vivo* excision procedure from the λZapII clone (described in Example 2) containing the entire coding sequence of cadherin-4. Using a helper virus, the sequences were excised from λZapII in the form of Bluescript plasmid. The plasmid was then cut with *Hind*III and blunt-ended with T4 polymerase. The resulting DNA fragment was redigested with *Spe*I to generate a cadherin-4 cDNA fragment having a blunt end and a *Spe*I sticky end. The fragment was purified by agarose gel electrophoresis and subcloned into the pRC/RSV expression vector that had been previously digested with *Spe*I and *Xba*I (the *Xba*I end was blunt-ended with T4 polymerase).

The λgt11 clone containing the entire coding sequence of cadherin-5 (described in Example 2) was cut with *Eco*RI and the resulting fragment containing the cadherin-5 sequences was purified by agarose gel electrophoresis. The purified fragment was then subcloned into the *Eco*RI site of the Bluescript plasmid. Cadherin-5 sequences were cut from the resulting construct with *Hinc*II and *Xba*I and subcloned into the *Not*I-*Xba*I site of the pRC/RSV vector.

The full length cDNA encoding rat cadherin-8 was excised from the Uni-ZAP clone described in Example 1 by digestion with *Kpn*I, followed by blunt-ending and re-digestion with *Spe*I. The cadherin-8 encoding fragment was purified by agarose gel electrophoresis and was subcloned into the pRC/RSV vector which had been digested with *Xba*I, blunt-ended and redigested with *Spe*I.

### B. Transfection of L Cells

Mouse fibroblast L cells were transfected with the human cadherin-4 and -5 and rat cadherin-8 expression constructs by a Ca²⁺ phosphate precipitation method and stable transfectants were obtained by G418 selection. Cadherin-4 and -8 transfectant cells showed a morphology similar to that of parental L cells (fibroblastic), but cadherin-5 transfectant cells exhibited a flattened morphology. Neuro 2a cells (ATCC CCL131) were also transfected by a Ca²⁺ phosphate precipitation procedure with the cadherin-4 and cadherin-8 expression constructs. Cadherin-4 transfectants showed epithelial structure, suggesting that cadherin-4 has activity in epithelial structure formation and may be involved in the neural tissue development.

### C. Northern and Western Blot Assays of Cadherin mRNA and Protein Expression in Transfected Cells

Both cadherin-4, -5 and -8 transfectants showed mRNA of the expected size of 3.5 kb, 3.2 kb and 3 kb, respectively, in Northern blot analysis using the appropriate full length human cDNAs as a probe. (See Example 6A for a description of the Northern blot assay.)

For Western blots, cadherin-4, -5 and -8 transfectants were washed with PBS and SDS-PAGE sample buffer was added directly to the cells. SDS-PAGE (Laemmli) was carried out and and gels were blotted electrophoretically onto PVDF membrane. The membranes were incubated in TBS containing 5% skim milk for 2 hours at room temperature and then were incubated with the appropriate polyclonal antibody in TBS containing 0.05% Tween 20 for 1 hour at room temperature. After four washes (of 5 minutes each) with TBS containing 0.05% Tween 20, the membranes were incubated with alkaline phosphatase conjugated anti-rabbit IgG antibody (Promega Corp., Madison, WI) in TBS containing 0.05% Tween 20 for 1 hour at room temperature. The membranes were then washed again four times with TBS containing 0.05% Tween 20 at room temperature and developed by using Promega Western blue. Cadherin-4, -5 and -8 polyclonal antibodies each reacted with a band of about 130 KD.

### D. Calcium Protection from Trypsin Digestion

Since cadherins have been shown to be protected from trypsin digestion by Ca²⁺, the effect of Ca²⁺ on trypsin treatment (0.01 % soybean trypsin for 30 minutes at 37°C) of human cadherin-4 and -5 and rat cadherin-8 expressed on the surface of transfected L cells was examined. Two mM Ca²⁺ protected the cadherin-4 from the trypsin digestion, but cadherin-5 and cadherin-8 were digested easily even in the presence of 1-5 mM of Ca²⁺.

### E. Cell-Cell Adhesion Assay

The cell-cell adhesion activity of the transfected cells was assayed by a re-aggregation assay as described in Yoshida-Noro *et al.*, *Devel. Biol., 101,* 19-27 (1984). Briefly, transfectants were grown to near confluency and then dispersed into single cells with mild trypsin treatment (0.01 % for 15 minutes) in the presence of 2mM Ca²⁺. After washing, the trypsinized cells were incubated in Hepes buffered saline (HBS) containing 2mM CaCl₂, 1% BSA and 20 µg/ml deoxynuclease on a rotary shaker at 50 rpm for 30 to 60 minutes and then cell aggregation was monitored. Cadherin-4 transfectant cells aggregated within 30 minutes and formed relatively large aggregates, whereas cadherin-5 transfectant cells did not aggregate under the same conditions. However, cadherin-5 transfectants gradually re-aggregated and formed relatively small aggregate after prolonged incubation (4-5 hours or more). Similarly, cadherin-8 transfectants did not show significant cell adhesion activity. Parental L cells did not show cell adhesion under the same conditions. The sensitivity of cadherin-5 and cadherin-8 to trypsin digestion may account for the reduced cell adhesion seen in the reaggregation assay because the transfected L cells are initially dispersed with trypsin in the assay.

### Example 6

The expression of mRNAs encoding cadherins of the invention was examined in rat brain, kidney, liver, lung and skin and in various human cells by Northern blot analysis. The expression of cadherin protein was also examined in endothelial cells and leukocytes by immunofluorescence or immunoblotting.

### A. Northern Blot Assays of Rat Tissue and Human Cells

Poly(A)⁺ RNA from rat brain, kidney, liver, lung and skin was prepared as described in Example 1 for rat brain. The RNA preparations were then electrophoresed in an 0.8% agarose gel under denaturing conditions and transferred onto a nitrocellulose filter. Northern blot analyses were carried out according to a method described in Thomas, *Proc. Natl. Acad. Sci. USA, 77*, 5201-5202 (1980). Filters were hybridized with rat cadherin PCR fragments (described in Example 1) labeled with ³²P, including fragments corresponding to cadherins-4 through -11. The final hybridization wash was in 0.2X standard saline citrate containing 0.1% sodium dodecyl sulfate at 65°C for 10 minutes.

Cadherin-4 and cadherin-8 through -10 mRNAs were detected only in rat brain. The cadherin-8 PCR fragment hybridized to a major band of about 3.5 kb and a minor band of about 4.5 kb in rat brain. The mRNAs detected may be alternative splicing products and may correspond to the truncated and full length cadherin-8 clones described in Example 1. Cadherin-6 and -7 probes gave weak, signals on rat brain mRNA even after prolonged exposure. Cadherins-5, -6 and -11 mRNAs were detected in rat brain and other rat tissues including cadherin-5 mRNA in lung and kidney, cadherin-6 mRNA in kidney, and cadherin-11 mRNA in liver.

The expression of cadherin-8 and -11 in cultured human SK-N-SH neuroblastoma cells (ATCC HTB11), U251MG glioma cells and Y79 retinoblastoma cells (ATCC HTB18) was also assayed by Northern blot. Human cDNAs encoding cadherins-8 and -11 (described in Example 2) were labelled with ³²P and used as probes of poly(A)⁺ RNA prepared from the cells using an Invitrogen FastTrack kit.

The Northern blot procedure detected cadherin-8 RNA in the neuroblastoma and retinoblastoma cell lines, while cadherin-11 RNA was detected only in neuroblastoma cells. These results indicate that at least some of the cadherins of the invention are expressed in neurons and glial cells and/or their precursor cells.

Cadherin-5 RNA was detected by Northern blot assay of HUVECs (Clonetics), but was not detected in A431 human epidermoid carcinoma cells (ATCC CRL1555) or IMR90 human fibroblast cells (ATCC CCL186).

### B. Immunoflourescence of Endothelial Cells and Immunoblotting of Leukocytes

Cultured endothelial cells isolated from bovine aorta, bovine brain microvasculature and human umbilical vein were subjected to immunofluorescence microscopy using anti-C5 polyclonal antibodies. Cadherin-5 protein at the cell junctions which was in close association with the peripheral actin microfilaments was labelled.

In contrast, when freshly isolated leukocytes (human PMN, lymphocytes and monocytes) or the monocyte-like cell line U937 were analyzed for the expression of cadherin-5 by immunoblotting using polyclonal antibodies and a monoclonal antibody (30O6E) to cadherin-5, no cadherin-5 was detected. Furthermore, using a pan-cadherin antibody [Geiger *et al., J. Cell Science, 97*: 607-614 (1990)] specific for the cytoplasmic tail, no other cadherins were detected in these cell populations.

### Example 7

Three *in vitro* transendothelial migration assays were utilized to show that cadherin-5 may participate in the movement of leukocytes across the intercellular junctions of endothelium.

### A. Transmigration Assays

The migration of leukocytes (either human polymorphonuclear neutrophils or rat T cells) was followed for specific periods of time (15 minutes for PMNs and 2 hours for T cells). Immunofluorescent labeling of leukocytes using antibodies to specific cellular markers was used distinguish between leukocytes and endothelium. The polyclonal antibodies described in Example 4 were used to measure changes in the distribution of cadherin-5. An antibody (Novocastra Laboratories Ltd., United Kingdom) to PE-CAM1 (CD31) which is an intercellular junction molecule in endothelium was used as a control.

The role of cadherin-5 in the transmigration of polymorphonuclear neutrophils (PMNs) across HUVEcs was analyzed. The system utilized, which is described in Furie *et al., J. Immunol, 143*: 3309-3317 (1989), has been characterized with regard to electrical resistance of the endothelium and the adhesion molecules used in transmigration. HUVEcs were isolated in the absence of growth factor and cultured on human amniotic connective tissue in a two-chamber system. PMN migration on IL1β-treated HUVEcs has previously been shown to involve E-selectin and β₂ integrins (CD11/CD18). See Furie *et al. J. Immunol., 148*: 2395-2484 (1992).

In the first assay, transmigration of PMNs was followed as an 11 minute time course on HUVEcs pretreated for four hours with IL1β (1.5 U/ml) (Collaborative Research Inc., Beford, MA). Prior to addition of neutrophils, antibodies to cadherin-5 heavily labelled the cell junctions of the HUVEcs in a continuous pattern. Pretreatment of the endothelial monolayer with IL1β had no effect on the distribution of cadherin-5 in the HUVEc monolayer compared to a control untreated culture. In the second assay, chemotaxis of PMNs across HUVEcs was stimulated by leukotriene B₄ (LTB₄) (Sigma) which was placed in the bottom chamber at 10⁻⁷M while neutrophils were added to the upper chamber. Chemotaxis of PMNs to LTB₄ across the endothelial monolayer was previously shown to be blocked by antibodies to CD11a, CD11b and ICAM-1. [See Furie *et al., Blood, 78*: 2089-2097 (1991)] In both assays, PMNs were identified with anti-CD45 antibody (Becton Dickinson, San Jose, CA).

In both assays during the 11-minute time course, the majority of the PMNs that adhered also transmigrated. Addition of neutrophils caused a rapid redistribution and regional loss of cadherin-5 even at the earliest time point (3 minutes). CD31 was also lost at sites of disruption of the monolayer, but in general appeared to be more stable during the transmigration process. The loss of cadherin-5 is probably the result of proteases released from the neutrophils during transmigration.

In a third assay, CD4 antigen activated rat T cells were utilized instead of PMNs (for a two-hour time course). Rat brain microvascular endothelium was grown on Transwell 5 micron polycarbonate membranes (Costar, Cambridge, MA). T cells were identified using an anti-CD4 antibody (Serotec, Indianapolis, IN). In this assay, the loss of cadherin-5 immunolabeling did not occur during transendothelial migration even though 10% of the T cells had crossed the endothelium after two hours. These results demonstrate differential effects of PMN versus T cells on intercellular junctions during tranendothelial migration. Analysis by confocal microscopy suggests that CD4 antigen-activated T cells and PMNs have a ligand that is able to interact with cadherin-5 on the endothelium during transmigration. Photomicrographs from confocal analysis show that during leukocyte transendothelial migration leukocytes can be found spanning the intercellular junction. The leukocyte separates the cell junction and cadherin-5 remains on adjacent cells even though the endothelial cells are not in contact.

### B. Adhesion of PMNs and T Cells to Cadherin-5

To quantitate the binding of PMNs and activated T-cells to cadherin-5, a cell-substrate adhesion assay was developed. This assay utilized plate-bound fusion proteins containing various extracellular subdomains of cadherin-5 (EC1-2 or EC2-4, see Example 4) and measured the binding of dye-labelled leukocytes to cadherin-5 protein using a cytofluor 2300 (Millipore, Bedford, MA).

The purified fusion proteins were absorbed to styrene plates and the binding of dye-labeled leukocytes to the fusion proteins was compared to binding to maltose binding protein and heat denatured bovine serum albumin (BSA) which was used to block nonspecific binding. The fusion proteins were dissolved in PBS containing Ca²⁺ and Mg²⁺, diluted into coating buffer and incubated overnight at 4°C. The plates were blocked with heat denatured BSA and then incubated with calcien (Molecular Probes, Eugene, OR)-labelled cells for 1 hour at 37°C. Results of the assay are presented in FIGURE 1 wherein the relative fluorescence values reported are the mean value of three samples.

PMNs bound to fusion proteins comprising the EC2-4 of cadherin-5, but preferentially bound to fusion proteins comprisng EC1-2. These results are consistent with presence of cadherin subdomain 2 sequences in both fusion proteins. CD4 antigen activated T cells bound EC2-4 fusion protein. All these results, which indicate that PMNs interact with a more terminal or exposed subdomain of cadherin-5, are consistent with the rate that these cell types cross the endothelium, PMNs transmigrate in a few minutes and T cells require 30-60 minutes. The binding of U937 cells could be blocked in a dose dependent manner by polyclonal antisera made to the cadherin-5 EC2-4 subdomains.

The results presented in the foregoing paragraph in combination with the results presented in Example 6B that leukocytes do not express cadherins suggests that the counter ligand to which cadherin-5 binds on leukocytes is a distantly related cadherin or is not a cadherin. Cadherin binding has previously been thought to be homotypic.

### Example 8

Expression of cadherin-5 in the blood-brain barrier in the endothelium of the cerebral cortex was assayed by Western blot and immunocytochemistry.

A SDS lysate was prepared by boiling bovine or macaque capillaries in SDS sample buffer for 2 minutes and then drawing the extract through a 25 G syringe needle. The extract was centrifuged in a microfuge for 15 minutes at 4°C. Protein concentration in the supernatant was determined by the BCA method (Pierce) using bovine serum albumin as a standard. Samples of the supernatent (75µg) were separated by SDS-PAGE (Laemmli) and electrophoretically transferred to nitrocellulose. The nitrocellulose was blocked with 5% milk and 10% FBS in Tris-buffered saline, pH 8.0, containing 0.05% Tween 20. Cadherin-5 specific monoclonal antibodies (30Q4H and 45C6A) were added. After washing to remove unbound antibody, the filters were incubated with alkaline phosphatase-conjugated anti-mouse IgG (Promega, Madison, WI). Reactive bands were visualized by addition of NBT/BCIP (Sigma, St. Louis, MO). Expression of cadherin-5 was detected in the freshly isolated bovine and macaque capillaries.

The Western blot results were confirmed by immunocytochemistry using the cadherin-5 antibodies 30Q4H and 45C6A. Macaque cerebral cortex was incubated in 15% sucrose in PBS for 30 minutes at 4°C and embedded in OCT compound (Tissue-Tek, Elkhart, IN) in cryomolds and quickly frozen. Six micron sections were cut and placed on glass slides. The slides were washed with PBS and fixed in 3% p-formaldehyde for 5 minutes. To permeabilize the tissue sections the slides were immersed in -20°C acetone for 10 minutes and air dried. The sections were blocked with 2% goat serum and 1 % BSA in PBS for 30 minutes and then incubated with the primary antisera for 1 hour at room temperature. The sections were rinsed 3 times in PBS containing 0.1% BSA and incubated with biotinylated anti-rabbit or anti-mouse IgG (Vector Laboratories, Burlingame, CA) in 1 % BSA in PBS for 30 minutes. After rinsing 3 times, strepavidin-conjugated with horseradish peroxidase (Vector Laboratories) was added for 30 minutes and washed 3 times. Immunolabeling was detected by reaction with diaminobenzoic acid in the presence of NiCl₂. The monoclonal antibody 45C6A only appeared to label larger vessels and the monoclonal antibody 30Q4H labeled both large and microvessels. The cell junctions of cerebral capillaries were labelled with the anti-cadherin-5 antibodies in a localized site.

These results and the results presented in Example 7 suggest cadherin-5 is involved in maintenance of the blood-brain barrier and that cadherin-5 peptides or cadherin-5 specific monoclonal antibodies may be able to open the blood-brain barrier.

### Example 9

Patent Cooperation Treaty (PCT) International Publication No. WO 91/04745 discusses fragments of cell adhesion molecules and antibodies to cell adhesion molecules which are purported to disrupt-microvascular and endothelial cell tight junctions.

Three cadherin-5 peptides corresponding to the cell binding domain [HAV region, Blaschuk *et al., Devel. Biol., 139*: 227-229 (1990)], the calcium binding region A1 and the calcium binding region B1 of E-cadherin [Ringwald *et al., EMBO J., 6*: 3647-3653 (1987)] were tested for the ability to affect the permeability of brain endothelium. The peptides utilized had the following sequences:
Peptide 1 (Amino acids 114 to 128 of SEQ ID NO: 50)
   LTAVIVDKDTGENLE,
Peptide 2 (Amino acids 132 to 145 of SEQ ID NO: 50)
   SFTIKVHDVNDNWP, and
Peptide 3 (Amino acids 168 to 178 of SEQ ID NO: 50)
   SVTAVDADDPT, respectively.

Permeability was measured using a two-chamber culture system (Costar). Rat brain microvascular endothelium was grown on 12 mm Transwell filters with 3 micron pores (Costar) in the culture system. When the monolayers were confluent, two weeks after plating, ³H-inulin (201 mCi/g) (New England Nuclear, Boston, MA) was added to the upper chamber. Cadherin-5 peptide at 100 µg/ml was added to both the upper and lower chambers. Radioactivity appearing in the bottom chamber was measured at 15 minute intervals over a two hour time course carried out at 37°C and was compared to the radioactivity appearing in the bottom chamber of cultures where no peptide was added or where no endothelial cells were present.

Both peptides 1 and 3 increased endothelium permeability in comparison to control cultures. The increase in permeability obtained with peptide 3 was 2.5-fold and the increase with peptide 1 was 1.5-fold over the controls. Peptide 2 had no effect on permeability.

### Example 10

The functional properties of cadherins involve not only specific intercellular interactions, but also involve intracellular interactions with the cytoskeleton. Immunoprecipitation experiments utilizing the cadherin-5-specific rabbit polyclonal antibodies and the monoclonal antibody 30Q8A (see Example 4) were performed to determine with which proteins cadherin-5 interacts on an intracellular level.

Endothelial cells were metabolically labeled overnight with 50 µCi/ml of [³⁵S]-methionine and were then extracted with 0.5% Triton X-100 in 10mM HEPES pH 7.4, 0.15M NaCl, 2mM EDTA, 2mM EGTA, 1mM phenanthroline and protease inhibitors. The inhibitors included 1mM PMSF, 10 µg/ml aprotinin, leupeptin, pepstatin A, antipain, soybean trypsin inhibitor, 100 µg/ml chymostatin and TPCK, 40 µg/ml of TPCK and bestatin, 50 µg/ml of benzamidine, 1mM o-vanidate and 20mM NaF. After 20 minutes on ice, the cells were scraped and centrifuged in a microfuge for 30 minutes at 4°C. The supernatant was precleared and either polyclonal anti-cadherin-5 or normal rabbit serum was added and incubated overnight at 4°C. Protein A-sepharose (Pharmacia, Piscataway, NJ) was added for 2 hours at 4°C and centrifuged. A first low stringency wash with 10mM HEPES pH 7.4, 0.15M NaCl, 2mM EDTA and 2mM EGTA containing 1% Triton X-100, 0.5%. DOC and 0.2% SDS was performed. A second high stringency wash was performed with the same buffer containing 2% SDS. A final wash was then performed with Tris-buffered saline, and the samples were boiled and analyzed on SDS/PAGE (7%). Three bands with molecular weights of 104 KD, 95 KD, and 82 KD were identified as associated with cadherin-5.

Three intracellular proteins, termed catenins, have previously been identified by their ability to bind to the cytoplasmic domain of E-cadherin. These proteins have been designated α, β, and γ catenins and have molecular weights of 102 KD, 88 KD and 80 KD, respectively [Ozawa *et al.*, *EMBO J. 8*: 1711-1717 (1989)]. The association of catenins with E-cadherin seem to be required for E-cadherin function because deletion of the cytoplasmic domain of E-cadherin results in loss of cell adhesion function and catenin binding. The molecular cloning of α-catenin has shown it to be a vinculin-like protein [Nagafuki *et al., Cell, 65*: 849-857 (1991); Herrenkenecht *et al., Proc. Natl. Acad. Sci. USA, 88*: 9156-9160 (1991)]. The amino acid sequence of the *Xenopus* β-catenin [McCrea *et al., Science, 254*: 1359-1361 (1991)] exhibits 63% similarity to the human protein plakoglobin [Franke *et al., Proc. Natl. Acad. Sci. USA, 86*: 4027-4031 (1989)]. Plakoglobin has been localized to both the cytoplasmic region of desmosome and adherens junctions in epithelial cells. The desmonsomal component desmoglein I interacts with plakoglobin and is a member of the cadherin superfamily [Koch *et al*., Eur. *J. Cell. Biol., 53*: 1-12 (1990)]. Plakoglobin has a molecular weight of 82 KD and may be the γ-catenin [Peifer *et al., J. Cell Biol., 118*: 681-691 (1992)]. Even though endothelial cells lack desmosome, they have been shown to contain plakoglobin-associated with intercellular junctions [Franke *et al., Biol. of the Cell, 59*: 205-218 (1987)]. Other cytoskeletal elements associated with cadherins are ankyrin and fodrin [Nelson *et al., J. Cell Biol., 110*: 349-357 (1990)].

To identify whether plakoglobin was one of the proteins complexed to cadherin-5, an unlabeled lysate of bovine aortic endothelial cells was made and immunoprecipitation was carried out as described above using anti-cadherin-5 antibody. The unlabelled immunoprecipitates were separated by SDS/PAGE and then electrophoretically transferred to nitrocellulose. The membrane was blocked with 5% milk in Tris-buffered saline, pH 8.0, containing 0.05% Tween 20 (TBST) and then was incubated with the murine monoclonal antibody PG5.1 (IBI Research Products, Cambridge, MA) to plakoglobin in blocking solution (1:20) for 1 hour at room temperature. The membrane was washed with TBST and then incubated with goat anti-mouse IgG conjugated to alkaline phosphatase. An 82 KD protein was identified using NBT/BCIP under both low and high stringency wash conditions. These results demonstrate that plakoglobin is tightly associated with the cytoplasmic domain of cadherin-5 in endothelium. Immunofluorescence studies of regenerated endothelium show that cadherin-5 and plakoglobin are localized to the cell junctions and are coordinately regulated.

The interation of cadherin-5 with plakoglobin may be a target for modulation of cadherin-5 activity.

While the present invention has been described in terms of preferred embodiments, it is understood that variations and improvements will occur to those skilled in the art. Thus, only such limitations as appear in the appended claims should be placed on the scope of the invention.

## Claims

1. A purified and isolated polynucleotide encoding a cadherin selected from the group consisting of cadherin-6, cadherin-7, cadherin-9 and cadherin-10.

2. The polynucleotide of claim 1 which is a DNA sequence.

3. The polynucleotide of claim 2 which is a cDNA sequence or biological replica thereof.

4. The polynucleotide of claim 3 which is SEQ ID NO: 51.

5. The polynucleotide of claim 3 which is SEQ ID NO: 15.

6. The polynucleotide of claim 3 which is SEQ ID NO: 19 or SEQ ID NO: 33.

7. The polynucleotide of claim 3 which is SEQ ID NO: 55.

8. The polynucleotide of claim 2 which is a genomic DNA or a biological replica thereof.

9. The DNA of claim 2 which is a wholly or partially chemically synthesized DNA or a biological replica thereof.

10. A biologically functional DNA vector comprising a DNA according to claim 2.

11. The vector of claim 10 wherein said DNA is operatively linked to an expression control DNA sequence.

12. A host cell stably transformed or transfected with a DNA according to claim 2 in a manner allowing the expression in said host cell of the cadherin polypeptide encoded thereby.

13. A method for producing a cadherin polypeptide comprising the steps of growing a host cell according to claim 12 in a suitable nutrient medium and isolating the cadherin from said cell or from the medium of its growth.

14. A purified and isolated full length cadherin polypeptide selected from the group consisting of cadherin-6 polypeptide (SEQ ID NO: 52), cadherin-7 polypeptide (SEQ ID NO: 16), cadherin-9 polypeptide (SEQ ID NO: 20 or 34) and cadherin-10 polypeptide (SEQ ID NO: 56).

15. A hybridoma cell line producing a monoclonal antibody specific for a cadherin selected from the group consisting of cadherin-6, cadherin-7, cadherin-9 and cadherin-10.

16. A hybridoma cell line producing a monoclonal antibody specific for cadherin-5 selected from the group consisting of 30Q8A (ATCC HB11316), 30Q4H (ATCC HB11317), 45A5G (ATCC HB11318), 30S2F (ATCC HB11319), 45C6A (ATCC HB11320) and 30T11G (ATCC 11324).

17. A monoclonal antibody produced by the hybridoma cell line of claim 16.

18. An antibody substance specific for a cadherin selected from the group consisting of cadherin-6, cadherin-7, cadherin-9 and cadherin-10.

19. A method for modulating the binding capability of a cadherin selected from the group consisting of cadherin-6, cadherin-7, cadherin-9 and cadherin-10 comprising contacting the cadherin with an antibody substance specific for said cadherin according to claim 18.

20. A method for modulating the binding capability of a cadherin selected from the group consisting of cadherin-6, cadherin-7, cadherin-9 and cadherin-10 comprising contacting the cadherin with a polypeptide or peptide ligand of the cadherin.

21. A method for modulating the binding capability of a cadherin selected from the group consisting of cadherin-6, cadherin-7, cadherin-9 and cadherin-10 comprising contacting the cadherin with a peptide of said cadherin.


SEQUENCE LISTING

(1) GENERAL INFORMATION:

(i) APPLICANT: Suzuki, Shintaro

(ii) TITLE OF INVENTION: CADHERIN MATERIALS AND METHODS

(iii) NUMBER OF SEQUENCES: 62

(iv) CORRESPONDENCE ADDRESS:
(A) ADDRESSEE: Marshall, O'Toole, Gerstein, Murray &
Borun
(B) STREET: 6300 Sears Tower, 233 S. Wacker Drive
(C) CITY: Chicago
(D) STATE: Illinois
(E) COUNTRY: USA
(F) ZIP: 60606

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: US 07/872,643
(B) FILING DATE: 17 APR 1992

(viii) ATTORNEY/AGENT INFORMATION:
(A) NAME: Noland, Greta E.
(B) REGISTRATION NUMBER: 35,302
(C) REFERENCE/DOCKET NUMBER: 31340

(ix) TELECOMMUNICATION INFORMATION:
(A) TELEPHONE: (312) 474-6300
(B) TELEFAX: (312) 474-0448
(C) TELEX: 25-3856

(2) INFORMATION FOR SEQ ID NO:1:

(i) SEQUENCE CHARACTERISTICS:
(A) LENGTH: 6 amino acids
(B) TYPE: amino acid
(D) TOPOLOGY: linear

(ii) MOLECULE TYPE: peptide

(xi) SEQUENCE DESCRIPTION: SEQ ID NO:1:

Thr Ala Pro Pro Tyr Asp
1               5

(2) INFORMATION FOR SEQ ID NO:2:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:2:

GAATTCACNG CNCCNCCNTA YGA 23

(2) INFORMATION FOR SEQ ID NO:3:

(i) SEQUENCE CHARACTERISTICS:
(A) LENGTH: 6 amino acids
(B) TYPE: amino acid
(D) TOPOLOGY: linear

(ii) MOLECULE TYPE: peptide

(xi) SEQUENCE DESCRIPTION: SEQ ID NO:3:

Phe Lys Lys Leu Ala Asp
1               5

(2) INFORMATION FOR SEQ ID NO:4:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:4:

GAATTCTCNG CNARYTTYTT RAA 23

(2) INFORMATION FOR SEQ ID NO:5:

(i) SEQUENCE CHARACTERISTICS:
(A) LENGTH: 6 amino acids
(B) TYPE: amino acid
(D) TOPOLOGY: linear

(ii) MOLECULE TYPE: peptide

(ix) FEATURE:
(A) NAME/KEY: Modified-site
(B) LOCATION: 2
(D) OTHER INFORMATION: /note= "The amino acid at this
position is a proline or a glycine."

(ix) FEATURE:
(A) NAME/KEY: Modified-site
(B) LOCATION: 3
(D) OTHER INFORMATION: /note= "The amino acid at this
position is a leucine, an isoleucine or a valine."

(ix) FEATURE:
(A) NAME/KEY: Modified-site
(B) LOCATION: 5
(D) OTHER INFORMATION: /note= "The amino acid at this
position is a phenylalanine or a tyrosine."

(xi) SEQUENCE DESCRIPTION: SEQ ID NO:5:

Lys Xaa Xaa Asp Xaa Glu
1               5

(2) INFORMATION FOR SEQ ID NO:6:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:6:

GAATTCAARS SNNTNGAYTW YGA                                    23

(2) INFORMATION FOR SEQ ID NO:7:

(i) SEQUENCE CHARACTERISTICS:
(A) LENGTH: 6 amino acids
(B) TYPE: amino acid
(D) TOPOLOGY: linear

(ii) MOLECULE TYPE: peptide

(ix) FEATURE:
(A) NAME/KEY: Modified-site
(B) LOCATION: 1
(D) OTHER INFORMATION: /note= "The amino acid at this
position is an asparagine or an aspartic acid."

(ix) FEATURE:
(A) NAME/KEY: Modified-site
(B) LOCATION: 3
(D) OTHER INFORMATION: /note= "The amino acid at this
position is an alanine or a proline."

(xi) SEQUENCE DESCRIPTION: SEQ ID NO:7:

Xaa Glu Xaa Pro Xaa Phe
1               5

(2) INFORMATION FOR SEQ ID NO:8:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:8:

GAATTCRAAN NNNGGNGSYT CRT                                    23

(2) INFORMATION FOR SEQ ID NO:9:

(i) SEQUENCE CHARACTERISTICS:
(A) LENGTH: 117 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:9:

TCCCTGCTGG TCTTCGACTA CGAAGGCAGC GGTTCTACTG CAGGCTCTGT CAGCTCCCTG 60

AACTCCTCCA GCTCCGGGGA TCAAGATTAC GACTACTTGA ATGACTGGGG GCCCCGG 117

(2) INFORMATION FOR SEQ ID NO:10:

(i) SEQUENCE CHARACTERISTICS:
(A) LENGTH: 39 amino acids
(B) TYPE: amino acid
(D) TOPOLOGY: linear

(ii) MOLECULE TYPE: protein

(xi) SEQUENCE DESCRIPTION: SEQ ID NO:10:

Ser Leu Leu Val Phe Asp Tyr Glu Gly Ser Gly Ser Thr Ala Gly Ser
1               5                   10                  15

Val Ser Ser Leu Asn Ser Ser Ser Ser Gly Asp Gln Asp Tyr Asp Tyr
            20                  25                  30

Leu Asn Asp Trp Gly Pro Arg
        35

(2) INFORMATION FOR SEQ ID NO:11:

(i) SEQUENCE CHARACTERISTICS:
(A) LENGTH: 120 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:11:

ACACTGCACA TCTACGGCTA CGAGGGCACA GAGTCCATCG CAGAGTCCCT CAGCTCCCTG 60

AGCACCAATT CCTCCGACTC TGACATCGAC TATGACTTCC TCAATGACTG GGGACCCAGG 120

(2) INFORMATION FOR SEQ ID NO:12:

(i) SEQUENCE CHARACTERISTICS:
(A) LENGTH: 40 amino acids
(B) TYPE: amino acid
(D) TOPOLOGY: linear

(ii) MOLECULE TYPE: protein

(xi) SEQUENCE DESCRIPTION: SEQ ID NO:12:

Thr Leu His Ile Tyr Gly Tyr Glu Gly Thr Glu Ser Ile Ala Glu Ser
1               5                   10                  15

Leu Ser Ser Leu Ser Thr Asn Ser Ser Asp Ser Asp Ile Asp Tyr Asp
            20                  25                  30

Phe Leu Asn Asp Trp Gly Pro Arg
        35                  40

(2) INFORMATION FOR SEQ ID NO:13:

(i) SEQUENCE CHARACTERISTICS:
(A) LENGTH: 120 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:13:

TCCTTGGCCA CCTATGCCTA CGAAGGAACT GGCTCGGTGG CCGACTCCCT GAGCTCACTA 60

GAATCAGTGA CCACAGATGG AGACCAAGAT TATGACTATT TGAGTGACTG GGGCCCTCGA 120

(2) INFORMATION FOR SEQ ID NO:14:

(i) SEQUENCE CHARACTERISTICS:
(A) LENGTH: 40 amino acids
(B) TYPE: amino acid
(D) TOPOLOGY: linear

(ii) MOLECULE TYPE: protein

(xi) SEQUENCE DESCRIPTION: SEQ ID NO:14:

Ser Leu Ala Thr Tyr Ala Tyr Glu Gly Thr Gly Ser Val Ala Asp Ser
1               5                   10                  15

Leu Ser Ser Leu Glu Ser Val Thr Thr Asp Gly Asp Gln Asp Tyr Asp
            20                  25                  30

Tyr Leu Ser Asp Trp Gly Pro Arg
        35                  40

(2) INFORMATION FOR SEQ ID NO:15:

(i) SEQUENCE CHARACTERISTICS:
(A) LENGTH: 120 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:15:

TCGCTTCAGA CTTATGCATT TGAAGGAAAT GGCTCAGTAG CTGAATCTCT CAGTTCTTTA 60

GATTCTAACA GCTCGAACTC TGATCAGAAT TATGACTACC TTAGTGACTG GGGTCCTCTC 120

(2) INFORMATION FOR SEQ ID NO:16:

(i) SEQUENCE CHARACTERISTICS:
(A) LENGTH: 40 amino acids
(B) TYPE: amino acid
(D) TOPOLOGY: linear

(ii) MOLECULE TYPE: protein

(xi) SEQUENCE DESCRIPTION: SEQ ID NO:16:

Ser Leu Gln Thr Tyr Ala Phe Glu Gly Asn Gly Ser Val Ala Glu Ser
1               5                   10                  15

Leu Ser Ser Leu Asp Ser Asn Ser Ser Asn Ser Asp Gln Asn Tyr Asp
            20                  25                  30

Tyr Leu Ser Asp Trp Gly Pro Arg
        35                  40

(2) INFORMATION FOR SEQ ID NO:17:

(i) SEQUENCE CHARACTERISTICS:
(A) LENGTH: 120 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:17:

TCCATTCAGA TTTATGGCTA TGAAGGCCGA GGGTCTGTGG CTGGCTCTCT CAGCTCGTTG 60

GAGTCCACCA CATCAGACTC AGACCAGAAT TTTGACTACC TCAGTGACTG GGGTCCCCGC 120

(2) INFORMATION FOR SEQ ID NO:18:

(i) SEQUENCE CHARACTERISTICS:
(A) LENGTH: 40 amino acids
(B) TYPE: amino acid
(D) TOPOLOGY: linear

(ii) MOLECULE TYPE: protein

(xi) SEQUENCE DESCRIPTION: SEQ ID NO:18:

Ser Ile Gln Ile Tyr Gly Tyr Glu Gly Arg Gly Ser Val Ala Gly Ser
1               5                   10                  15

Leu Ser Ser Leu Glu Ser Thr Thr Ser Asp Ser Asp Gln Asn Phe Asp
            20                  25                  30

Tyr Leu Ser Asp Trp Gly Pro Arg
        35                  40

(2) INFORMATION FOR SEQ ID NO:19:

(i) SEQUENCE CHARACTERISTICS:
(A) LENGTH: 120 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:19:

TCCTTGGCCA CTTACGCCTA TGAAGGGAAT GATTCTGTAG CCAATTCTCT CAGCTCCTTA 60

GAATCTCTCA CAGCTGATTG TACCCAGGAT TATGACTACC TTAGTGACTG GGGGCCACGC 120

(2) INFORMATION FOR SEQ ID NO:20:

(i) SEQUENCE CHARACTERISTICS:
(A) LENGTH: 40 amino acids
(B) TYPE: amino acid
(D) TOPOLOGY: linear

(ii) MOLECULE TYPE: protein

(xi) SEQUENCE DESCRIPTION: SEQ ID NO:20:

Ser Leu Ala Thr Tyr Ala Tyr Glu Gly Asn Asp Ser Val Ala Asn Ser
1               5                   10                  15

Leu Ser Ser Leu Glu Ser Leu Thr Ala Asp Cys Asn Gln Asp Tyr Asp
            20                  25                  30

Tyr Leu Ser Asp Trp Gly Pro Arg
        35                  40

(2) INFORMATION FOR SEQ ID NO:21:

(i) SEQUENCE CHARACTERISTICS:
(A) LENGTH: 120 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:21:

TCGCTGGCTA CCTATGCCTA TGAAGGAAAC GACTCTGTTG CTGAATCTCT GAGCTCCTTA 60

GAATCAGGTA CCACTGAAGG AGACCAAAAC TACGATTACC TTCGAGAATG GGGGCCTCGG 120

(2) INFORMATION FOR SEQ ID NO:22:

(i) SEQUENCE CHARACTERISTICS:
(A) LENGTH: 40 amino acids
(B) TYPE: amino acid
(D) TOPOLOGY: linear

(ii) MOLECULE TYPE: protein

(xi) SEQUENCE DESCRIPTION: SEQ ID NO:22:

Ser Leu Ala Thr Tyr Ala Tyr Glu Gly Asn Asp Ser Val Ala Glu Ser
1               5                   10                  15

Leu Ser Ser Leu Glu Ser Gly Thr Thr Glu Gly Asp Gln Asn Tyr Asp
            20                  25                  30

Tyr Leu Arg Glu Trp Gly Pro Arg
        35                  40

(2) INFORMATION FOR SEQ ID NO:23:

(i) SEQUENCE CHARACTERISTICS:
(A) LENGTH: 120 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:23:

TCCATCCAAA TCTATGGTTA TGAGGGCAGG GGTTCCGTGG CTGGGTCCCT GAGCTCCTTG 60

GAGTCTGCCA CCACAGATTC GGACCTGGAC TACGACTATC TACAGAACTG GGGACCTCGG 120

(2) INFORMATION FOR SEQ ID NO:24:

(i) SEQUENCE CHARACTERISTICS:
(A) LENGTH: 40 amino acids
(B) TYPE: amino acid
(D) TOPOLOGY: linear

(ii) MOLECULE TYPE: protein

(xi) SEQUENCE DESCRIPTION: SEQ ID NO:24:

Ser Ile Gln Ile Tyr Gly Tyr Glu Gly Arg Gly Ser Val Ala Gly Ser
1               5                   10                  15

Leu Ser Ser Leu Glu Ser Ala Thr Thr Asp Ser Asp Leu Asp Tyr Asp
            20                  25                  30

Tyr Leu Gln Asn Trp Gly Pro Arg
        35                  40

(2) INFORMATION FOR SEQ ID NO:25:

(i) SEQUENCE CHARACTERISTICS:
(A) LENGTH: 150 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:25:

AAGCGGTTTG ATTACGAGAT CTCTGCCTTT CACACCCTGC TGATCAAAGT GGAGAATGAG 60

GACCCATTGG TACCCGACGT CTCCTATGGC CCCAGCTCCA CGGCCACTGT CCACATCACG 120

GTCTTGGATG TCAACGAGGG ACCAGTCTTC 150

(2) INFORMATION FOR SEQ ID NO:26:

(i) SEQUENCE CHARACTERISTICS:
(A) LENGTH: 50 amino acids
(B) TYPE: amino acid
(D) TOPOLOGY: linear

(ii) MOLECULE TYPE: protein

(xi) SEQUENCE DESCRIPTION: SEQ ID NO:26:

Lys Arg Phe Asp Tyr Glu Ile Ser Ala Phe His Thr Leu Leu Ile Lys
1               5                   10                  15

Val Glu Asn Glu Asp Pro Leu Val Pro Asp Val Ser Tyr Gly Pro Ser
            20                  25                  30

Ser Thr Ala Thr Val His Ile Thr Val Leu Asp Val Asn Glu Gly Pro
        35                  40                  45

Val Phe
    50

(2) INFORMATION FOR SEQ ID NO:27:

(i) SEQUENCE CHARACTERISTICS:
(A) LENGTH: 150 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:27:

AAGGGTATGG ATTATGAGCT GAACCGTGCC TCCATGCTGA CCATAATGGT GTCCAACCAG 60

GCGCCCCTGG CCAGCGGGAT CCAGATGTCC TTCCAGTCCA CAGTGGGGGT AACCATCTCT 120

GTCACCGATG TCAACGAAGC CCCCTACTTC 150

(2) INFORMATION FOR SEQ ID NO:28:

(i) SEQUENCE CHARACTERISTICS:
(A) LENGTH: 50 amino acids
(B) TYPE: amino acid
(D) TOPOLOGY: linear

(ii) MOLECULE TYPE: protein

(xi) SEQUENCE DESCRIPTION: SEQ ID NO:28:

Lys Gly Met Asp Tyr Glu Leu Asn Arg Ala Ser Met Leu Thr Ile Met
1               5                   10                  15

Val Ser Asn Gln Ala Pro Leu Ala Ser Gly Ile Gln Met Ser Phe Gln
            20                  25                  30

Ser Thr Val Gly Val Thr Ile Ser Val Thr Asp Val Asn Glu Ala Pro
        35                  40                  45

Tyr Phe
    50

(2) INFORMATION FOR SEQ ID NO:29:

(i) SEQUENCE CHARACTERISTICS:
(A) LENGTH: 153 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:29:

AAACGACTGG ATTTTGAACT CATCCAGCAG TACACGTTCC ACATCGAGGC CACAGACCCC 60

ACTATCAGAC TCGGATACCT GAGCAGCACT GCGGGCAAAA ACAAAGCCAA GATCATCATC 120

AATGTCCTAG ATGTGGATGA GCCCCCTGTT TTC 153

(2) INFORMATION FOR SEQ ID NO:30:

(i) SEQUENCE CHARACTERISTICS:
(A) LENGTH: 51 amino acids
(B) TYPE: amino acid
(D) TOPOLOGY: linear

(ii) MOLECULE TYPE: protein

(xi) SEQUENCE DESCRIPTION: SEQ ID NO:30:

Lys Arg Leu Asp Phe Glu Leu Ile Gln Gln Tyr Thr Phe His Ile Glu
1               5                   10                  15

Ala Thr Asp Pro Thr Ile Arg Leu Gly Tyr Leu Ser Ser Thr Ala Gly
            20                  25                  30

Lys Asn Lys Ala Lys Ile Ile Ile Asn Val Leu Asp Val Asp Glu Pro
        35                  40                  45

Pro Val Phe
    50

(2) INFORMATION FOR SEQ ID NO:31:

    (i) SEQUENCE CHARACTERISTICS:
        (A) LENGTH: 153 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:31:

AAGGGTTTGG ATTTTGAAAA GAAGAAAGTG TATACCCTTA AAGTGGAAGC CTCCAATCCT 60

TATGTTGAGC CACGATTTCT CTACTTGGGG CCTTTCAAAG ATTCAGCCAC GGTTAGAATT 120

GTGGTGGAGG ATGTAGATGA ACCTCCTGCC TTC 153

(2) INFORMATION FOR SEQ ID NO:32:

    (i) SEQUENCE CHARACTERISTICS:
        (A) LENGTH: 51 amino acids
        (B) TYPE: amino acid
        (D) TOPOLOGY: linear

    (ii) MOLECULE TYPE: protein

    (xi) SEQUENCE DESCRIPTION: SEQ ID NO:32:

Lys Gly Leu Asp Phe Glu Lys Lys Lys Val Tyr Thr Leu Lys Val Glu
1               5                   10                  15

Ala Ser Asn Pro Tyr Val Glu Pro Arg Phe Leu Tyr Leu Gly Pro Phe
            20                  25                  30

Lys Asp Ser Ala Thr Val Arg Ile Val Val Glu Asp Val Asp Glu Pro
        35                  40                  45

Pro Ala Phe
    50

(2) INFORMATION FOR SEQ ID NO:33:

    (i) SEQUENCE CHARACTERISTICS:
        (A) LENGTH: 153 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:33:

AAGCCTCTGG ACTTTGAGAC CAAAAAATCC TATACTCTGA AGGTGGAGGC AGCCAATATC 60

CACATCGACC CACGTTTCAG TGGCAGGGGA CCCTTTAAAG ATACAGCAAC AGTCAAAATT 120

GTTGTAGAGG ATGCTGATGA GCCTCCGGTC TTC 153

(2) INFORMATION FOR SEQ ID NO:34:

(i) SEQUENCE CHARACTERISTICS:
(A) LENGTH: 51 amino acids
(B) TYPE: amino acid
(D) TOPOLOGY: linear

(ii) MOLECULE TYPE: protein

(xi) SEQUENCE DESCRIPTION: SEQ ID NO:34:

Asp Ala Leu Asp Phe Glu Thr Lys Lys Ser Tyr Thr Leu Lys Val Glu
1               5                   10                  15

Ala Ala Asn Ile His Ile Asp Pro Arg Phe Ser Gly Arg Gly Pro Phe
            20                  25                  30

Lys Asp Thr Ala Thr Val Lys Ile Val Val Glu Asp Ala Asp Glu Pro
        35                  40                  45

Pro Val Phe
    50

(2) INFORMATION FOR SEQ ID NO:35:

(i) SEQUENCE CHARACTERISTICS:
(A) LENGTH: 152 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:35:

AAGGGGGTGG ACTATGAAGC CAAAACAAGT TATACCCTGC GCATAGAAGC TGCAAATCGA 60

GATGCTGATC CCCGGTTTCT GAGCTTGGGT CCATTCAGTG ACACAACAAC AGTTAAGATA 120

ATTGTGGAAG ACGTGGATGA ACCCCCGTACT C 152

(2) INFORMATION FOR SEQ ID NO:36:

(i) SEQUENCE CHARACTERISTICS:
(A) LENGTH: 51 amino acids
(B) TYPE: amino acid
(D) TOPOLOGY: linear

(ii) MOLECULE TYPE: protein

(xi) SEQUENCE DESCRIPTION: SEQ ID NO:36:

Lys Gly Val Asp Tyr Glu Ala Lys Thr Ser Tyr Thr Leu Arg Ile Glu
1               5                   10                  15

Ala Ala Asn Arg Asp Ala Asp Pro Arg Phe Leu Ser Leu Gly Pro Phe
            20                  25                  30

Ser Asp Thr Thr Thr Val Lys Ile Ile Val Glu Asp Val Asp Glu Pro
        35                  40                  45

Pro Tyr Ser
    50

(2) INFORMATION FOR SEQ ID NO:37:

    (i) SEQUENCE CHARACTERISTICS:
        (A) LENGTH: 153 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:37:

AAGCCACTTG ACTATGAGAA CCGAAGACTA TATACACTGA AGGTGGAGGC AGAAAATACC 60

CATGTGGATC CACGTTTTTA CTATTTAGGG CCATTCAAAG ATACAACAAT TGTAAAAATC 120

TCCATAGAAG ACGTGGATGA GCCACCCCCC TTT 153

(2) INFORMATION FOR SEQ ID NO:38:

    (i) SEQUENCE CHARACTERISTICS:
        (A) LENGTH: 51 amino acids
        (B) TYPE: amino acid
        (D) TOPOLOGY: linear

    (ii) MOLECULE TYPE: protein

    (xi) SEQUENCE DESCRIPTION: SEQ ID NO:38:

Lys Pro Leu Asp Tyr Glu Asn Arg Arg Leu Tyr Thr Leu Lys Val Glu
1               5                   10                  15

Ala Glu Asn Thr His Val Asp Pro Arg Phe Tyr Tyr Leu Gly Pro Phe
            20                  25                  30

Lys Asp Thr Thr Ile Val Lys Ile Ser Ile Glu Asp Val Asp Glu Pro
        35                  40                  45

Pro Pro Phe
    50

(2) INFORMATION FOR SEQ ID NO:39:

    (i) SEQUENCE CHARACTERISTICS:
        (A) LENGTH: 153 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:39:

AGGGGTGTGG ATTATGAAAC CAAAAGAGCA TATAGCTTGA AGGTAGAGGC GGCCAATGTA 60

CACATTGATC CGAAGTTCAT CAGCAATGGA CCTTTCAAGG ACACAGTGAC TGTCAAGATT 120

GCAGTAGAAG ATGCCAATGA GCCCCCTCCC TTC 153

(2) INFORMATION FOR SEQ ID NO:40:

(i) SEQUENCE CHARACTERISTICS:
(A) LENGTH: 51 amino acids
(B) TYPE: amino acid
(D) TOPOLOGY: linear

(ii) MOLECULE TYPE: protein

(xi) SEQUENCE DESCRIPTION: SEQ ID NO:40:

Arg Gly Val Asp Tyr Glu Thr Lys Arg Ala Tyr Ser Leu Lys Val Glu
1               5                   10                  15

Ala Ala Asn Val His Ile Asp Pro Lys Phe Ile Ser Asn Gly Pro Phe
            20                  25                  30

Lys Asp Thr Val Thr Val Lys Ile Ala Val Glu Asp Ala Asn Glu Pro
        35                  40                  45

Pro Pro Phe
    50

(2) INFORMATION FOR SEQ ID NO:41:

(i) SEQUENCE CHARACTERISTICS:
(A) LENGTH: 3136 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:41:

GGCACGAGCG CAAGCCGGGG AGCGCTCGGC CCAGAATTAG TGGATGGATT TGGAATCTCC 60

CTGCCTCCTC CAAGCTCCGC CACTGCCACT TTAGGCAGAG ACCTGAGCGT CAACACGCGA 120

GCCGTACTTT TAGGCTGCGG ACACTGAGCC CAGCGCGCCA GCTTCGCATC TCCGCACCAG 180

GCTCCACAGC TCGGAGAGGC ATGAACGCGA TCCGGAGGAG ACTACCCTGC GCGCGGGGAT 240

CCGTGGACAT TAGCCGCTCT CGGGAACTGA CCCCCAGCTC CTTCAGCCAT TTATGAATCC 300

AGAGGCTTGA GATTTTTTTC CGCATCCCGG AGCCCGACCT GAGAAATTTC AATGAAAAGG 360

AAAGTCAATG GATCGTGGTC TTGGAAAAGC TGCTTAGACA TGTCTGTTTC CCGGCTCTCT 420

GAACCCGTGG CAGAGCTGTA AGTAAGCGCT TCACAGTGCG TGATGAATTG GATGGCTTCG 480

GACCCGAGGC AAAAAAAATA ATTGTCTCAT TTTCGTGCTG ATTTGCTTAA CTGGTGGGAC 540

CATGCCAGAA AGGCTAGCTG AGACGCTTTT GGACCTCTGG ACTCCATTAA TAATATTATG 600

GATTACTCTT CCCTCTTTTG TGTACATGGC TCCGATGAAT CAGGCTCACG TTTTAACTAC 660

TGGATCCCCT TTGGAACTAA GCAGGCAGAG TGAAGAAATG CGGATTTTGA ACCGCTCCAA 720

AAGAGGTTGG GTTTGGAATC AAATGTTTGT TCTGGAAGAA TTTTCTGGAC CTGAACCGAT 780

TCTCGTTGGC CGGTTACACA CAGATCTGGA TCCTGGGAGC AAAAAAATCA AGTATATCCT 840

ATCGGGTGAT GGAGCCGGCA CAATCTTTCA AATAAACGAT ATAACTGGAG ACATCCATGC 900
TATCAAAAGA CTTGACCGAG AGGAAAAGGC TGAGTATACG TTAACAGCTC AGGCAGTGGA 960
CTGGGAGACA AACAAACCTC TCGAGCCTCC TTCTGAATTT ATTATTAAGG TTCAAGACAT 1020
CAACGACAAT GCCCCCGAGT TTCTCAATGG ACCTTACCAT GCTACTGTTC CAGAGATGTC 1080
CATCTTGGGT ACATCTGTCA CTAATGTAAC GGCCACTGAT GCTGACGATC CAGTTTATGG 1140
AAACAGTGCA AAGTTGGTTT ACAGTATCTT GGAGGGACAG CCGTATTTTT CCATTGAGCC 1200
TGAAACAGCT ATTATAAAAA CTGCCCTTCC TAACATGGAC AGAGAGGCCA AGGAGGAATA 1260
CCTGGTTGTA ATTCAAGCCA AAGATATGGG TGGGCATTCC GGTGGTCTGT CTGGAACCAC 1320
GACACTCACA GTGACGCTTA CCGATGTGAA TGACAATCCT CCAAAATTTG CTCAAAGTTT 1380
GTATCACTTC TCAGTACCAG AAGATGTGGT CCTTGGCACT GCAATAGGAA GGGTTAAAGC 1440
CAATGACCAG GATATTGGTG AAAATGCACA ATCTTCCTAT GACATCATTG ATGGAGATGG 1500
GACAGCACTA TTTGAAATCA CTTCTGATGC CCAGGCACAG GATGGTGTTA TAAGACTAAG 1560
AAAGCCTCTG GACTTTGAGA CCAAAAAATC CTATACTCTG AAGGTGGAGG CAGCCAATAT 1620
CCACATCGAC CCACGTTTCA GTGGCAGGGG ACCCTTTAAA GATACAGCAA CAGTCAAAAT 1680
TGTTGTAGAG GATGCTGATG AGCCTCCGGT CTTCTCTTCA CCGACTTACC TCCTTGAAGT 1740
TCATGAAAAT GCTGCCTTGA ACTCTGTGAT TGGCCAAGTG ACAGCTCGTG ACCCTGATAT 1800
CACTTCCAGC CCAATAAGGT TTTCCATTGA CCGCCACACT GACTTGGAGA GACAGTTCAA 1860
CATCAATGCA GATGATGGGA AGATAACACT GGCGACCCCA CTGGACAGAG AACTAAGTGT 1920
GTGGCACAAC ATCTCCATCA TTGCTACTGA GATCAGGAAC CACAGTCAGA TATCGCGAGT 1980
GCCTGTTGCT ATTAAAGTGC TGGATGTCAA TGACAACGCC CCTGAATTCG CGTCCGAATA 2040
TGAGGCATTT TTATGTGAAA ATGGAAAACC CGGCCAAGTC ATTCAAACAG TAAGCGCCAT 2100
GGACAAAGAC GATCCCAAAA ATGGACATTT TTTCTTGTAC AGTCTTCTTC CAGAAATGGT 2160
CAACAACCCA AATTTCACCA TCAAGAAAAA CGAAGATAAT TCCCTGAGCA TTCTGGCAAA 2220
ACATAATGGA TTCAACCGCC AGAAGCAAGA AGTCTACCTT CTGCCTATCG TGATCAGTGA 2280
CAGTGGGAAC CCCCCTCTGA GTAGCACCAG TACCCTGACC ATCCGCGTCT GTGGCTGTAG 2340
CAATGACGGC GTGGTTCAGT CGTGCAATGT CGAAGCTTAT GTCCTTCCTA TTGGGCTCAG 2400
TATGGGCGCG TTAATTGCTA TATTAGCCTG CATCATTTTG CTGCTCGTCA TTGTGGTTCT 2460
GTTCGTTACC CTGAGGCGGC ATAAAAATGA ACCACTAATA ATCAAAGATG ATGAAGACGT 2520
TCGAGAAAAC ATCATTCGCT ACGACGACGA AGGAGGCGGG GAGGAGGACA CAGAGGCTTT 2580
TGACATTGCA ACTTTGCAAA ACCCAGATGG AATTAATGGA TTTTTACCCC GTAAGGATAT 2640
TAAACCAGAT TTGCAGTTTA TGCCAAGGCA AGGGCTTGCT CCAGTTCCAA ATGGTGTTGA 2700
TGTCGATGAA TTTATAAATG TAAGGCTTCA TGAGGCAGAT AATGACCCCA CGGCCCCACC 2760
ATATGACTCC ATTCAGATTT ATGGCTATGA AGGCCGAGGG TCTGTGGCTG GCTCTCTCAG 2820
CTCGTTGGAG TCCACCACAT CAGACTCAGA CCAGAATTTT GACTACCTCA GTGACTGGGG 2880

TCCCCGCTTT AAGAGACTGG GCGAACTCTA CTCTGTTGGT GAAAGTGACA AAGAAACTTG 2940

ACAGTGGATT ACATAAATAA TCAATGGAAC TGAGCATTCT GTAATATTCT AGGGTCACTC 3000

CCCTTAGATG CAACAAATGT GGCTATTTGT TTTAGAGGCA AGTTTAGCAC CAATCATCTA 3060

TAAACTCAAC CACATTTTAA TGTTGAACCA AAAAAAATAA TAAAAAATAA AAAGTATATG 3120

TTAGGAGGTG AAAAAA 3136

(2) INFORMATION FOR SEQ ID NO:42:

(i) SEQUENCE CHARACTERISTICS:
(A) LENGTH: 799 amino acids
(B) TYPE: amino acid
(D) TOPOLOGY: linear

(ii) MOLECULE TYPE: protein

(xi) SEQUENCE DESCRIPTION: SEQ ID NO:42:

Met Pro Glu Arg Leu Ala Glu Thr Leu Leu Asp Leu Trp Thr Pro Leu
1               5                   10                  15

Ile Ile Leu Trp Ile Thr Leu Pro Ser Phe Val Tyr Met Ala Pro Met
            20                  25                  30

Asn Gln Ala His Val Leu Thr Thr Gly Ser Pro Leu Glu Leu Ser Arg
        35                  40                  45

Gln Ser Glu Glu Met Arg Ile Leu Asn Arg Ser Lys Arg Gly Trp Val
    50                  55                  60

Trp Asn Gln Met Phe Val Leu Glu Glu Phe Ser Gly Pro Glu Pro Ile
65                  70                  75                  80

Leu Val Gly Arg Leu His Thr Asp Leu Asp Pro Gly Ser Lys Lys Ile
                85                  90                  95

Lys Tyr Ile Leu Ser Gly Asp Gly Ala Gly Thr Ile Phe Gln Ile Asn
            100                 105                 110

Asp Ile Thr Gly Asp Ile His Ala Ile Lys Arg Leu Asp Arg Glu Glu
        115                 120                 125

Lys Ala Glu Tyr Thr Leu Thr Ala Gln Ala Val Asp Trp Glu Thr Asn
    130                 135                 140

Lys Pro Leu Glu Pro Pro Ser Glu Phe Ile Ile Lys Val Gln Asp Ile
145                 150                 155                 160

Asn Asp Asn Ala Pro Glu Phe Leu Asn Gly Pro Tyr His Ala Thr Val
                165                 170                 175

Pro Glu Met Ser Ile Leu Gly Thr Ser Val Thr Asn Val Thr Ala Thr
            180                 185                 190

Asp Ala Asp Asp Pro Val Tyr Gly Asn Ser Ala Lys Leu Val Tyr Ser
        195                 200                 205

Ile Leu Glu Gly Gln Pro Tyr Phe Ser Ile Glu Pro Glu Thr Ala Ile
    210                 215                 220

Ile Lys Thr Ala Leu Pro Asn Met Asp Arg Glu Ala Lys Glu Glu Tyr
225                 230                 235                 240

Leu Val Val Ile Gln Ala Lys Asp Met Gly Gly His Ser Gly Gly Leu
                245                 250                 255

Ser Gly Thr Thr Thr Leu Thr Val Thr Leu Thr Asp Val Asn Asp Asn
            260                 265                 270

Pro Pro Lys Phe Ala Gln Ser Leu Tyr His Phe Ser Val Pro Glu Asp
        275                 280                 285

Val Val Leu Gly Thr Ala Ile Gly Arg Val Lys Ala Asn Asp Gln Asp
    290                 295                 300

Ile Gly Glu Asn Ala Gln Ser Ser Tyr Asp Ile Ile Asp Gly Asp Gly
305                 310                 315                 320

Thr Ala Leu Phe Glu Ile Thr Ser Asp Ala Gln Ala Gln Asp Gly Val
                325                 330                 335

Ile Arg Leu Arg Lys Pro Leu Asp Phe Glu Thr Lys Lys Ser Tyr Thr
            340                 345                 350

Leu Lys Val Glu Ala Ala Asn Ile His Ile Asp Pro Arg Phe Ser Gly
        355                 360                 365

Arg Gly Pro Phe Lys Asp Thr Ala Thr Val Lys Ile Val Val Glu Asp
    370                 375                 380

Ala Asp Glu Pro Pro Val Phe Ser Ser Pro Thr Tyr Leu Leu Glu Val
385                 390                 395                 400

His Glu Asn Ala Ala Leu Asn Ser Val Ile Gly Gln Val Thr Ala Arg
                405                 410                 415

Asp Pro Asp Ile Thr Ser Ser Pro Ile Arg Phe Ser Ile Asp Arg His
            420                 425                 430

Thr Asp Leu Glu Arg Gln Phe Asn Ile Asn Ala Asp Asp Gly Lys Ile
        435                 440                 445

Thr Leu Ala Thr Pro Leu Asp Arg Glu Leu Ser Val Trp His Asn Ile
    450                 455                 460

Ser Ile Ile Ala Thr Glu Ile Arg Asn His Ser Gln Ile Ser Arg Val
465                 470                 475                 480

Pro Val Ala Ile Lys Val Leu Asp Val Asn Asp Asn Ala Pro Glu Phe
                485                 490                 495

Ala Ser Glu Tyr Glu Ala Phe Leu Cys Glu Asn Gly Lys Pro Gly Gln
            500                 505                 510

Val Ile Gln Thr Val Ser Ala Met Asp Lys Asp Asp Pro Lys Asn Gly
        515                 520                 525

His Phe Phe Leu Tyr Ser Leu Leu Pro Glu Met Val Asn Asn Pro Asn
    530                 535                 540

Phe Thr Ile Lys Lys Asn Glu Asp Asn Ser Leu Ser Ile Leu Ala Lys
545                 550                 555                 560

His Asn Gly Phe Asn Arg Gln Lys Gln Glu Val Tyr Leu Leu Pro Ile
                565                 570                 575

Val Ile Ser Asp Ser Gly Asn Pro Pro Leu Ser Ser Thr Ser Thr Leu
            580                 585                 590

Thr Ile Arg Val Cys Gly Cys Ser Asn Asp Gly Val Val Gln Ser Cys
        595                 600                 605

Asn Val Glu Ala Tyr Val Leu Pro Ile Gly Leu Ser Met Gly Ala Leu
    610                 615                 620

Ile Ala Ile Leu Ala Cys Ile Ile Leu Leu Leu Val Ile Val Val Leu
625                 630                 635                 640

Phe Val Thr Leu Arg Arg His Lys Asn Glu Pro Leu Ile Ile Lys Asp
                645                 650                 655

Asp Glu Asp Val Arg Glu Asn Ile Ile Arg Tyr Asp Asp Glu Gly Gly
            660                 665                 670

Gly Glu Glu Asp Thr Glu Ala Phe Asp Ile Ala Thr Leu Gln Asn Pro
        675                 680                 685

Asp Gly Ile Asn Gly Phe Leu Pro Arg Lys Asp Ile Lys Pro Asp Leu
    690                 695                 700

Gln Phe Met Pro Arg Gln Gly Leu Ala Pro Val Pro Asn Gly Val Asp
705                 710                 715                 720

Val Asp Glu Phe Ile Asn Val Arg Leu His Glu Ala Asp Asn Asp Pro
                725                 730                 735

Thr Ala Pro Pro Tyr Asp Ser Ile Gln Ile Tyr Gly Tyr Glu Gly Arg
            740                 745                 750

Gly Ser Val Ala Gly Ser Leu Ser Ser Leu Glu Ser Thr Thr Ser Asp
        755                 760                 765

Ser Asp Gln Asn Phe Asp Tyr Leu Ser Asp Trp Gly Pro Arg Phe Lys
    770                 775                 780

Arg Leu Gly Glu Leu Tyr Ser Val Gly Glu Ser Asp Lys Glu Thr
785                 790                 795

(2) INFORMATION FOR SEQ ID NO:43:

(i) SEQUENCE CHARACTERISTICS:
(A) LENGTH: 3043 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:43:

GGCACGAGCG CAAGCCGGGG AGCGCTCGGC CCAGAATTAG TGGATGGATT TGGAATCTCC 60

CTGCCTCCTC CAAGCTCCGC CACTGCCACT TTAGGCAGAG ACCTGAGCGT CAACACGCGA 120

GCCGTACTTT TAGGCTGCGG ACACTGAGCC CAGCGCGCCA GCTTCGCATC TCCGCACCAG 180

GCTCCACAGC TCGGAGAGGC ATGAACGCGA TCCGGAGGAG ACTACCCTGC GCGCGGGGAT 240

CCGTGGACAT TAGCCGCTCT CGGGAACTGA CCCCCAGCTC CTTCAGCCAT TTATGAATCC 300

AGAGGCTTGA GATTTTTTTC CGCATCCCGG AGCCCGACCT GAGAAATTTC AATGAAAAGG 360

AAAGTCAATG GATCGTGGTC TTGGAAAAGC TGCTTAGACA TGTCTGTTTC CCGGCTCTCT 420

GAACCCGTGG CAGAGCTGTA AGTAAGCGCT TCACAGTGCG TGATGAATTG GATGGCTTCG 480
GACCCGAGGC AAAAAAAATA ATTGTCTCAT TTTCGTGCTG ATTTGCTTAA CTGGTGGGAC 540
CATGCCAGAA AGGCTAGCTG AGACGCTTTT GGACCTCTGG ACTCCATTAA TAATATTATG 600
GATTACTCTT CCCTCTTTTG TGTACATGGC TCCGATGAAT CAGGCTCACG TTTTAACTAC 660
TGGATCCCCT TTGGAACTAA GCAGGCAGAG TGAAGAAATG CGGATTTTGA ACCGCTCCAA 720
AAGAGGTTGG GTTTGGAATC AAATGTTTGT TCTGGAAGAA TTTTCTGGAC CTGAACCGAT 780
TCTCGTTGGC CGGTTACACA CAGATCTGGA TCCTGGGAGC AAAAAAATCA AGTATATCCT 840
ATCGGGTGAT GGAGCCGGCA CAATCTTTCA AATAAACGAT ATAACTGGAG ACATCCATGC 900
TATCAAAAGA CTTGACCGAG AGGAAAAGGC TGAGTATACG TTAACAGCTC AGGCAGTGGA 960
CTGGGAGACA AACAAACCTC TCGAGCCTCC TTCTGAATTT ATTATTAAGG TTCAAGACAT 1020
CAACGACAAT GCCCCCGAGT TTCTCAATGG ACCTTACCAT GCTACTGTTC CAGAGATGTC 1080
CATCTTGGGT ACATCTGTCA CTAATGTAAC GGCCACTGAT GCTGACGATC CAGTTTATGG 1140
AAACAGTGCA AAGTTGGTTT ACAGTATCTT GGAGGGACAG CCGTATTTTT CCATTGAGCC 1200
TGAAACAGCT ATTATAAAAA CTGCCCTTCC TAACATGGAC AGAGAGGCCA AGGAGGAATA 1260
CCTGGTTGTA ATTCAAGCCA AAGATATGGG TGGGCATTCC GGTGGTCTGT CTGGAACCAC 1320
GACACTCACA GTGACGCTTA CCGATGTGAA TGACAATCCT CCAAAATTTG CTCAAAGTTT 1380
GTATCACTTC TCAGTACCAG AAGATGTGGT CCTTGGCACT GCAATAGGAA GGGTTAAAGC 1440
CAATGACCAG GATATTGGTG AAAATGCACA ATCTTCCTAT GACATCATTG ATGGAGATGG 1500
GACAGCACT[?]A TTTGAAATCA CTTCTGATGC CCAGGCACAG GATGGTGTTA TAAGACTAAG 1560
AAAGCCTCTG GACTTTGAGA CCAAAAAATC CTATACTCTG AAGGTGGAGG CAGCCAATAT 1620
CCACATCGAC CCACGTTTCA GTGGCAGGGG ACCCTTTAAA GATACAGCAA CAGTCAAAAT 1680
TGTTGTAGAG GATGCTGATG AGCCTCCGGT CTTCTCTTCA CCGACTTACC TCCTTGAAGT 1740
TCATGAAAAT GCTGCCTTGA ACTCTGTGAT TGGCCAAGTG ACAGCTCGTG ACCCTGATAT 1800
CACTTCCAGC CCAATAAGGT TTTCCATTGA CCGCCACACT GACTTGGAGA GACAGTTCAA 1860
CATCAATGCA GATGATGGGA AGATAACACT GGCGACCCCA CTGGACAGAG AACTAAGTGT 1920
GTGGCACAAC ATCTCCATCA TTGCTACTGA GATCAGGAAC CACAGTCAGA TATCGCGAGT 1980
GCCTGTTGCT ATTAAAGTGC TGGATGTCAA TGACAACGCC CCTGAATTCG CGTCCGAATA 2040
TGAGGCATTT TTATGTGAAA ATGGAAAACC CGGCCAAGTA AATATCTCCA TGTTGTTAAT 2100
ACTGAATATG TTTGTATACA ACTGTTTCCT AGTTAATTAA CCTGCATTAC TTCCTGATTT 2160
TGCATTGGTT GGATTTACAA AGTCACAGGC AGGAAACTCC TCCAAGCGGT AACAGAAGGG 2220
AATATTTGTC TTTCTCAGAT GTTAATTCTC TTCTAACTTA GGAACCAATT GGCTCAGAAA 2280
GTGTGATGAT CTGCTCTGCT CTGACCCCAG CCAAATCACT GTCTTAAAAT ACATCACATA 2340
TGGGTGATGG CTGGGGACAG TCTTACAGTG CAGAAGGTTG AAATCGCCAT CAATTGGCAA 2400
GAATCTAAAG AATAGCTCAT GGGAAGCATG CATTTTTGTT TTATGTTGAA AAGAAGATTA 2460

ATGCACAAAT GTGGAATGCA AAAAAACACA GTAGTTTATA GAAAGCTCTA TGTAGTGGTA 2520

CTTATGTCTG TACACATATT TGCAAGTTTA GTAAACATAA TGTAGACATC AAATTGTTAG 2580

ATATGCCCCT AAGGCATTTC AATATGTAGA GGTAAGACTC CTAAGGCATA GATGGGGATA 2640

ATGAAGACAA AAATAAAGGG CAGAAAAATG TATAAAATAG AACAGACAGA AATACACTAA 2700

AGATCTAAAG ATAGAAGCAG GAAAGAGGGG AGGGAGGGAG GGAGACAGGG CTGGAAGAAG 2760

ATAGGGTGGG AGGGAGGGAA GGAGAGTCAA GGCTCAGGGT GTGGGGGGGA AGGTAAAATG 2820

CAAAACAAAA TCTACAGAAA CCACTATACT CTGAATGTCA AAATGCAACT AACCTATGTA 2880

AAATCACCCA ACCACATGTG TAATAGATTT ATTTTAACGA GGTGCCGGAG TACTGTATGT 2940

TTAAGAAATT TATCATTTTT CAACTTCCTA ATTTATTTCT GGATGGTGAC ATTTTAATTT 3000

AAATAAACAG CAGCTGACAG CATGAAAAAA AAAAAAAAAA AAA 3043

(2) INFORMATION FOR SEQ ID NO:44:

(i) SEQUENCE CHARACTERISTICS:
(A) LENGTH: 532 amino acids
(B) TYPE: amino acid
(D) TOPOLOGY: linear

(ii) MOLECULE TYPE: protein

(xi) SEQUENCE DESCRIPTION: SEQ ID NO:44:

Met Pro Glu Arg Leu Ala Glu Thr Leu Leu Asp Leu Trp Thr Pro Leu
1               5                   10                  15

Ile Ile Leu Trp Ile Thr Leu Pro Ser Phe Val Tyr Met Ala Pro Met
            20                  25                  30

Asn Gln Ala His Val Leu Thr Thr Gly Ser Pro Leu Glu Leu Ser Arg
        35                  40                  45

Gln Ser Glu Glu Met Arg Ile Leu Asn Arg Ser Lys Arg Gly Trp Val
    50                  55                  60

Trp Asn Gln Met Phe Val Leu Glu Glu Phe Ser Gly Pro Glu Pro Ile
65                  70                  75                  80

Leu Val Gly Arg Leu His Thr Asp Leu Asp Pro Gly Ser Lys Lys Ile
                85                  90                  95

Lys Tyr Ile Leu Ser Gly Asp Gly Ala Gly Thr Ile Phe Gln Ile Asn
            100                 105                 110

Asp Ile Thr Gly Asp Ile His Ala Ile Lys Arg Leu Asp Arg Glu Glu
        115                 120                 125

Lys Ala Glu Tyr Thr Leu Thr Ala Gln Ala Val Asp Trp Glu Thr Asn
    130                 135                 140

Lys Pro Leu Glu Pro Pro Ser Glu Phe Ile Ile Lys Val Gln Asp Ile
145                 150                 155                 160

Asn Asp Asn Ala Pro Glu Phe Leu Asn Gly Pro Tyr His Ala Thr Val
                165                 170                 175

Pro Glu Met Ser Ile Leu Gly Thr Ser Val Thr Asn Val Thr Ala Thr
            180                 185                 190

Asp Ala Asp Asp Pro Val Tyr Gly Asn Ser Ala Lys Leu Val Tyr Ser
        195                 200                 205

Ile Leu Glu Gly Gln Pro Tyr Phe Ser Ile Glu Pro Glu Thr Ala Ile
    210                 215                 220

Ile Lys Thr Ala Leu Pro Asn Met Asp Arg Glu Ala Lys Glu Glu Tyr
225                 230                 235                 240

Leu Val Val Ile Gln Ala Lys Asp Met Gly Gly His Ser Gly Gly Leu
                245                 250                 255

Ser Gly Thr Thr Thr Leu Thr Val Thr Leu Thr Asp Val Asn Asp Asn
            260                 265                 270

Pro Pro Lys Phe Ala Gln Ser Leu Tyr His Phe Ser Val Pro Glu Asp
        275                 280                 285

Val Val Leu Gly Thr Ala Ile Gly Arg Val Lys Ala Asn Asp Gln Asp
    290                 295                 300

Ile Gly Glu Asn Ala Gln Ser Ser Tyr Asp Ile Ile Asp Gly Asp Gly
305                 310                 315                 320

Thr Ala Leu Phe Glu Ile Thr Ser Asp Ala Gln Ala Gln Asp Gly Val
                325                 330                 335

Ile Arg Leu Arg Lys Pro Leu Asp Phe Glu Thr Lys Lys Ser Tyr Thr
            340                 345                 350

Leu Lys Val Glu Ala Ala Asn Ile His Ile Asp Pro Arg Phe Ser Gly
        355                 360                 365

Arg Gly Pro Phe Lys Asp Thr Ala Thr Val Lys Ile Val Val Glu Asp
    370                 375                 380

Ala Asp Glu Pro Pro Val Phe Ser Ser Pro Thr Tyr Leu Leu Glu Val
385                 390                 395                 400

His Glu Asn Ala Ala Leu Asn Ser Val Ile Gly Gln Val Thr Ala Arg
                405                 410                 415

Asp Pro Asp Ile Thr Ser Ser Pro Ile Arg Phe Ser Ile Asp Arg His
            420                 425                 430

Thr Asp Leu Glu Arg Gln Phe Asn Ile Asn Ala Asp Asp Gly Lys Ile
        435                 440                 445

Thr Leu Ala Thr Pro Leu Asp Arg Glu Leu Ser Val Trp His Asn Ile
    450                 455                 460

Ser Ile Ile Ala Thr Glu Ile Arg Asn His Ser Gln Ile Ser Arg Val
465                 470                 475                 480

Pro Val Ala Ile Lys Val Leu Asp Val Asn Asp Asn Ala Pro Glu Phe
                485                 490                 495

Ala Ser Glu Tyr Glu Ala Phe Leu Cys Glu Asn Gly Lys Pro Gly Gln
            500                 505                 510

Val Asn Ile Ser Met Leu Leu Ile Leu Asn Met Phe Val Tyr Asn Cys
        515                 520                 525

Phe Leu Val Asn
    530

(2) INFORMATION FOR SEQ ID NO:45:

(i) SEQUENCE CHARACTERISTICS:
(A) LENGTH: 2490 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:45:

GGCACGAGGG CCAGTTGAGC CAGAGTCAGA ATTTGTGATC AAAATTCACG ATATCAACGA 60
CAATGAGCCT ACATTCCCAG AAGAAATTTA TACAGCCAGC GTTCCTGAAA TGTCTGTTGT 120
AGGTACTTCT GTGGTGCAAG TCACAGCTAC AGATGCCGAT GACCCTTCAT ATGGAAACAG 180
CGCCAGAGTC ATTTACAGCA TACTTCAAGG GCAGCCTTAT TTCTCTGTGG AACCAGAAAC 240
AGGTATCATA AGGACAGCTC TACCAAACAT GAACAGAGAG AACAAGGAAC AGTACCAGGT 300
GGTTATTCAA GCCAAGGACA TGGGCGGTCA GATGGGGGGT CTGTCTGGAA CCACCACAGT 360
GAACATCACT CTCACAGATG TCAACGACAA TCCTCCTCGC TTCCCCCAAA ACACCATCCA 420
TCTGCGAGTT CTTGAATCCT CTCCAGTTGG CACAGCTGTG GGAAGTGTAA AAGCCACCGA 480
TGCTGACACG GGGAAAAATG CCGAAGTGGA TTACCGCATT ATTGATGGAG ATGGCACAGA 540
TATGTTTGAC ATTATAACTG AGAAGGACAC ACAGGAAGGC ATCATCACTG TGAAAAAGCC 600
ACTTGACTAT GAGAACCGAA GACTATATAC TCTGAAGGTG GAGGCAGAAA ATACCCATGT 660
GGATCCACGT TTTTACTATT TAGGGCCATT CAAAGATACA ACAATTGTAA AAATCTCCAT 720
AGAAGACGTG GATGAGCCTC CAGTTTTCAG TCGATCCTCC TATCTGTTTG AGGTTCATGA 780
GGATATTGAA GTGGGCACAA TCATCGGTAC TGTAATGGCA AGAGACCCAG ATTCTACTTC 840
CAGTCCCATC AGATTTACTT TAGATCGCCA TACTGATCTT GACAGGATCT TTAACATTCA 900
TTCTGGAAAC GGATCACTTT ATACATCAAA GCCACTTGAT CGTGAACTAT CTCAATGGCA 960
CAACCTTACC GTCATAGCTG CCGAGATCAA TAATCCTAAA GAAACAACTC GTGTGTCTGT 1020
TTTTGTGAGG ATTTTGGATG TTAATGACAA CGCTCCACAA TTTGCTGTGT TTTATGACAC 1080
ATTTGTATGT GAAAATGCCA GACCAGGACA GCTGATACAG ACAATAAGTG CAGTTGACAA 1140
AGATGACCCC TTAGGTGGAC AGAAGTTCTT CTTCAGTTTG GCTGCTGTGA ATCCTAACTT 1200
CACAGTGCAA GACAATGAAG ACAACACTGC CAGAATTTTA ACCAGAAAGA ATGGCTTCAA 1260
CCGTCATGAA ATAAGCACCT ACCTACTGCC GGTAGTGATA TCTGATAATG ACTACCCCAT 1320
TCAGAGCAGC ACTGGCACCC TGACGATCCG TGTTTGCGCC TGTGACAGCC AGGGCAACAT 1380
GCAGTCCTGC AGTGCCGAAG CCCTGCTCCT TCCTGCTGGC CTCAGCACTG GCGCCTTGAT 1440
CGCCATTCTT CTCTGCATCA TCATTCTGCT GGTTATAGTA GTCCTCTTTG CAGCCCTGAA 1500
AAGGCAACGG AAGAAAGAGC CTCTGATTTT ATCCAAAGAA GACATCAGAG ACAACATTGT 1560
GAGCTATAAC GACGAAGGTG GCGGAGAGGA GGACACCCAA CCCTTTGATA TTGGAACCCT 1620

GAGGAATCCT GCAGCTATCG AGGAGAAAAA GCTGCGGCGA GATATCATTC CTGAAACGTT 1680

ATTTATACCG CGGCGGACTC CTACGGCCCC GGATAACACG GATGTCCGGG ATTTCATTAA 1740

TGAGCGCCTC AAAGAGCACG ACTTGGACCC CACTGCGCCT CCCTACGACT CGCTGGCTAC 1800

CTATGCCTAT GAAGGAAACG ACTCTGTTGC TGAATCTCTG AGCTCCTTAG AATCAGGTAC 1860

CACTGAAGGA GACCAAAACT ACGATTACCT TCGAGAATGG GGGCCTCGGT TTAATAAACT 1920

AGCAGAAATG TACGGTGGTG GTGAGAGCGA CAAAGACGCT TAGCCTGGCC CCTGAGCTCT 1980

GTTCAACGAG ATACGTAACT TTGCAGACAT TGTCTCCACT TCACAATATT TGATATTCAG 2040

GAGAAAAAAT TCCTGCCACT CAGCACAAGT TTCCCACCTA TTTCTTAATT TGTTCATTAA 2100

TTATATTAAT TCCTTCCTGT AGAATGTCTC ATGGGATATA TACGACATTT TATTTAATCA 2160

CTTCCAAGAG CCAAAGCTAT GGAAATTCAA TGTTGCCCAT CTTAGTAAAT AAAAGAAACC 2220

CGAGCAGGAT AGTTCTCCCT TAAGCAACCT CACGAACAAG TCGCTTCTGT TAGATACACG 2280

TCTTGCCCTT GCAAATGAAG CTTTGAAAAG ACGAAGAAAA CATTTAAGAT GTATCCTGTT 2340

CTGTACATTA AGTTTAAAAA AAAAAGTCCA TGTGGTGTTA GTAGGTGTGA TATGCAGCCT 2400

GGTATACGAG CATTCGTGCA ATTTCATTTC ATCAAATTCT ATCTGCTAAT GTTTTATATT 2460

TATATTTTTG TATTTATTTT TTAAAAAAAA 2490

(2) INFORMATION FOR SEQ ID NO:46:

(i) SEQUENCE CHARACTERISTICS:
(A) LENGTH: 653 amino acids
(B) TYPE: amino acid
(D) TOPOLOGY: linear

(ii) MOLECULE TYPE: protein

(xi) SEQUENCE DESCRIPTION: SEQ ID NO:46:

Ala Arg Gly Pro Val Glu Pro Glu Ser Glu Phe Val Ile Lys Ile His
1               5                   10                  15

Asp Ile Asn Asp Asn Glu Pro Thr Phe Pro Glu Glu Ile Tyr Thr Ala
            20                  25                  30

Ser Val Pro Glu Met Ser Val Val Gly Thr Ser Val Val Gln Val Thr
        35                  40                  45

Ala Thr Asp Ala Asp Asp Pro Ser Tyr Gly Asn Ser Ala Arg Val Ile
    50                  55                  60

Tyr Ser Ile Leu Gln Gly Gln Pro Tyr Phe Ser Val Glu Pro Glu Thr
65                  70                  75                  80

Gly Ile Ile Arg Thr Ala Leu Pro Asn Met Asn Arg Glu Asn Lys Glu
                85                  90                  95

Gln Tyr Gln Val Val Ile Gln Ala Lys Asp Met Gly Gly Gln Met Gly
            100                 105                 110

Gly Leu Ser Gly Thr Thr Thr Val Asn Ile Thr Leu Thr Asp Val Asn
        115                 120                 125

Asp Asn Pro Pro Arg Phe Pro Gln Asn Thr Ile His Leu Arg Val Leu
    130                 135                 140

Glu Ser Ser Pro Val Gly Thr Ala Val Gly Ser Val Lys Ala Thr Asp
145                 150                 155                 160

Ala Asp Thr Gly Lys Asn Ala Glu Val Asp Tyr Arg Ile Ile Asp Gly
                165                 170                 175

Asp Gly Thr Asp Met Phe Asp Ile Ile Thr Glu Lys Asp Thr Gln Glu
            180                 185                 190

Gly Ile Ile Thr Val Lys Lys Pro Leu Asp Tyr Glu Asn Arg Arg Leu
        195                 200                 205

Tyr Thr Leu Lys Val Glu Ala Glu Asn Thr His Val Asp Pro Arg Phe
    210                 215                 220

Tyr Tyr Leu Gly Pro Phe Lys Asp Thr Thr Ile Val Lys Ile Ser Ile
225                 230                 235                 240

Glu Asp Val Asp Glu Pro Pro Val Phe Ser Arg Ser Ser Tyr Leu Phe
                245                 250                 255

Glu Val His Glu Asp Ile Glu Val Gly Thr Ile Ile Gly Thr Val Met
            260                 265                 270

Ala Arg Asp Pro Asp Ser Thr Ser Ser Pro Ile Arg Phe Thr Leu Asp
        275                 280                 285

Arg His Thr Asp Leu Asp Arg Ile Phe Asn Ile His Ser Gly Asn Gly
    290                 295                 300

Ser Leu Tyr Thr Ser Lys Pro Leu Asp Arg Glu Leu Ser Gln Trp His
305                 310                 315                 320

Asn Leu Thr Val Ile Ala Ala Glu Ile Asn Asn Pro Lys Glu Thr Thr
                325                 330                 335

Arg Val Ser Val Phe Val Arg Ile Leu Asp Val Asn Asp Asn Ala Pro
            340                 345                 350

Gln Phe Ala Val Phe Tyr Asp Thr Phe Val Cys Glu Asn Ala Arg Pro
        355                 360                 365

Gly Gln Leu Ile Gln Thr Ile Ser Ala Val Asp Lys Asp Asp Pro Leu
    370                 375                 380

Gly Gly Gln Lys Phe Phe Phe Ser Leu Ala Ala Val Asn Pro Asn Phe
385                 390                 395                 400

Thr Val Gln Asp Asn Glu Asp Asn Thr Ala Arg Ile Leu Thr Arg Lys
                405                 410                 415

Asn Gly Phe Asn Arg His Glu Ile Ser Thr Tyr Leu Leu Pro Val Val
            420                 425                 430

Ile Ser Asp Asn Asp Tyr Pro Ile Gln Ser Ser Thr Gly Thr Leu Thr
        435                 440                 445

Ile Arg Val Cys Ala Cys Asp Ser Gln Gly Asn Met Gln Ser Cys Ser
    450                 455                 460

Ala Glu Ala Leu Leu Leu Pro Ala Gly Leu Ser Thr Gly Ala Leu Ile
465                 470                 475                 480

Ala Ile Leu Leu Cys Ile Ile Ile Leu Leu Val Ile Val Val Leu Phe
                485                 490                 495

Ala Ala Leu Lys Arg Gln Arg Lys Lys Glu Pro Leu Ile Leu Ser Lys
            500                     505                     510

Glu Asp Ile Arg Asp Asn Ile Val Ser Tyr Asn Asp Glu Gly Gly Gly
        515                     520                     525

Glu Glu Asp Thr Gln Pro Phe Asp Ile Gly Thr Leu Arg Asn Pro Ala
    530                     535                     540

Ala Ile Glu Glu Lys Lys Leu Arg Arg Asp Ile Ile Pro Glu Thr Leu
545                     550                     555                     560

Phe Ile Pro Arg Arg Thr Pro Thr Ala Pro Asp Asn Thr Asp Val Arg
                565                     570                     575

Asp Phe Ile Asn Glu Arg Leu Lys Glu His Asp Leu Asp Pro Thr Ala
            580                     585                     590

Pro Pro Tyr Asp Ser Leu Ala Thr Tyr Ala Tyr Glu Gly Asn Asp Ser
        595                     600                     605

Val Ala Glu Ser Leu Ser Ser Leu Glu Ser Gly Thr Thr Glu Gly Asp
    610                     615                     620

Gln Asn Tyr Asp Tyr Leu Arg Glu Trp Gly Pro Arg Phe Asn Lys Leu
625                     630                     635                     640

Ala Glu Met Tyr Gly Gly Gly Glu Ser Asp Lys Asp Ala
                645                     650

(2) INFORMATION FOR SEQ ID NO:47:

     (i) SEQUENCE CHARACTERISTICS:
          (A) LENGTH: 3048 base pairs
          (B) TYPE: nucleic acid
          (C) STRANDEDNESS: single
          (D) TOPOLOGY: linear

    (ii) MOLECULE TYPE: cDNA

    (xi) SEQUENCE DESCRIPTION: SEQ ID NO:47:

CGCCGGCGGG GAAGATGACC GCGGGCGCCG GCGTGCTCCT TCTGCTGCTC TCGCTCTCCG      60

GCGCGCTCCG GGCCCATAAT GAGGATCTTA CAACTAGAGA GACCTGCAAG GCTGGGTTCT     120

CTGAAGATGA TTACACGGCA TTAATCTCCC AAAATATTCT AGAAGGGGAA AAGCTACTTC     180

AAGTCAAGTT CAGCAGCTGT GTGGGGACCA AGGGGACACA ATATGAGACC AACAGCATGG     240

ACTTCAAAGT TGGGGCAGAT GGGACAGTCT TCGCCACCCG GGAGCTGCAG GTCCCCTCCG     300

AGCAGGTGGC GTTCACGGTG ACTGCATGGG ACAGCCAGAC AGCAGAGAAA TGGGACGCCG     360

TGGTGCGGTT GCTGGTGGCC CAGACCTCGT CCCCGCACTC TGGACACAAG CCGCAGAAAG     420

GAAAGAAGGT CGTGGCTCTG GACCCCTCTC CGCCTCCGAA GGACACCCTG CTGCCGTGGC     480

CCCAGCACCA GAACGCCAAC GGGCTGAGGC GGCGCAAACG GGACTGGGTC ATCCCACCCA     540

TCAACGTGCC CGAGAACTCG CGCGGGCCCT TCCCGCAGCA GCTCGTGAGG ATCCGGTCCG     600

ACAAAGACAA TGACATCCCC ATCCGGTACA GCATCACGGG AGTGGGTGCC GACCAGCCCC     660

CCATGGAGGT CTTCAGCATT AACTCCATGT CCGGCCGGAT GTACGTCACA AGGCCCATGG     720

ACCGGGAGGA GCACGCCTCT TACCACCTCC GAGCCCACGC TGTGGACATG AATGGCAACA 780
AGGTGGAGAA CCCCATCGAC CTGTACATCT ACGTCATCGA CATGAATGAC AACCACCCTG 840
AGTTCATCAA CCAGGTCTAC AACTGCTCCG TGGACGAGGG CTCCAAGCCA GGCACCTACG 900
TGATGACCAT CACGGCCAAC GATGCTGACG ACAGCACCAC GGCCAACGGG ATGGTGCGGT 960
ACCGGATCGT GACCCAGACC CCACAGAGCC CGTCCCAGAA TATGTTCACC ATCAACAGCG 1020
AGACTGGAGA TATCGTCACA GTGGCGGCTG GCTGGGACCG AGAGAAAGTT CAGCAGTACA 1080
CAGTCATCGT TCAGGCCACA GATATGGAAG GAAATCTCAA CTATGGCCTC TCAAACACAG 1140
CCACAGCCAT CATCACGGTG ACAGATGTGA ATGACAACCC GTCAGAATTT ACCGCCAGCA 1200
CGTTTGCAGG GGAGGTCCCC GAAAACAGCG TGGAGACCGT GGTCGCAAAC CTCACGGTGA 1260
TGGACCGAGA TCAGCCCCAC TCTCCAAACT GGAATGCCGT TTACCGCATC ATCAGTGGGG 1320
ATCCATCCGG GCACTTCAGC GTCCGCACAG ACCCCGTAAC CAACGAGGGC ATGGTCACCG 1380
TGGTGAAGGC AGTCGACTAC GAGCTCAACA GAGCTTTCAT GCTGACAGTG ATGGTGTCCA 1440
ACCAGGCGCC CCTGGCCAGC GGAATCCAGA TGTCCTTCCA GTCCACGGCA GGGGTGACCA 1500
TCTCCATCAT GGACATCAAC GAGGCTCCCT ACTTCCCCTC AAACCACAAG CTGATCCGCC 1560
TGGAGGAGGG CGTGCCCCCC GGCACCGTGC TGACCACGTT TTCAGCTGTG GACCCTGACC 1620
GGTTCATGCA GCAGGCTGTG AGATACTCAA AGCTGTCAGA CCCAGCGAGC TGGCTGCACA 1680
TCAATGCCAC CAACGGCCAG ATCACCACGG TGGCAGTGCT GGACCGTGAG TCCCTCTACA 1740
CCAAAAACAA CGTCTACGAG GCCACCTTCC TGGCAGCTGA CAATGGGATA CCCCCGGCCA 1800
GCGGCACCGG GACCCTCCAG ATCTATCTCA TTGACATCAA CGACAACGCC CCTGAGCTGC 1860
TGCCCAAGGA GGCGCAGATC TGCGAGAGGC CCAACCTGAA CGCCATCAAC ATCACGGCGG 1920
CCGACGCTGA CGTGCACCCC AACATCGGCC CCTACGTCTT CGAGCTGCCC TTTGTCCCGG 1980
CGGCCGTGCG GAAGAACTGG ACCATCACCC GCCTGAACGG TGACTATGCC CAACTCAGCT 2040
TGCGCATCCT GTACCTGGAG GCCGGGATGT ATGACGTCCC CATCATCGTC ACAGACTCTG 2100
GAAACCCTCC CCTGTCCAAC ACGTCCATCA TCAAAGTCAA GGTGTGCCCA TGTGATGACA 2160
ACGGGGACTG CACCACCATT GGCGCAGTGG CAGCGGCTGG TCTGGGCACC GGTGCCATCG 2220
TGGCCATCCT CATCTGCATC CTCATCCTGC TGACCATGGT CCTGCTGTTT GTCATGTGGA 2280
TGAAGCGGCG AGAGAAGGAG CGCCACACGA AGCAGCTGCT CATTGACCCC GAGGACGACG 2340
TCCGCGAAAA GATCCTCAAG TATGACGAGG AAGGCGGTGG CGAGGAGGAC CAGGACTACG 2400
ACCTCAGCCA GCTGCAGCAG CCGGAAGCCA TGGGGCACGT GCCAAGCAAA GCCCCTGGCG 2460
TGCGTCGCGT GGATGAGCGG CCGGTGGGCC CTGAGCCCCA GTACCCGATC AGGCCCATGG 2520
TGCCGCACCC AGGCGACATC GGTGACTTCA TCAATGAGGG ACTCCGCGCT GCTGACAACG 2580
ACCCCACGGC ACCCCCCTAT GACTCCCTGC TGGTCTTCGA CTACGAGGGG AGCGGCTCCA 2640
CCGCAGGCTC CGTCAGCTCC CTGAACTCAT CCAGTTCCGG GGACCAAGAC TACGATTACC 2700
TCAACGACTG GGGCCCCAGA TTCAAGAAGC TGGCGGACAT GTATGGAGGT GGTGAAGAGG 2760

ATTGACTGAC CTCGCATCTT CGGACCGAAG TGAGAGCCGT GCTCGGACGC CGGAGGAGCA 2820

GGACTGAGCA GAGGCGGCCG GTCTTCCCGA CTCCCTGCGG CTGTGTCCTT AGTGCTGTTA 2880

GGAGGCCCCC CAATCCCCAC GTTGAGCTGT CTAGCATGAG CACCCACCCC CACAGCGCCC 2940

TGCACCCGGC CGCTGCCCAG CACCGCGCTG GCTGGCACTG AAGGACAGCA AGAGGCACTC 3000

TGTCTTCACT TGAATTTCCT AGAACAGAAG CACTGTTTTT AAAAAAAG 3048

(2) INFORMATION FOR SEQ ID NO:48:

(i) SEQUENCE CHARACTERISTICS:
(A) LENGTH: 916 amino acids
(B) TYPE: amino acid
(D) TOPOLOGY: linear

(ii) MOLECULE TYPE: protein

(xi) SEQUENCE DESCRIPTION: SEQ ID NO:48:

Met Thr Ala Gly Ala Gly Val Leu Leu Leu Leu Leu Ser Leu Ser Gly
1               5                   10                  15

Ala Leu Arg Ala His Asn Glu Asp Leu Thr Thr Arg Glu Thr Cys Lys
            20                  25                  30

Ala Gly Phe Ser Glu Asp Asp Tyr Thr Ala Leu Ile Ser Gln Asn Ile
        35                  40                  45

Leu Glu Gly Glu Lys Leu Leu Gln Val Lys Phe Ser Ser Cys Val Gly
    50                  55                  60

Thr Lys Gly Thr Gln Tyr Glu Thr Asn Ser Met Asp Phe Leu Val Gly
65                  70                  75                  80

Ala Asp Gly Thr Val Phe Ala Thr Arg Glu Leu Gln Val Pro Ser Glu
                85                  90                  95

Gln Val Ala Phe Thr Val Thr Ala Trp Asp Ser Gln Thr Ala Glu Lys
            100                 105                 110

Trp Asp Ala Val Val Arg Leu Leu Val Ala Gln Thr Ser Ser Pro His
        115                 120                 125

Ser Gly His Lys Pro Gln Lys Gly Lys Lys Val Val Ala Leu Asp Pro
    130                 135                 140

Ser Pro Pro Pro Lys Asp Thr Leu Leu Pro Trp Pro Gln His Gln Asn
145                 150                 155                 160

Ala Asn Gly Leu Arg Arg Arg Lys Arg Asp Trp Val Ile Pro Pro Ile
                165                 170                 175

Asn Val Pro Glu Asn Ser Arg Gly Pro Phe Pro Gln Gln Leu Val Arg
            180                 185                 190

Ile Arg Ser Asp Lys Asp Asn Asp Ile Pro Ile Arg Tyr Ser Ile Thr
        195                 200                 205

Gly Val Gly Ala Asp Gln Pro Pro Met Glu Val Phe Ser Ile Asn Ser
    210                 215                 220

Met Ser Gly Arg Met Tyr Val Thr Arg Pro Met Asp Arg Glu Glu His
225                 230                 235                 240

Ala Ser Tyr His Leu Arg Ala His Ala Val Asp Met Asn Gly Asn Lys
                245                 250                 255

Val Glu Asn Pro Ile Asp Leu Tyr Ile Tyr Val Ile Asp Met Asn Asp
            260                 265                 270

Asn His Pro Glu Phe Ile Asn Gln Val Tyr Asn Cys Ser Val Asp Glu
        275                 280                 285

Gly Ser Lys Pro Gly Thr Tyr Val Met Thr Ile Thr Ala Asn Asp Ala
    290                 295                 300

Asp Asp Ser Thr Thr Ala Asn Gly Met Val Arg Tyr Arg Ile Val Thr
305                 310                 315                 320

Gln Thr Pro Gln Ser Pro Ser Gln Asn Met Phe Thr Ile Asn Ser Glu
                325                 330                 335

Thr Gly Asp Ile Val Thr Val Ala Ala Gly Trp Asp Arg Glu Lys Val
            340                 345                 350

Gln Gln Tyr Thr Val Ile Val Gln Ala Thr Asp Met Glu Gly Asn Leu
        355                 360                 365

Asn Tyr Gly Leu Ser Asn Thr Ala Thr Ala Ile Ile Thr Val Thr Asp
    370                 375                 380

Val Asn Asp Asn Pro Ser Glu Phe Thr Ala Ser Thr Phe Ala Gly Glu
385                 390                 395                 400

Val Pro Glu Asn Ser Val Glu Thr Val Val Ala Asn Leu Thr Val Met
                405                 410                 415

Asp Arg Asp Gln Pro His Ser Pro Asn Trp Asn Ala Val Tyr Arg Ile
            420                 425                 430

Ile Ser Gly Asp Pro Ser Gly His Phe Ser Val Arg Thr Asp Pro Val
        435                 440                 445

Thr Asn Glu Gly Met Val Thr Val Val Lys Ala Val Asp Tyr Glu Leu
    450                 455                 460

Asn Arg Ala Phe Met Leu Thr Val Met Val Ser Asn Gln Ala Pro Leu
465                 470                 475                 480

Ala Ser Gly Ile Gln Met Ser Phe Gln Ser Thr Ala Gly Val Thr Ile
                485                 490                 495

Ser Ile Met Asp Ile Asn Glu Ala Pro Tyr Phe Pro Ser Asn His Lys
            500                 505                 510

Leu Ile Arg Leu Glu Glu Gly Val Pro Pro Gly Thr Val Leu Thr Thr
        515                 520                 525

Phe Ser Ala Val Asp Pro Asp Arg Phe Met Gln Gln Ala Val Arg Tyr
    530                 535                 540

Ser Lys Leu Ser Asp Pro Ala Ser Trp Leu His Ile Asn Ala Thr Asn
545                 550                 555                 560

Gly Gln Ile Thr Thr Val Ala Val Leu Asp Arg Glu Ser Leu Tyr Thr
                565                 570                 575

Lys Asn Asn Val Tyr Glu Ala Thr Phe Leu Ala Ala Asp Asn Gly Ile
            580                 585                 590

Pro Pro Ala Ser Gly Thr Gly Thr Leu Gln Ile Tyr Leu Ile Asp Ile
        595                 600                 605

Asn Asp Asn Ala Pro Glu Leu Leu Pro Lys Glu Ala Gln Ile Cys Glu
    610                 615                 620

Arg Pro Asn Leu Asn Ala Ile Asn Ile Thr Ala Ala Asp Ala Asp Val
625                 630                 635                 640

His Pro Asn Ile Gly Pro Tyr Val Phe Glu Leu Pro Phe Val Pro Ala
                645                 650                 655

Ala Val Arg Lys Asn Trp Thr Ile Thr Arg Leu Asn Gly Asp Tyr Ala
            660                 665                 670

Gln Leu Ser Leu Arg Ile Leu Tyr Leu Glu Ala Gly Met Tyr Asp Val
        675                 680                 685

Pro Ile Ile Val Thr Asp Ser Gly Asn Pro Pro Leu Ser Asn Thr Ser
    690                 695                 700

Ile Ile Lys Val Lys Val Cys Pro Cys Asp Asp Asn Gly Asp Cys Thr
705                 710                 715                 720

Thr Ile Gly Ala Val Ala Ala Ala Gly Leu Gly Thr Gly Ala Ile Val
                725                 730                 735

Ala Ile Leu Ile Cys Ile Leu Ile Leu Leu Thr Met Val Leu Leu Phe
            740                 745                 750

Val Met Trp Met Lys Arg Arg Glu Lys Glu Arg His Thr Lys Gln Leu
        755                 760                 765

Leu Ile Asp Pro Glu Asp Asp Val Arg Glu Lys Ile Leu Lys Tyr Asp
    770                 775                 780

Glu Glu Gly Gly Gly Glu Glu Asp Gln Asp Tyr Asp Leu Ser Gln Leu
785                 790                 795                 800

Gln Gln Pro Glu Ala Met Gly His Val Pro Ser Lys Ala Pro Gly Val
                805                 810                 815

Arg Arg Val Asp Glu Arg Pro Val Gly Pro Glu Pro Gln Tyr Pro Ile
            820                 825                 830

Arg Pro Met Val Pro His Pro Gly Asp Ile Gly Asp Phe Ile Asn Glu
        835                 840                 845

Gly Leu Arg Ala Ala Asp Asn Asp Pro Thr Ala Pro Pro Tyr Asp Ser
    850                 855                 860

Leu Leu Val Phe Asp Tyr Glu Gly Ser Gly Ser Thr Ala Gly Ser Val
865                 870                 875                 880

Ser Ser Leu Asn Ser Ser Ser Ser Gly Asp Gln Asp Tyr Asp Tyr Leu
                885                 890                 895

Asn Asp Trp Gly Pro Arg Phe Lys Lys Leu Ala Asp Met Tyr Gly Gly
            900                 905                 910

Gly Glu Glu Asp
        915

(2) INFORMATION FOR SEQ ID NO:49:

(i) SEQUENCE CHARACTERISTICS:
(A) LENGTH: 3164 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:49:

CTCCACTCAC GCTCAGCCCT GGACGGACAG GCAGTCCAAC GGAACAGAAA CATCCCTCAG 60
CCCACAGGCA CGATCTGTTC CTCCTGGGAA GATGCAGAGG CTATGATGCT CCTCGCCACA 120
TCGGGCGCCT GCCTGGGCCT GCTGGCAGTG GCAGCAGTGG CAGCAGCAGG TGCTAACCCT 180
GCCCAACGGG ACACCCACAG CCTGCTGCCC ACCCACCGGC GCCAAAAGAG AGATTGGATT 240
TGGAACCAGA TGCACATTGA TGAAGAGAAA AACACCTCAC TTCCCCATCA TGTAGGCAAG 300
ATCAAGTCAA GCGTGAGTCG CAAGAATGCC AAGTACCTGC TCAAAGGAGA ATATGTGGGC 360
AAGGTCTTCC GGGTCGATGC AGAGACAGGA GACGTGTTCG CCATTGAGAG GCTGGACCGG 420
GAGAATATCT CAGAGTACCA CCTCACTGCT GTCATTGTGG ACAAGGACAC TGGCGAAAAC 480
CTGGAGACTC CTTCCAGCTT CACCATCAAA GTTCATGACG TGAACGACAA CTGGCCTGTG 540
TTCACGCATC GGTTGTTCAA TGCGTCCGTG CCTGAGTCGT CGGCTGTGGG GACCTCAGTC 600
ATCTCTGTGA CAGCAGTGGA TGCAGACGAC CCCACTGTGG GAGACCACGC CTCTGTCATG 660
TACCAAATCC TGAAGGGGAA AGAGTATTTT GCCATCGATA ATTCTGGACG TATTATCACA 720
ATAACGAAAA GCTTGGACCG AGAGAAGCAG GCCAGGTATG AGATCGTGGT GGAAGCGCGA 780
GATGCCCAGG GCCTCCGGGG GGACTCGGGC ACGGCCACCG TGCTGGTCAC TCTGCAAGAC 840
ATCAATGACA ACTTCCCCTT CTTCACCCAG ACCAAGTACA CATTTGTCGT GCCTGAAGAC 900
ACCCGTGTGG GCACCTCTGT GGGCTCTCTG TTTGTTGAGG ACCCAGATGA GCCCCAGAAC 960
CGGATGACCA AGTACAGCAT CTTGCGGGGC GACTACCAGG ACGCTTTCAC CATTGAGACA 1020
AACCCCGCCC ACAACGAGGG CATCATCAAG CCCATGAAGC CTCTGGATTA TGAATACATC 1080
CAGCAATACA GCTTCATAGT CGAGGCCACA GACCCCACCA TCGACCTCCG ATACATGAGC 1140
CCTCCCGCGG GAAACAGAGC CCAGGTCATT ATCAACATCA CAGATGTGGA CGAGCCCCCC 1200
ATTTTCCAGC AGCCTTTCTA CCACTTCCAG CTGAAGGAAA ACCAGAAGAA GCCTCTGATT 1260
GGCACAGTGC TGGCCATGGA CCCTGATGCG GCTAGGCATA GCATTGGATA CTCCATCCGC 1320
AGGACCAGTG ACAAGGGCCA GTTCTTCCGA GTCACAAAAA AGGGGGACAT TTACAATGAG 1380
AAAGAACTGG ACAGAGAAGT CTACCCCTGG TATAACCTGA CTGTGGAGGC CAAAGAACTG 1440
GATTCCACTG GAACCCCCAC AGGAAAAGAA TCCATTGTGC AAGTCCACAT TGAAGTTTTG 1500
GATGAGAATG ACAATGCCCC GGAGTTTGCC AAGCCCTACC AGCCCAAAGT GTGTGAGAAC 1560
GCTGTCCATG GCCAGCTGGT CCTGCAGATC TCCGCAATAG ACAAGGACAT AACACCACGA 1620

AACGTGAAGT TCAAATTCAT CTTGAATACT GAGAACAACT TTACCCTCAC GGATAATCAC 1680
GATAACACGG CCAACATCAC AGTCAAGTAT GGGCAGTTTG ACCGGGAGCA TACCAAGGTC 1740
CACTTCCTAC CCGTGGTCAT CTCAGACAAT GGGATGCCAA GTCGCACGGG CACCAGCACG 1800
CTGACCGTGG CCGTGTGCAA GTGCAACGAG CAGGGCGAGT TCACCTTCTG CGAGGATATG 1860
GCCGCCCAGG TGGGCGTGAG CATCCAGGCA GTGGTAGCCA TCTTACTCTG CATCCTCACC 1920
ATCACAGTGA TCACCCTGCT CATCTTCCTG CGGCGGCGGC TCCGGAAGCA GGCCCGCGCG 1980
CACGGCAAGA GCGTGCCGGA GATCCACGAG CAGCTGGTCA CCTACGACGA GGAGGGCGGC 2040
GGCGAGATGG ACACCACCAG CTACGATGTG TCGGTGCTCA ACTCGGTGCG CCGCGGCGGG 2100
GCCAAGCCCC CGCGGCCCGC GCTGGACGCC CGGCCTTCCC TCTATGCGCA GGTGCAGAAG 2160
CCACCGAGGC ACGCGCCTGG GGCACACGGA GGGCCCGGGG AGATGGCAGC CATGATCGAG 2220
GTGAAGAAGG ACGAGGCGGA CCACGACGGC GACGGCCCCC CCTACGACAC GCTGCACATC 2280
TACGGCTACG AGGGCTCCGA GTCCATAGCC GAGTCCCTCA GCTCCCTGGG CACCGACTCA 2340
TCCGACTCTG ACGTGGATTA CGACTTCCTT AACGACTGGG GACCCAGGTT TAAGATGCTG 2400
GCTGAGCTGT ACGGCTCGGA CCCCCGGGAG GAGCTGCTGT ATTAGGCGGC CGAGGTCACT 2460
CTGGGCCTGG GGACCCAAAC CCCCTGCAGC CCAGGCCAGT CAGACTCCAG GCACCACAGC 2520
CTCCAAAAAT GGCAGTGACT CCCCAGCCCA GCACCCCTTC CTCGTGGGTC CCAGAGACCT 2580
CATCAGCCTT GGGATAGCAA ACTCCAGGTT CCTGAAATAT CCAGGAATAT ATGTCAGTGA 2640
TGACTATTCT CAAATGCTGG CAAATCCAGG CTGGTGTTCT GTCTGGGCTC AGACATCCAC 2700
ATAACCCTGT CACCCACAGA CCGCCGTCTA ACTCAAAGAC TTCCTCTGGC TCCCCAAGGC 2760
TGCAAAGCAA AACAGACTGT GTTTAACTGC TGCAGGGTCT TTTTCTAGGG TCCCTGAACG 2820
CCCTGGTAAG GCTGGTGAGG TCCTGGTGCC TATCTGCCTG GAGGCAAAGG CCTGGACAGC 2880
TTGACTTGTG GGGCAGGATT CTCTGCAGCC CATTCCCAAG GGAGACTGAC CATCATGCCC 2940
TCTCTCGGGA GCCCTAGCCC TGCTCCAACT CCATACTCCA CTCCAAGTGC CCCACCACTC 3000
CCCAACCCCT CTCCAGGCCT GTCAAGAGGG AGGAAGGGGC CCCATGGCAG CTCCTGACCT 3060
TGGGTCCTGA AGTGACCTCA CTGGCCTGCC ATGCCAGTAA CTGTGCTGTA CTGAGCACTG 3120
AACCACATTC AGGGAAATGG CTTATTAAAC TTTGAAGCAA CTGT 3164

(2) INFORMATION FOR SEQ ID NO:50:

(i) SEQUENCE CHARACTERISTICS:
(A) LENGTH: 780 amino acids
(B) TYPE: amino acid
(D) TOPOLOGY: linear

(ii) MOLECULE TYPE: protein

(xi) SEQUENCE DESCRIPTION: SEQ ID NO:50:

Met Met Leu Leu Ala Thr Ser Gly Ala Cys Leu Gly Leu Leu Ala Val
1               5                   10                  15

Ala Ala Val Ala Ala Ala Gly Ala Asn Pro Ala Gln Arg Asp Thr His
            20                  25                  30

Ser Leu Leu Pro Thr His Arg Arg Gln Lys Arg Asp Trp Ile Trp Asn
        35                  40                  45

Gln Met His Ile Asp Glu Glu Lys Asn Thr Ser Leu Pro His His Val
    50                  55                  60

Gly Lys Ile Lys Ser Ser Val Ser Arg Lys Asn Ala Lys Tyr Leu Leu
65                  70                  75                  80

Lys Gly Glu Tyr Val Gly Lys Val Phe Arg Val Asp Ala Glu Thr Gly
                85                  90                  95

Asp Val Phe Ala Ile Glu Arg Leu Asp Arg Glu Asn Ile Ser Glu Tyr
            100                 105                 110

His Leu Thr Ala Val Ile Val Asp Lys Asp Thr Gly Glu Asn Leu Glu
        115                 120                 125

Thr Pro Ser Ser Phe Thr Ile Lys Val His Asp Val Asn Asp Asn Trp
    130                 135                 140

Pro Val Phe Thr His Arg Leu Phe Asn Ala Ser Val Pro Glu Ser Ser
145                 150                 155                 160

Ala Val Gly Thr Ser Val Ile Ser Val Thr Ala Val Asp Ala Asp Asp
                165                 170                 175

Pro Thr Val Gly Asp His Ala Ser Val Met Tyr Gln Ile Leu Lys Gly
            180                 185                 190

Lys Glu Tyr Phe Ala Ile Asp Asn Ser Gly Arg Ile Ile Thr Ile Thr
        195                 200                 205

Lys Ser Leu Asp Arg Glu Lys Gln Ala Arg Tyr Glu Ile Val Val Glu
    210                 215                 220

Ala Arg Asp Ala Gln Gly Leu Arg Gly Asp Ser Gly Thr Ala Thr Val
225                 230                 235                 240

Leu Val Thr Leu Gln Asp Ile Asn Asp Asn Phe Pro Phe Phe Thr Gln
                245                 250                 255

Thr Lys Tyr Thr Phe Val Val Pro Glu Asp Thr Arg Val Gly Thr Ser
            260                 265                 270

Val Gly Ser Leu Phe Val Glu Asp Pro Asp Glu Pro Gln Asn Arg Met
        275                 280                 285

Thr Lys Tyr Ser Ile Leu Arg Gly Asp Tyr Gln Asp Ala Phe Thr Ile
    290                 295                 300

Glu Thr Asn Pro Ala His Asn Glu Gly Ile Ile Lys Pro Met Lys Pro
305                 310                 315                 320

Leu Asp Tyr Glu Tyr Ile Gln Gln Tyr Ser Phe Ile Val Glu Ala Thr
                325                 330                 335

Asp Pro Thr Ile Asp Leu Arg Tyr Met Ser Pro Pro Ala Gly Asn Arg
            340                 345                 350

Ala Gln Val Ile Ile Asn Ile Thr Asp Val Asp Glu Pro Pro Ile Phe
        355                 360                 365

Gln Gln Pro Phe Tyr His Phe Gln Leu Lys Glu Asn Gln Lys Lys Pro
    370                 375                 380
Leu Ile Gly Thr Val Leu Ala Met Asp Pro Asp Ala Ala Arg His Ser
385                 390                 395                 400
Ile Gly Tyr Ser Ile Arg Arg Thr Ser Asp Lys Gly Gln Phe Phe Arg
                405                 410                 415
Val Thr Lys Lys Gly Asp Ile Tyr Asn Glu Lys Glu Leu Asp Arg Glu
            420                 425                 430
Val Tyr Pro Trp Tyr Asn Leu Thr Val Glu Ala Lys Glu Leu Asp Ser
        435                 440                 445
Thr Gly Thr Pro Thr Gly Lys Glu Ser Ile Val Gln Val His Ile Glu
    450                 455                 460
Val Leu Asp Glu Asn Asp Asn Ala Pro Glu Phe Ala Lys Pro Tyr Gln
465                 470                 475                 480
Pro Lys Val Cys Glu Asn Ala Val His Gly Gln Leu Val Leu Gln Ile
                485                 490                 495
Ser Ala Ile Asp Lys Asp Ile Thr Pro Arg Asn Val Lys Phe Lys Phe
            500                 505                 510
Ile Leu Asn Thr Glu Asn Asn Phe Thr Leu Thr Asp Asn His Asp Asn
        515                 520                 525
Thr Ala Asn Ile Thr Val Lys Tyr Gly Gln Phe Asp Arg Glu His Thr
    530                 535                 540
Lys Val His Phe Leu Pro Val Val Ile Ser Asp Asn Gly Met Pro Ser
545                 550                 555                 560
Arg Thr Gly Thr Ser Thr Leu Thr Val Ala Val Cys Lys Cys Asn Glu
                565                 570                 575
Gln Gly Glu Phe Thr Phe Cys Glu Asp Met Ala Ala Gln Val Gly Val
            580                 585                 590
Ser Ile Gln Ala Val Val Ala Ile Leu Leu Cys Ile Leu Thr Ile Thr
        595                 600                 605
Val Ile Thr Leu Leu Ile Phe Leu Arg Arg Arg Leu Arg Leu Gln Ala
    610                 615                 620
Arg Ala His Gly Lys Ser Val Pro Glu Ile His Glu Gln Leu Val Thr
625                 630                 635                 640
Tyr Asp Glu Glu Gly Gly Gly Glu Met Asp Thr Thr Ser Tyr Asp Val
                645                 650                 655
Ser Val Leu Asn Ser Val Arg Arg Gly Gly Ala Lys Pro Pro Arg Pro
            660                 665                 670
Ala Leu Asp Ala Arg Pro Ser Leu Tyr Ala Gln Val Gln Lys Pro Pro
        675                 680                 685
Arg His Ala Pro Gly Ala His Gly Gly Pro Gly Glu Met Ala Ala Met
    690                 695                 700
Ile Glu Val Lys Lys Asp Glu Ala Asp His Asp Gly Asp Gly Pro Pro
705                 710                 715                 720

Tyr Asp Thr Leu His Ile Tyr Gly Tyr Glu Gly Ser Glu Ser Ile Ala
                725                 730                 735

Glu Ser Leu Ser Ser Leu Gly Thr Asp Ser Ser Asp Ser Asp Val Asp
            740                 745                 750

Tyr Asp Phe Leu Asn Asp Trp Gly Pro Arg Phe Lys Met Leu Ala Glu
        755                 760                 765

Leu Tyr Gly Ser Asp Pro Arg Glu Glu Leu Leu Tyr
    770                 775                 780

(2) INFORMATION FOR SEQ ID NO:51:

(i) SEQUENCE CHARACTERISTICS:
(A) LENGTH: 1369 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:51:

TGTAGATGAG CCACCTGTCT TCAGCAAACT GGCCTACATC TTACAAATAA GAGAAGATGC 60

TCAGATAAAC ACCACAATAG GCTCCGTCAC AGCCCAAGAT CCAGATGCTG CCAGGAATCC 120

TGTCAAGTAC TCTATAGATC GACACACAGA TATGGACAGA ATATTCAACA TTGATTCTGG 180

AAATGGTTCG ATTTTTACAT CGAAACTTCT TGACCGAGAA ACACTGCTAT GGCACAACAT 240

TACAGTGATA GCAACAGAGA TCAATAATCC AAAGCAAAGT AGTCGAGTAC CTCTATATAT 300

TAAAGTTCTA GATGTCAATG ACAACGCCCC AGAATTTGCT GAGTTCTATG AAACTTTTGT 360

CTGTGAAAAA GCAAAGGCAG ATCAGTTGAT TCAGACCTTG CATGCTGTTA GCAAGGATGA 420

CCCTTATAGT GGGCACCAAT TTTCGTTTTC CTTGGCCCCT GAAGCAGCCA GTGGCTCAAA 480

CTTTACCATT CAAGACAACA AAGACAACAC GGCGGGAATC TTAACTCGGA AAAATGGCTA 540

TAATAGACAC GAGATGAGCA CCTATCTCTT GCCTGTGGTC ATTTCAGACA ACGACTACCC 600

AGTTCAAAGC AGCACTGGGA CAGTGACTGT CCGGGTCTGT GCATGTGACC ACCACGGGAA 660

CATGCAATCC TGCCATGCGG AGGCGCTCAT CCACCCCACG GGACTGAGCA CGGGGGCTCT 720

GGTTGCCATC CTTCTGTGCA TCGTGATCCT ACTAGTGACA GTGGTGCTGT TTGCAGCTCT 780

GAGGCGGCAG CGAAAAAAAG AGCCTTTGAT CATTTCCAAA GAGGACATCA GAGATAACAT 840

TGTCAGTTAC AACGACGAAG GTGGTGGAGA GGAGGACACC CAGGCTTTTG ATATCGGCAC 900

CCTGAGGAAT CCTGAAGCCA TAGAGGACAA CAAATTACGA AGGGACATTG TGCCCGAAGC 960

CCTTTTCCTA CCCCGACGGA CTCCAACAGC TCGCGACAAC ACCGATGTCA GAGATTTCAT 1020

TAACCAAAGG TTAAAGGAAA ATGACACGGA CCCCACTGCC CCGCCATACG ACTCCCTGGC 1080

CACTTACGCC TATGAAGGCA CTGGCTCCGT GGCGGATTCC CTGAGCTCGC TGGAGTCAGT 1140

GACCACGGAT GCAGATCAAG ACTATGATTA CCTTTAGTGA CTGGGACCTC GATTCAAAAA 1200

GCTTGCAGAT ATGTATGGAG GAGTGGACAG TGACAAAGAC TCCTAATCTG TTGCCTTTTT 1260

CATTTTCCAA TACGACACTG AAATATGTGA AGTGGCTATT TCTTTATATT TATCCACTAC 1320

TCCGTGAAGG CTTCTCTGTT CTACCCGTTC CAAAAGCCAA TGGCTGCAG 1369

(2) INFORMATION FOR SEQ ID NO:52:

(i) SEQUENCE CHARACTERISTICS:
(A) LENGTH: 414 amino acids
(B) TYPE: amino acid
(D) TOPOLOGY: linear

(ii) MOLECULE TYPE: protein

(xi) SEQUENCE DESCRIPTION: SEQ ID NO:52:

Val Asp Glu Pro Pro Val Phe Ser Lys Leu Ala Tyr Ile Leu Gln Ile
1               5                   10                  15

Arg Glu Asp Ala Gln Ile Asn Thr Thr Ile Gly Ser Val Thr Ala Gln
            20                  25                  30

Asp Pro Asp Ala Ala Arg Asn Pro Val Lys Tyr Ser Ile Lys Arg His
        35                  40                  45

Thr Asp Met Asp Arg Ile Phe Asn Ile Asp Ser Gly Asn Gly Ser Ile
    50                  55                  60

Phe Thr Ser Lys Leu Leu Lys Arg Glu Thr Leu Leu Trp His Asn Ile
65                  70                  75                  80

Thr Val Ile Ala Thr Glu Ile Asn Asn Pro Lys Gln Ser Ser Arg Val
                85                  90                  95

Pro Leu Tyr Ile Lys Val Leu Asp Val Asn Asp Asn Ala Pro Glu Phe
            100                 105                 110

Ala Glu Phe Tyr Glu Thr Phe Val Cys Glu Lys Ala Lys Ala Asp Gln
        115                 120                 125

Leu Ile Gln Thr Leu His Ala Val Asp Lys Asp Asp Pro Tyr Ser Gly
    130                 135                 140

His Gln Phe Ser Phe Ser Leu Ala Pro Glu Ala Ala Ser Gly Ser Asn
145                 150                 155                 160

Phe Thr Ile Gln Asp Asn Lys Asp Asn Thr Ala Gly Ile Leu Thr Arg
                165                 170                 175

Lys Asn Gly Tyr Asn Arg His Glu Met Ser Thr Tyr Leu Leu Pro Val
            180                 185                 190

Val Ile Ser Asp Asn Asp Tyr Pro Val Gln Ser Ser Thr Gly Thr Val
        195                 200                 205

Thr Val Arg Val Cys Ala Cys Asp His His Gly Asn Met Gln Ser Cys
    210                 215                 220

His Ala Glu Ala Leu Ile His Pro Thr Gly Leu Ser Thr Gly Ala Leu
225                 230                 235                 240

Val Ala Ile Leu Leu Cys Ile Val Ile Leu Leu Val Thr Val Val Leu
                245                 250                 255

Phe Ala Ala Leu Arg Arg Gln Arg Lys Lys Glu Pro Leu Ile Ile Ser
            260                 265                 270

Lys Glu Asp Ile Arg Asp Asn Ile Val Ser Tyr Asn Asp Glu Gly Gly
        275                 280                 285

Gly Glu Glu Asp Thr Gln Ala Phe Asp Ile Gly Thr Leu Arg Asn Pro
    290                 295                 300

Glu Ala Ile Glu Asp Asn Lys Leu Arg Arg Asp Ile Val Pro Glu Ala
305                 310                 315                 320

Leu Phe Leu Pro Arg Arg Thr Pro Thr Ala Arg Asp Asn Thr Asp Val
                325                 330                 335

Arg Asp Phe Ile Asn Gln Arg Leu Lys Glu Asn Asp Thr Asp Pro Thr
            340                 345                 350

Ala Pro Pro Tyr Asp Ser Leu Ala Thr Tyr Ala Tyr Glu Gly Thr Gly
        355                 360                 365

Ser Val Ala Asp Ser Leu Ser Ser Leu Glu Ser Val Thr Thr Asp Ala
    370                 375                 380

Asp Gln Asp Tyr Asp Tyr Leu Ser Asp Trp Gly Pro Arg Phe Lys Lys
385                 390                 395                 400

Leu Ala Asp Met Tyr Gly Gly Val Asp Ser Asp Lys Asp Ser
                405                 410

(2) INFORMATION FOR SEQ ID NO:53:

     (i) SEQUENCE CHARACTERISTICS:
          (A) LENGTH: 2550 base pairs
          (B) TYPE: nucleic acid
          (C) STRANDEDNESS: single
          (D).TOPOLOGY: linear

    (ii) MOLECULE TYPE: cDNA


    (xi) SEQUENCE DESCRIPTION: SEQ ID NO:53:

CAGGAAATGC TCTTGGATCT CTGGACTCCA TTAATAATAT TATGGATTAC TCTTCCCCCT      60

TGCATTTACA TGGCTCCGAT GAATCAGTCT CAAGTTTTAA TGAGTGGATC CCCTTTGGAA     120

CTAAACAGTC TGGGTGAAGA ACAGCGAATT TTGAACCGCT CCAAAAGAGG CTGGGTTTGG     180

AATCAAATGT TTGTCCTGGA AGAGTTTTCT GGACCTGAAC CGATTCTTGT TGGCCGGCTA     240

CACACAGACC TGGATCCTGG GAGCAAAAAA ATCAAGTATA TCCTATCAGG TGATGGAGCT     300

GGGACCATAT TTCAAATAAA TGATGTAACT GGAGATATCC ATGCTATAAA AAGACTTGAC     360

CGGGAGGAAA AGGCTGAGTA TACCCTAACA GCTCAAGCAG TGGACTGGGA GACAAGCAAA     420

CCTCTGGAGC CTCCTTCTGA ATTTATTATT AAAGTTCAAG ACATCAATGA CAATGCACCA     480

GAGTTTCTTA ATGGACCCTA TCATGCTACT GTGCCAGAAA TGTCCATTTT GGGTACATCT     540

GTCACTAACG TCACTGCGAC CGACGCTGAT GACCCAGTTT ATGGAAACAG TGCAAAGTTG     600

GTTTATAGTA TATTGGAAGG GCAGCCTTAT TTTTCCATTG AGCCTGAAAC AGCTATTATA     660

AAAACTGCCC TTCCCAACAT GGACAGAGAA GCCAAGGAGG AGTACCTGGT TGTTATCCAA     720

GCCAAAGATA TGGGTGGACA CTCTGGTGGC CTGTCTGGGA CCACGACACT TACAGTGACT     780

CTTACTGATG TTAATGACAA TCCTCCAAAA TTTGCACAGA GCCTGTATCA CTTCTCAGTA 840
CCGGAAGATG TGGTTCTTGG CACTGCAATA GGAAGGGTGA AGGCCAATGA TCAGGATATT 900
GGTGAAAATG CACAGTCATC ATATGATATC ATCGATGGAG ATGGAACAGC ACTTTTTGAA 960
ATCACTTCTG ATGCCCAGGC CCAGGATGGC ATTATAAGGC TAAGAAAACC TCTGGACTTT 1020
GAGACCAAAA AATCCTATAC GCTAAAGGAT GAGGCAGCCA ATGTCCATAT TGACCCACGC 1080
TTCAGTGGCA GGGGGCCCTT TAAAGACACG GCGACAGTCA AAATCGTGGT TGAAGATGCT 1140
GATGAGCCTC CGGTCTTCTC TTCACCGACT TACCTACTTG AAGTTCATGA AAATGCTGCT 1200
CTAAACTCCG TGATTGGGCA AGTGACTGCT CGTGACCCTG ATATCACTTC CAGTCCTATA 1260
AGGTTTTCCA TCGACCGGCA CACTGACCTG GAGAGGCAGT TCAACATTAA TGCAGACGAT 1320
GGGAAGATAA CGCTGGCAAC ACCACTTGAC AGAGAATTAA GTGTATGGCA CAACATAACA 1380
ATCATTGCTA CTGAAATTAG GAACCACAGT CAGATATCAC GAGTACCTGT TGCTATTAAA 1440
GTGCTGGATG TCAATGACAA CGCCCCTGAA TTCGCATCCG AATATGAGGC ATTTTTATGT 1500
GAAAATGGAA AACCCGGCCA AGTCATTCAA ACTGTTAGCG CCATGGACAA AGATGATCCC 1560
AAAAACGGAC ATTATTTCTT ATACAGTCTC CTTCCAGAAA TGGTCAACAA TCCGAATTTC 1620
ACCATCAAGA AAAATGAAGA TAATTCCCTC AGTATTTTGG CAAAGCATAA TGGATTCAAC 1680
CGCCAGAAGC AAGAAGTCTA TCTTTTACCA ATCATAATCA GTGATAGTGG AAATCCTCCA 1740
CTGAGCAGCA CTAGCACCTT GACAATCAGG GTCTGTGGCT GCAGCAATGA CGGTGTCGTC 1800
CAGTCTTGCA ATGTCGAAGC TTATGTCCTT CCAATTGGAC TCAGTATGGG CGCCTTAATT 1860
GCCATATTAG CATGCATCAT TTTGCTGTTA GTCATCGTGG TGCTGTTTGT AACTCTACGG 1920
CGGCATCAAA AAAATGAACC ATTAATTATC AAAGATGATG AAGACGTTCG AGAAAACATC 1980
ATTCGCTACG ATGATGAAGG AGGAGGGGAG GAGGACACAG AGGCTTTTGA CATTGCAACT 2040
TTACAAAATC CAGATGGAAT TAATGGATTT TTACCCCGTA AGGATATTAA ACCAGATTTG 2100
CAGTTTATGC CAAGGCAAGG GCTTGCTCCA GTTCCAAATG GTGTTGATGT CGATGAATTT 2160
ATAAATGTAA GGCTGCATGA GGCAGATAAT GATCCCACAG CCCCGCCATA TGACTCCATT 2220
CAAATATATG GCTATGAAGG CCGAGGGTCA GTGGCTGGCT CCCTCAGCTC CTTGGAGTCC 2280
ACCACATCAG ACTCAGACCA GAATTTTGAC TACCTCAGTG ACTGGGGTCC CCGCTTTAAG 2340
AGACTGGGCG AACTCTACTC TGTTGGTGAA AGTGACAAAG AAACTTGACA GTGGATTATA 2400
AATAAATCAC TGGAACTGAG CATTCTGTAA TATTCTAGGG TCACTCCCCT TAGATACAAC 2460
CAATGTGGCT ATTTGTTTAG AGGCAAGTTT AGCACCAGTC ATCTATAACT CAACCACATT 2520
TAATGTTGAC AAAAAGATAA TAAATAAAAA 2550

(2) INFORMATION FOR SEQ ID NO:54:

(i) SEQUENCE CHARACTERISTICS:
(A) LENGTH: 793 amino acids
(B) TYPE: amino acid
(D) TOPOLOGY: linear

(ii) MOLECULE TYPE: protein

(xi) SEQUENCE DESCRIPTION: SEQ ID NO:54:

Met Leu Leu Asp Leu Trp Thr Pro Leu Ile Ile Leu Trp Ile Thr Leu
1               5                   10                  15

Pro Pro Cys Ile Tyr Met Ala Pro Met Asn Gln Ser Gln Val Leu Met
            20                  25                  30

Ser Gly Ser Pro Leu Gln Leu Asn Ser Leu Gly Glu Glu Gln Arg Ile
        35                  40                  45

Leu Asn Arg Ser Lys Arg Gly Trp Val Trp Asn Gln Met Phe Val Leu
    50                  55                  60

Glu Glu Phe Ser Gly Pro Glu Pro Ile Leu Val Gly Arg Leu His Thr
65                  70                  75                  80

Asp Leu Asp Pro Gly Ser Lys Lys Ile Lys Tyr Ile Leu Ser Gly Asp
                85                  90                  95

Gly Ala Gly Thr Ile Phe Gln Ile Asn Asp Val Thr Gly Asp Ile His
            100                 105                 110

Ala Ile Lys Arg Leu Asp Arg Glu Glu Lys Ala Glu Tyr Thr Leu Thr
        115                 120                 125

Ala Gln Ala Val Asp Trp Glu Thr Ser Lys Pro Leu Glu Pro Pro Ser
    130                 135                 140

Glu Phe Ile Ile Lys Val Gln Asp Ile Asn Asp Asn Ala Pro Glu Phe
145                 150                 155                 160

Leu Asn Gly Pro Tyr His Ala Thr Val Pro Glu Met Ser Ile Leu Gly
                165                 170                 175

Thr Ser Val Thr Asn Val Thr Ala Thr Asp Ala Asp Asp Pro Val Tyr
            180                 185                 190

Gly Asn Ser Ala Lys Leu Val Tyr Ser Ile Leu Glu Gly Gln Pro Tyr
        195                 200                 205

Phe Ser Ile Glu Pro Glu Thr Ala Ile Ile Lys Thr Ala Leu Pro Asn
    210                 215                 220

Met Asp Arg Glu Ala Lys Glu Glu Tyr Leu Val Val Ile Gln Ala Lys
225                 230                 235                 240

Asp Met Gly Gly His Ser Gly Gly Leu Ser Gly Thr Thr Thr Leu Thr
                245                 250                 255

Val Thr Leu Thr Asp Val Asn Asp Asn Pro Pro Lys Phe Ala Gln Ser
            260                 265                 270

Leu Tyr His Phe Ser Val Pro Glu Asp Val Val Leu Gly Thr Ala Ile
        275                 280                 285

Gly Arg Val Lys Ala Asn Asp Gln Asp Ile Gly Glu Asn Ala Gln Ser
    290                 295                 300

Ser Tyr Asp Ile Ile Asp Gly Asp Gly Thr Ala Leu Phe Glu Ile Thr
305                 310                 315                 320

Ser Asp Ala Gln Ala Gln Asp Gly Ile Ile Arg Leu Arg Lys Pro Leu
                325                 330                 335

Asp Phe Glu Thr Lys Lys Ser Tyr Thr Leu Lys Asp Glu Ala Ala Asn
            340                 345                 350

Val His Ile Asp Pro Arg Phe Ser Gly Arg Gly Pro Phe Lys Asp Thr
        355                 360                 365

Ala Thr Val Lys Ile Val Val Glu Asp Ala Asp Glu Pro Pro Val Phe
    370                 375                 380

Ser Ser Pro Thr Tyr Leu Leu Glu Val His Glu Asn Ala Ala Leu Asn
385                 390                 395                 400

Ser Val Ile Gly Gln Val Thr Ala Arg Asp Pro Asp Ile Thr Ser Ser
                405                 410                 415

Pro Ile Arg Phe Ser Ile Asp Arg His Thr Asp Leu Glu Arg Gln Phe
            420                 425                 430

Asn Ile Asn Ala Asp Asp Gly Lys Ile Thr Leu Ala Thr Pro Leu Asp
        435                 440                 445

Arg Glu Leu Ser Val Trp His Asn Ile Thr Ile Ile Ala Thr Glu Ile
    450                 455                 460

Arg Asn His Ser Gln Ile Ser Arg Val Pro Val Ala Ile Lys Val Leu
465                 470                 475                 480

Asp Val Asn Asp Asn Ala Pro Glu Phe Ala Ser Glu Tyr Glu Ala Phe
                485                 490                 495

Leu Cys Glu Asn Gly Lys Pro Gly Gln Val Ile Gln Thr Val Ser Ala
            500                 505                 510

Met Asp Lys Asp Asp Pro Lys Asn Gly His Tyr Phe Leu Tyr Ser Leu
        515                 520                 525

Leu Pro Glu Met Val Asn Asn Pro Asn Phe Thr Ile Lys Lys Asn Glu
    530                 535                 540

Asp Asn Ser Leu Ser Ile Leu Ala Lys His Asn Gly Phe Asn Arg Gln
545                 550                 555                 560

Lys Gln Glu Val Tyr Leu Leu Pro Ile Ile Ile Ser Asp Ser Gly Asn
                565                 570                 575

Pro Pro Leu Ser Ser Thr Ser Thr Leu Thr Ile Arg Val Cys Gly Cys
            580                 585                 590

Ser Asn Asp Gly Val Val Gln Ser Cys Asn Val Glu Ala Tyr Val Leu
        595                 600                 605

Pro Ile Gly Leu Ser Met Gly Ala Leu Ile Ala Ile Leu Ala Cys Ile
    610                 615                 620

Ile Leu Leu Leu Val Ile Val Val Leu Phe Val Thr Leu Arg Arg His
625                 630                 635                 640

Gln Lys Asn Glu Pro Leu Ile Ile Lys Asp Asp Glu Asp Val Arg Glu
                645                 650                 655

Asn Ile Ile Arg Tyr Asp Asp Glu Gly Gly Gly Glu Glu Asp Thr Glu
            660                 665                 670

Ala Phe Asp Ile Ala Thr Leu Gln Asn Pro Asp Gly Ile Asn Gly Phe
        675                 680                 685

Leu Pro Arg Lys Asp Ile Lys Pro Asp Leu Gln Phe Met Pro Arg Gln
    690                 695                 700

Gly Leu Ala Pro Val Pro Asn Gly Val Asp Val Asp Glu Phe Ile Asn
705                 710                 715                 720

Val Arg Leu His Glu Ala Asp Asn Asp Pro Thr Ala Pro Pro Tyr Asp
                725                 730                 735

Ser Ile Gln Ile Tyr Gly Tyr Glu Gly Arg Gly Ser Val Ala Gly Ser
            740                 745                 750

Leu Ser Ser Leu Glu Ser Thr Thr Ser Asp Ser Asp Gln Asn Phe Asp
        755                 760                 765

Tyr Leu Ser Asp Trp Gly Pro Arg Phe Lys Arg Leu Gly Glu Leu Tyr
    770                 775                 780

Ser Val Gly Glu Ser Asp Lys Glu Thr
785                 790

(2) INFORMATION FOR SEQ ID NO:55:

(i) SEQUENCE CHARACTERISTICS:
(A) LENGTH: 730 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 2..730

(xi) SEQUENCE DESCRIPTION: SEQ ID NO:55:

G AAT TCG AGC TCG GTA CCC GGG GAT CCT CTA GAG TCG ACC TGC AGT 46
Asn Ser Ser Ser Val Pro Gly Asp Pro Leu Glu Ser Thr Cys Ser
1               5                   10                  15

GCT GAA GCC CTG CTC CTC CCT GCC GGC CTC AGC ACT GGG GCC TTG ATC 94
Ala Glu Ala Leu Leu Leu Pro Ala Gly Leu Ser Thr Gly Ala Leu Ile
                20                  25                  30

GCC ATC CTC CTC TGC ATC ATC ATT CTA CTG GTT ATA GTA GTA CTG TTT 142
Ala Ile Leu Leu Cys Ile Ile Ile Leu Leu Val Ile Val Val Leu Phe
            35                  40                  45

GCA GCT CTG AAA AGA CAG CGA AAA AAA GAG CCT CTG ATC TTG TCA AAA 190
Ala Ala Leu Lys Arg Gln Arg Lys Lys Glu Pro Leu Ile Leu Ser Lys
        50                  55                  60

GAA GAT ATC AGA GAC AAC ATT GTG AGC TAT AAC GAT GAG GGT GGT GGA 238
Glu Asp Ile Arg Asp Asn Ile Val Ser Tyr Asn Asp Glu Gly Gly Gly
    65                  70                  75

GAG GAG GAC ACC CAG GCC TTT GAT ATC GGC ACC CTG AGG AAT CCT GCA 286
Glu Glu Asp Thr Gln Ala Phe Asp Ile Gly Thr Leu Arg Asn Pro Ala
80                  85                  90                  95

GCC ATT GAG GAA AAA AAG CTC CGG CGA GAT ATT ATT CCA GAA ACG TTA 334
Ala Ile Glu Glu Lys Lys Leu Arg Arg Asp Ile Ile Pro Glu Thr Leu
                100                 105                 110

TTT ATT CCT CGG AGG ACT CCT ACA GCT CCA GAT AAC ACG GAC GTC CGG 382
Phe Ile Pro Arg Arg Thr Pro Thr Ala Pro Asp Asn Thr Asp Val Arg
            115                 120                 125

GAT TTC ATT AAT GAA AGG CTA AAA GAG CAT GAT CTT GAC CCC ACC GCA 430
Asp Phe Ile Asn Glu Arg Leu Lys Glu His Asp Leu Asp Pro Thr Ala
        130                 135                 140

CCC CCC TAC GAC TCA CTT GCA ACC TAT GCC TAT GAA GGA AAT GAT TCC 478
Pro Pro Tyr Asp Ser Leu Ala Thr Tyr Ala Tyr Glu Gly Asn Asp Ser
    145                 150                 155

ATT GCT GAA TCT CTG AGT TCA TTA GAA TCA GGT ACT ACT GAA GGA GAC 526
Ile Ala Glu Ser Leu Ser Ser Leu Glu Ser Gly Thr Thr Glu Gly Asp
160                 165                 170                 175

CAA AAC TAC GAT TAC CTC CGA GAA TGG GGC CCT CGG TTT AAT AAG CTA 574
Gln Asn Tyr Asp Tyr Leu Arg Glu Trp Gly Pro Arg Phe Asn Lys Leu
                180                 185                 190

GCA GAA ATG TAT GGT GGT GGG GAA AGT GAC AAA GAC TCT TAA CGT AGG 622
Ala Glu Met Tyr Gly Gly Gly Glu Ser Asp Lys Asp Ser * Arg Arg
            195                 200                 205

ATA TAT GTT CTG TTC AAA CAA GAG AAA GTA ACT CTA CCC ATG CTG TCT 670
Ile Tyr Val Leu Phe Lys Gln Glu Lys Val Thr Leu Pro Met Leu Ser
        210                 215                 220

CCA CTT CAC AAT ATT TGA TAT TCA GGA GCA TTT CCT GCA GTC AGC ACA 718
Pro Leu His Asn Ile * Tyr Ser Gly Ala Phe Pro Ala Val Ser Thr
    225               230                 235

ATT TTT TTC TCA 730
Ile Phe Phe Ser
240

(2) INFORMATION FOR SEQ ID NO:56:

(i) SEQUENCE CHARACTERISTICS:
(A) LENGTH: 241 amino acids
(B) TYPE: amino acid
(D) TOPOLOGY: linear

(ii) MOLECULE TYPE: protein

(xi) SEQUENCE DESCRIPTION: SEQ ID NO:56:

Asn Ser Ser Ser Val Pro Gly Asp Pro Leu Glu Ser Thr Cys Ser Ala
1               5                   10                  15

Glu Ala Leu Leu Leu Pro Ala Gly Leu Ser Thr Gly Ala Leu Ile Ala
            20                  25                  30

Ile Leu Leu Cys Ile Ile Ile Leu Leu Val Ile Val Val Leu Phe Ala
        35                  40                  45

Ala Leu Lys Arg Gln Arg Lys Lys Glu Pro Leu Ile Leu Ser Lys Glu
    50                  55                  60

Asp Ile Arg Asp Asn Ile Val Ser Tyr Asn Asp Glu Gly Gly Gly Glu
65                  70                  75                  80

Glu Asp Thr Gln Ala Phe Asp Ile Gly Thr Leu Arg Asn Pro Ala Ala
                85                  90                  95

Ile Glu Glu Lys Lys Leu Arg Arg Asp Ile Ile Pro Glu Thr Leu Phe
            100                 105                 110

Ile Pro Arg Arg Thr Pro Thr Ala Pro Asp Asn Thr Asp Val Arg Asp
        115                 120                 125

Phe Ile Asn Glu Arg Leu Lys Glu His Asp Leu Asp Pro Thr Ala Pro
    130                 135                 140

Pro Tyr Asp Ser Leu Ala Thr Tyr Ala Tyr Glu Gly Asn Asp Ser Ile
145                 150                 155                 160

Ala Glu Ser Leu Ser Ser Leu Glu Ser Gly Thr Thr Glu Gly Asp Gln
                165                 170                 175

Asn Tyr Asp Tyr Leu Arg Glu Trp Gly Pro Arg Phe Asn Lys Leu Ala
            180                 185                 190

Glu Met Tyr Gly Gly Gly Glu Ser Asp Lys Asp Ser Arg Arg Ile Tyr
        195                 200                 205

Val Leu Phe Lys Gln Glu Lys Val Thr Leu Pro Met Leu Ser Pro Leu
    210                 215                 220

His Asn Ile Tyr Ser Gly Ala Phe Pro Ala Val Ser Thr Ile Phe Phe
225                 230                 235                 240

Ser

(2) INFORMATION FOR SEQ ID NO:57:

    (i) SEQUENCE CHARACTERISTICS:
        (A) LENGTH: 2625 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:57:

CGGCAGCCCT GACGTGATGA GCTCAACCAG CAGAGACATT CCATCCCAAG AGAGGTCTGC      60

GTGACGCGTC CGGGAGGCCA CCCTCAGCAA GACCACCGTA CAGTTGGTGG AAGGGGTGAC     120

AGCTGCATTC TCCTGTGCCT ACCACGTAAC CAAAAATGAA GGAGAACTAC TGTTTACAAG     180

CCGCCCTGGT GTGCCTGGGC ATGCTGTGCC ACAGCCATGC CTTTGCCCCA GAGCGGCGGG     240

GGCACCTGCG GCCCTCCTTC CATGGGCACC ATGAGAAGGG CAAGGAGGGG CAGGTGCTAC     300

AGCGCTCCAA GCGTGGCTGG GTCTGGAACC AGTTCTTCGT GATAGAGGAG TACACCGGGC     360

CTGACCCCGT GCTTGTGGGC AGGCTTCATT CAGATATTGA CTCTGGTGAT GGGAACATTA     420

AATACATTCT CTCAGGGGAA GGAGCTGGAA CCATTTTTGT GATTGATGAC AAATCAGGGA     480

ACATTCATGC CACCAAGACG TTGGATCGAG AAGAGAGAGC CCAGTACACG TTGATGGCTC     540

AGGCGGTGGA CAGGGACACC AATCGGCCAC TGGAGCCACC GTCGGAATTC ATTGTCAAGG     600

TCCAGGACAT TAATGACAAC CCTCCGGAGT TCCTGCACGA GACCTATCAT GCCAACGTGC 660
CTGAGAGGTC CAATGTGGGA ACGTCAGTAA TCCAGGTGAC AGCTTCAGAT GCAGATGACC 720
CCACTTATGG AAATAGCGCC AAGTTAGTGT ACAGTATCCT CGAAGGACAA CCCTATTTTT 780
CGGTGGAAGC ACAGACAGGT ATCATCAGAA CAGCCCTACC CAACATGGAC AGGGAGGCCA 840
AGGAGGAGTA CCACGTGGTG ATCCAGGCCA AGGACATGGG TGGACATATG GGCGGACTCT 900
CAGGGACAAC CAAAGTGACG ATCACACTGA CCGATGTCAA TGACAACCCA CCAAAGTTTC 960
CGCAGAGGCT ATACCAGATG TCTGTGTCAG AAGCAGCCGT CCCTGGGGAG GAAGTAGGAA 1020
GAGTGAAAGC TAAAGATCCA GACATTGGAG AAAATGGCTT AGTCACATAC AATATTGTTG 1080
ATGGAGATGG TATGGAATCG TTTGAAATCA CAACGGACTA TGAAACACAG GAGGGGGTGA 1140
TAAAGCTGAA AAAGCCTGTA GATTTTGAAA CCGAAAGAGC CTATAGCTTG AAGGTAGAGG 1200
CAGCCAACGT GCACATCGAC CCGAAGTTTA TCAGCAATGG CCCTTTCAAG GACACTGTGA 1260
CCGTCAAGAT CTCAGTAGAA GATGCTGATG AGCCCCCTAT GTTCTTGGCC CCAAGTTACA 1320
TCCACGAAGT CCAAGAAAAT GCAGCTGCTG GCACCGTGGT TGGGAGAGTG CATGCCAAAG 1380
ACCCTGATGC TGCCAACAGC CCGATAAGGT ATTCCATCGA TCGTCACACT GACCTCGACA 1440
GATTTTTCAC TATTAATCCA GAGGATGGTT TTATTAAAAC TACAAAACCT CTGGATAGAG 1500
AGGAAACAGC CTGGCTCAAC ATCACTGTCT TTGCAGCAGA AATCCACAAT CGGCATCAGG 1560
AAGCCCAAGT CCCAGTGGCC ATTAGGGTCC TTGATGTCAA CGATAATGCT CCCAAGTTTG 1620
CTGCCCCTTA TGAAGGTTTC ATCTGTGAGA GTGATCAGAC CAAGCCACTT TCCAACCAGC 1680
CAATTGTTAC AATTAGTGCA GATGACAAGG ATGACACGGC CAATGGACCA AGATTTATCT 1740
TCAGCCTACC CCCTGAAATC ATTCACAATC CAAATTTCAC AGTCAGAGAC AACCGAGATA 1800
ACACAGCAGG CGTGTACGCC CGGCGTGGAG GGTTCAGTCG GCAGAAGCAG GACTTGTACC 1860
TTCTGCCCAT AGTGATCAGC GATGGCGGCA TCCCGCCCAT GAGTAGCACC AACACCCTCA 1920
CCATCAAAGT CTGCGGGTGC GACGTGAACG GGGCACTGCT CTCCTGCAAC GCAGAGGCCT 1980
ACATTCTGAA CGCCGGCCTG AGCACAGGCG CCCTGATCGC CATCCTCGCC TGCATCGTCA 2040
TTCTCCTGGT CATTGTAGTA TTGTTTGTGA CCCTGAGAAG GCAAAAGAAA GAACCACTCA 2100
TTGTCTTTGA GGAAGAAGAT GTCCGTGAGA ACATCATTAC TTATGATGAT GAAGGGGGTG 2160
GGGAAGAAGA CACAGAAGCC TTTGATATTG CCACCCTCCA GAATCCTGAT GGTATCAATG 2220
GATTTATCCC CCGCAAAGAC ATCAAACCTG AGTATCAGTA CATGCCTAGA CCTGGGCTCC 2280
GGCCAGCGCC CAACAGCGTG GATGTCGATG ACTTCATCAA CACGAGAATA CAGGAGGCAG 2340
ACAATGACCC CACGGCTCCT CCTTATGACT CCATTCAAAT CTACGGTTAT GAAGGCAGGG 2400
GCTCAGTGGC CGGGTCCCTG AGCTCCCTAG AGTCGGCCAC CACAGATTCA GACTTGGACT 2460
ATGATTATCT ACAGAACTGG GGACCTCGTT TTAAGAAACT AGCAGATTTG TATGGTTCCA 2520
AAGACACTTT TGATGACGAT TCTTAACAAT AACGATACAA ATTTGGCCTT AAGAACTGTG 2580
TCTGGCGTTC TCAAGAATCT AGAAGATGTG TAACAGGTAT TTTTT 2625

(2) INFORMATION FOR SEQ ID NO:58:

(i) SEQUENCE CHARACTERISTICS:
(A) LENGTH: 796 amino acids
(B) TYPE: amino acid
(D) TOPOLOGY: linear

(ii) MOLECULE TYPE: protein

(xi) SEQUENCE DESCRIPTION: SEQ ID NO:58:

Met Lys Glu Asn Tyr Cys Leu Gln Ala Ala Leu Val Cys Leu Gly Met
1               5                   10                  15

Leu Cys His Ser His Ala Phe Ala Pro Glu Arg Arg Gly His Leu Arg
            20                  25                  30

Pro Ser Phe His Gly His His Glu Lys Gly Lys Glu Gly Gln Val Leu
        35                  40                  45

Gln Arg Ser Lys Arg Gly Trp Val Trp Asn Gln Phe Phe Val Ile Glu
    50                  55                  60

Glu Tyr Thr Gly Pro Asp Pro Val Leu Val Gly Arg Leu His Ser Asp
65                  70                  75                  80

Ile Asp Ser Gly Asp Gly Asn Ile Lys Tyr Ile Leu Ser Gly Glu Gly
                85                  90                  95

Ala Gly Thr Ile Phe Val Ile Asp Asp Lys Ser Gly Asn Ile His Ala
            100                 105                 110

Thr Lys Thr Leu Asp Arg Glu Glu Arg Ala Gln Tyr Thr Leu Met Ala
        115                 120                 125

Gln Ala Val Asp Arg Asp Thr Asn Arg Pro Leu Glu Pro Pro Ser Glu
    130                 135                 140

Phe Ile Val Lys Val Gln Asp Ile Asn Asp Asn Pro Pro Glu Phe Leu
145                 150                 155                 160

His Glu Thr Tyr His Ala Asn Val Pro Glu Arg Ser Asn Val Gly Thr
                165                 170                 175

Ser Val Ile Gln Val Thr Ala Ser Asp Ala Asp Asp Pro Thr Tyr Gly
            180                 185                 190

Asn Ser Ala Lys Leu Val Tyr Ser Ile Leu Glu Gly Gln Pro Tyr Phe
        195                 200                 205

Ser Val Glu Ala Gln Thr Gly Ile Ile Arg Thr Ala Leu Pro Asn Met
    210                 215                 220

Asp Arg Glu Ala Lys Glu Glu Tyr His Val Val Ile Gln Ala Lys Asp
225                 230                 235                 240

Met Gly Gly His Met Gly Gly Leu Ser Gly Thr Thr Lys Val Thr Ile
                245                 250                 255

Thr Leu Thr Asp Val Asn Asp Asn Pro Pro Lys Phe Pro Gln Arg Leu
            260                 265                 270

Tyr Gln Met Ser Val Ser Glu Ala Ala Val Pro Gly Glu Glu Val Gly
        275                 280                 285

Arg Val Lys Ala Lys Asp Pro Asp Ile Gly Glu Asn Gly Leu Val Thr
    290                 295                 300

Tyr Asn Ile Val Asp Gly Asp Gly Met Glu Ser Phe Glu Ile Thr Thr
305                 310                 315                 320

Asp Tyr Glu Thr Gln Glu Gly Val Ile Lys Leu Lys Lys Pro Val Asp
                325                 330                 335

Phe Glu Thr Glu Arg Ala Tyr Ser Leu Lys Val Glu Ala Ala Asn Val
            340                 345                 350

His Ile Asp Pro Lys Phe Ile Ser Asn Gly Pro Phe Lys Asp Thr Val
        355                 360                 365

Thr Val Lys Ile Ser Val Glu Asp Ala Asp Glu Pro Pro Met Phe Leu
    370                 375                 380

Ala Pro Ser Tyr Ile His Glu Val Gln Glu Asn Ala Ala Ala Gly Thr
385                 390                 395                 400

Val Val Gly Arg Val His Ala Lys Asp Pro Asp Ala Ala Asn Ser Pro
                405                 410                 415

Ile Arg Tyr Ser Ile Asp Arg His Thr Asp Leu Asp Arg Phe Phe Thr
            420                 425                 430

Ile Asn Pro Glu Asp Gly Phe Ile Lys Thr Thr Lys Pro Leu Asp Arg
        435                 440                 445

Glu Glu Thr Ala Trp Leu Asn Ile Thr Val Phe Ala Ala Glu Ile His
    450                 455                 460

Asn Arg His Gln Glu Ala Gln Val Pro Val Ala Ile Arg Val Leu Asp
465                 470                 475                 480

Val Asn Asp Asn Ala Pro Lys Phe Ala Ala Pro Tyr Glu Gly Phe Ile
                485                 490                 495

Cys Glu Ser Asp Gln Thr Lys Pro Leu Ser Asn Gln Pro Ile Val Thr
            500                 505                 510

Ile Ser Ala Asp Asp Lys Asp Asp Thr Ala Asn Gly Pro Arg Phe Ile
        515                 520                 525

Phe Ser Leu Pro Pro Glu Ile Ile His Asn Pro Asn Phe Thr Val Arg
    530                 535                 540

Asp Asn Arg Asp Asn Thr Ala Gly Val Tyr Ala Arg Arg Gly Gly Phe
545                 550                 555                 560

Ser Arg Gln Lys Gln Asp Leu Tyr Leu Leu Pro Ile Val Ile Ser Asp
                565                 570                 575

Gly Gly Ile Pro Pro Met Ser Ser Thr Asn Thr Leu Thr Ile Lys Val
            580                 585                 590

Cys Gly Cys Asp Val Asn Gly Ala Leu Leu Ser Cys Asn Ala Glu Ala
        595                 600                 605

Tyr Ile Leu Asn Ala Gly Leu Ser Thr Gly Ala Leu Ile Ala Ile Leu
    610                 615                 620

Ala Cys Ile Val Ile Leu Leu Val Ile Val Val Leu Phe Val Thr Leu
625                 630                 635                 640

Arg Arg Gln Lys Lys Glu Pro Leu Ile Val Phe Glu Glu Glu Asp Val
                645                 650                 655

Arg Glu Asn Ile Ile Thr Tyr Asp Asp Glu Gly Gly Gly Glu Glu Asp
            660                 665                 670

Thr Glu Ala Phe Asp Ile Ala Thr Leu Gln Asn Pro Asp Gly Ile Asn
        675                 680                 685

Gly Phe Ile Pro Arg Lys Asp Ile Lys Pro Glu Tyr Gln Tyr Met Pro
    690                 695                 700

Arg Pro Gly Leu Arg Pro Ala Pro Asn Ser Val Asp Val Asp Asp Phe
705                 710                 715                 720

Ile Asn Thr Arg Ile Gln Glu Ala Asp Asn Asp Pro Thr Ala Pro Pro
                725                 730                 735

Tyr Asp Ser Ile Gln Ile Tyr Gly Tyr Glu Gly Arg Gly Ser Val Ala
            740                 745                 750

Gly Ser Leu Ser Ser Leu Glu Ser Ala Thr Thr Asp Ser Asp Leu Asp
        755                 760                 765

Tyr Asp Tyr Leu Gln Asn Trp Gly Pro Arg Phe Lys Lys Leu Ala Asp
    770                 775                 780

Leu Tyr Gly Ser Lys Asp Thr Phe Asp Asp Asp Ser
785                 790                 795

(2) INFORMATION FOR SEQ ID NO:59:

     (i) SEQUENCE CHARACTERISTICS:
          (A) LENGTH: 2521 base pairs
          (B) TYPE: nucleic acid
          (C) STRANDEDNESS: single
          (D) TOPOLOGY: linear

    (ii) MOLECULE TYPE: cDNA

    (xi) SEQUENCE DESCRIPTION: SEQ ID NO:59:

CGGTGGAGGC CACAGACACC TCAAACCTGG ATTCCACAAT TCTACGTTAA GTGTTGGAGT      60

TTTTATTACT CTGCTGTAGG AAAGCCTTTG CCAATGCTTA CAAGGAACTG TTTATCCCTG     120

CTTCTCTGGG TTCTGTTTGA TGGAGGTCTC CTAACACCAC TACAACCACA GCCACAGCAG     180

ACTTTAGCCA CAGAGCCAAG AGAAAATGTT ATCCATCTGC CAGGACAACG GTCACATTTC     240

CAACGTGTTA AACGTGGCTG GGTATGGAAT CAATTTTTTG TGCTGGAAGA ATACGTGGGC     300

TCCGAGCCTC AGTATGTGGG AAAGCTCCAT TCCGACTTAG ACAAGGGAGA GGGCACTGTG     360

AAATACACCC TCTCAGGAGA TGGCGCTGGC ACCGTTTTTA CCATTGATGA AACCACAGGG     420

GACATTCATG CAATAAGGAG CCTAGATAGA GAAGAGAAAC CTTTCTACAC TCTTCGTGCT     480

CAGGCTGTGG ACATAGAAAC CAGAAAGCCC CTGGAGCCTG AATCAGAATT CATCATCAAA     540

GTGCAGGATA TTAATGATAA TGAGCCAAAG TTTTTGGATG GACCTTATGT TGCTACTGTT     600

CCAGAAATGT CTCCTGTGGG TGCATATGTA CTCCAGGTCA AGGCCACAGA TGCAGATGAC     660

CCGACCTATG GAAACAGTGC CAGAGTCGTT TACAGCATTC TTCAGGGACA ACCTTATTTC     720

TCTATTGATC CCAAGACAGG TGTTATTAGA ACAGCTTTGC CAAACATGGA CAGAGAAGTC     780

AAAGAACAAT ATCAAGTACT CATCCAAGCC AAGGATATGG GAGGACAGCT TGGAGGATTA 840
GCCGGAACAA CAATAGTCAA CATCACTCTC ACCGATGTCA ATGACAATCC ACCTCGATTC 900
CCCAAAAGCA TCTTCCACTT GAAAGTTCCT GAGTCTTCCC CTATTGGTTC AGCTATTGGA 960
AGAATAAGAG CTGTGGATCC TGATTTTGGA CAAAATGCAG AAATTGAATA CAATATTGTT 1020
CCAGGAGATG GGGGAAATTT GTTTGACATC GTCACAGATG AGGATACACA AGAGGGAGTC 1080
ATCAAATTGA AAAAGCCTTT AGATTTTGAA ACAAAGAAGG CATACACTTT CAAAGTTGAG 1140
GCTTCCAACC TTCACCTTGA CCACCGGTTT CACTCGGCGG GCCCTTTCAA AGACACAGCT 1200
ACGGTGAAGA TCAGCGTGCT GGACGTAGAT GAGCCACCGG TTTTCAGCAA GCCGCTCTAC 1260
ACCATGGAGG TTTATGAAGA CACTCCGGTA GGGACCATCA TTGGCGCTGT CACTGCTCAA 1320
GACCTGGATG TAGGCAGCGG TGCTGTTAGG TACTTCATAG ATTGGAAGAG TGATGGGGAC 1380
AGCTACTTTA CAATAGATGG AAATGAAGGA ACCATCGCCA CTAATGAATT ACTAGACAGA 1440
GAAAGCACTG CGCAGTATAA TTTCTCCATA ATTGCGAGTA AAGTTAGTAA CCCTTTATTG 1500
ACCAGCAAAG TCAATATACT GATTAATGTC TTAGATGTAA ATGAATTTCC TCCAGAAATA 1560
TCTGTGCCAT ATGAGACAGC CGTGTGTGAA AATGCCAAGC CAGGACAGAT AATTCAGATA 1620
GTCAGTGCTG CAGACCGAGA TCTTTCACCT GCTGGGCAAC AATTCTCCTT TAGATTATCA 1680
CCTGAGGCTG CTATCAAACC AAATTTTACA GTTCGTGACT TCAGAAACAA CACAGCGGGG 1740
ATTGAAACCC GAAGAAATGG ATACAGCCGC AGGCAGCAAG AGTTGTATTT CCTCCCTGTT 1800
GTAATAGAAG ACAGCAGCTA CCCTGTCCAG AGCAGCACAA ACACAATGAC TATTCGAGTC 1860
TGTAGATGTG ACTCTGATGG CACCATCCTG TCTTGTAATG TGGAAGCAAT TTTTCTACCT 1920
GTAGGACTTA GCACTGGGGC GTTGATTGCA ATTCTACTAT GCATTGTTAT ACTCTTAGCC 1980
ATAGTTGTAC TGTATGTAGC ACTGCGAAGG CAGAAGAAAA AGCACACCCT GATGACCTCT 2040
AAAGAAGACA TCAGAGACAA CGTCATCCAT TACGATGATG AAGGAGGTGG GGAGGAAGAT 2100
ACCCAGGCTT TCGACATCGG GGCTCTGAGA AACCCAAAAG TGATTGAGGA GAACAAAATT 2160
CGCAGGGATA TAAAACCAGA CTCTCTCTGT TTACCTCGTC AGAGACCACC CATGGAAGAT 2220
AACACAGACA TAAGGGATTT CATTCATCAA AGGCTACAGG AAAATGATGT AGATCCAACT 2280
GCCCCACCAA TCGATTCACT GGCCACATAT GCCTACGAAG GGAGTGGGTC CGTGGCAGAG 2340
TCCCTCAGCT CTATAGACTC TCTCACCACA GAAGCCGACC AGGACTATGA CTATCTGACA 2400
GACTGGGGAC CCCGCTTTAA AGTCTTGGCA GACATGTTTG GCGAAGAAGA GAGTTATAAC 2460
CCTGATAAAG TCACTTAAGG GAGTCGTGGA GGCTAAAATA CAACCGAGAG GGGAGATTTT 2520
T 2521

(2) INFORMATION FOR SEQ ID NO:60:

(i) SEQUENCE CHARACTERISTICS:
(A) LENGTH: 794 amino acids
(B) TYPE: amino acid
(D) TOPOLOGY: linear

(ii) MOLECULE TYPE: protein

(xi) SEQUENCE DESCRIPTION: SEQ ID NO:60:

Met Leu Thr Arg Asn Cys Leu Ser Leu Leu Leu Trp Val Leu Phe Asp
1               5                   10                  15

Gly Gly Leu Leu Thr Pro Leu Gln Pro Gln Pro Gln Gln Thr Leu Ala
            20                  25                  30

Thr Glu Pro Arg Glu Asn Val Ile His Leu Pro Gly Gln Arg Ser His
        35                  40                  45

Phe Gln Arg Val Lys Arg Gly Trp Val Trp Asn Gln Phe Phe Val Leu
    50                  55                  60

Glu Glu Tyr Val Gly Ser Glu Pro Gln Tyr Val Gly Lys Leu His Ser
65                  70                  75                  80

Asp Leu Asp Lys Gly Glu Gly Thr Val Lys Tyr Thr Leu Ser Gly Asp
                85                  90                  95

Gly Ala Gly Thr Val Phe Thr Ile Asp Glu Thr Thr Gly Asp Ile His
            100                 105                 110

Ala Ile Arg Ser Leu Asp Arg Glu Glu Lys Pro Phe Tyr Thr Leu Arg
        115                 120                 125

Ala Gln Ala Val Asp Ile Glu Thr Arg Lys Pro Leu Glu Pro Glu Ser
    130                 135                 140

Glu Phe Ile Ile Lys Val Gln Asp Ile Asn Asp Asn Glu Pro Lys Phe
145                 150                 155                 160

Leu Asp Gly Pro Tyr Val Ala Thr Val Pro Glu Met Ser Pro Val Gly
                165                 170                 175

Ala Tyr Val Leu Gln Val Lys Ala Thr Asp Ala Asp Asp Pro Thr Tyr
            180                 185                 190

Gly Asn Ser Ala Arg Val Val Tyr Ser Ile Leu Gln Gly Gln Pro Tyr
        195                 200                 205

Phe Ser Ile Asp Pro Lys Thr Gly Val Ile Arg Thr Ala Leu Pro Asn
    210                 215                 220

Met Asp Arg Glu Val Lys Glu Gln Tyr Gln Val Leu Ile Gln Ala Lys
225                 230                 235                 240

Asp Met Gly Gly Gln Leu Gly Gly Leu Ala Gly Thr Thr Ile Val Asn
                245                 250                 255

Ile Thr Leu Thr Asp Val Asn Asp Asn Pro Pro Arg Phe Pro Lys Ser
            260                 265                 270

Ile Phe His Leu Lys Val Pro Glu Ser Ser Pro Ile Gly Ser Gly Ile
        275                 280                 285

Gly Arg Ile Arg Ala Val Asp Pro Asp Phe Gly Gln Asn Ala Glu Ile
    290                 295                 300

Glu Tyr Asn Ile Val Pro Gly Asp Gly Gly Asn Leu Phe Asp Ile Val
305                 310                 315                 320

Thr Asp Glu Asp Thr Gln Glu Gly Val Ile Lys Leu Lys Lys Pro Leu
                325                 330                 335

Asp Phe Glu Thr Lys Lys Ala Tyr Thr Phe Lys Val Glu Ala Ser Asn
            340                 345                 350

Leu His Leu Asp His Arg Phe His Ser Ala Gly Pro Phe Lys Asp Thr
        355                 360                 365

Ala Thr Val Lys Ile Ser Val Leu Asp Val Asp Glu Pro Pro Val Phe
    370                 375                 380

Ser Lys Pro Leu Tyr Thr Met Glu Val Tyr Glu Asp Thr Pro Val Gly
385                 390                 395                 400

Thr Ile Ile Gly Ala Val Thr Ala Gln Asp Leu Asp Val Gly Ser Gly
                405                 410                 415

Ala Val Arg Tyr Phe Ile Asp Trp Lys Ser Asp Gly Asp Ser Tyr Phe
            420                 425                 430

Thr Ile Asp Gly Asn Glu Gly Thr Ile Ala Thr Asn Glu Leu Leu Asp
        435                 440                 445

Arg Glu Ser Thr Ala Gln Tyr Asn Phe Ser Ile Ile Ala Ser Lys Val
    450                 455                 460

Ser Asn Pro Leu Leu Thr Ser Lys Val Asn Ile Leu Ile Asn Val Leu
465                 470                 475                 480

Asp Val Asn Glu Phe Pro Pro Glu Ile Ser Val Pro Tyr Glu Thr Ala
                485                 490                 495

Val Cys Glu Asn Ala Lys Pro Gly Gln Ile Ile Gln Ile Val Ser Ala
            500                 505                 510

Ala Asp Arg Asp Leu Ser Pro Ala Gly Gln Gln Phe Ser Phe Arg Leu
        515                 520                 525

Ser Pro Glu Ala Ala Ile Lys Pro Asn Phe Thr Val Arg Asp Phe Arg
    530                 535                 540

Asn Asn Thr Ala Gly Ile Glu Thr Arg Arg Asn Gly Tyr Ser Arg Arg
545                 550                 555                 560

Gln Gln Glu Leu Tyr Phe Leu Pro Val Val Ile Glu Asp Ser Ser Tyr
                565                 570                 575

Pro Val Gln Ser Ser Thr Asn Thr Met Thr Ile Arg Val Cys Arg Cys
            580                 585                 590

Asp Ser Asp Gly Thr Ile Leu Ser Cys Asn Val Glu Ala Ile Phe Leu
        595                 600                 605

Pro Val Gly Leu Ser Thr Gly Ala Leu Ile Ala Ile Leu Leu Cys Ile
    610                 615                 620

Val Ile Leu Leu Ala Ile Val Val Leu Tyr Val Ala Leu Arg Arg Gln
625                 630                 635                 640

Lys Lys Lys His Thr Leu Met Thr Ser Lys Glu Asp Ile Arg Asp Asn
                645                 650                 655

Val Ile His Tyr Asp Asp Glu Gly Gly Gly Glu Glu Asp Thr Gln Ala
            660                 665                 670

Phe Asp Ile Gly Ala Leu Arg Asn Pro Lys Val Ile Glu Glu Asn Lys
        675                 680                 685

Ile Arg Arg Asp Ile Lys Pro Asp Ser Leu Cys Leu Pro Arg Gln Arg
    690                 695                 700

Pro Pro Met Glu Asp Asn Thr Asp Ile Arg Asp Phe Ile His Gln Arg
705                 710                 715                 720

Leu Gln Glu Asn Asp Val Asp Pro Thr Ala Pro Pro Ile Asp Ser Leu
                725                 730                 735

Ala Thr Tyr Ala Tyr Glu Gly Ser Gly Ser Val Ala Glu Ser Leu Ser
            740                 745                 750

Ser Ile Asp Ser Leu Thr Thr Glu Ala Asp Gln Asp Tyr Asp Tyr Leu
        755                 760                 765

Thr Asp Trp Gly Pro Arg Phe Lys Val Val Ala Asp Met Phe Gly Glu
    770                 775                 780

Glu Glu Ser Tyr Asn Pro Asp Lys Val Thr
785                 790

(2) INFORMATION FOR SEQ ID NO:61:

(i) SEQUENCE CHARACTERISTICS:
(A) LENGTH: 2690 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:61:

CTTCAAGGTT TTGCTGACTC AGTCTGGTAG TCAGAGTCTG CAGGAGAAGA CAGTTCAAGG 60

CAGGGCCTGG AGGATTGGAT CAGTTTAGGG ACAGGTCAAA GGCTGGCTTA GAGACCTTAG 120

AGGCAGGTTG CTTGGGTCGT TGAATGCTAG TCTGGTCCTG AGAGCCCTTT TCTCTGGCAA 180

CTGTGGACTC AGAGCTAACC AATTGTAGTT GGCAGTGGGG GTGAAGGGTG ATCCAGAGGC 240

CTGAGCTGCA GAGGGCACAA GAGAGAAAAG ATGTCTTAGA AAGAGCTTTG AGAACATGCC 300

TTGGCTGCTG GCAGGGACCT TGGATGGGGT AGTCTACACC CGGAAGTGCC TGCCTGCCAT 360

CCTCTAGTGG CTGCCTTGCA AAATATGCTC AGTGCAGCCG CGTGCATGAA TGAAAACGCC 420

GCCGGGCGCT TCTAGTCGGA CAAAATGCAG CCGAGAACTC CGCTCGTTCT GTGCGTTCTC 480

CTGTCCCAGG TGCTGCTGCT AACATCTGCA GAAGATTTGG ACTGCACTCC TGGATTTCAG 540

CAGAAAGTGT TCCATATCAA TCAGCCAGCT GAATTCATTG AGGACCAGTC AATTCTAAAC 600

TTGACCTTCA GTGACTGTAA GGGAAACGAC AAGCTACGCT ATGAGGTCTC GAGCCCATAC 660

TTCAAGGTGA ACAGCGATGG CGGCTTAGTT GCTCTGAGAA ACATAACTGC AGTGGGCAAA 720

ACTCTGTTCG TCCATGCACG GACCCCCCAT GCGGAAGATA TGGCAGAACT CGTGATTGTC 780

GGGGGGAAAG ACATCCAGGG CTCCTTGCAG GATATATTTA AATTTGCAAG AACTTCTCCT 840

GTCCCAAGAC AAAAGAGGTC CATTGTGGTA TCTCCCATTT TAATTCCAGA GAATCAGAGA 900

CAGCCTTTCC CAAGAGATGT TGGCAAGGTA GTCGATAGTG ACAGGCCAGA AAGGTCCAAG 960
TTCCGGCTCA CTGGAAAGGG AGTGGATCAA GAGCCTAAAG GAATTTTCAG AATCAATGAG 1020
AACACAGGGA GCGTCTCCGT GACACGGACC TTGGACAGAG AAGTAATCGC TGTTTATCAA 1080
CTATTTGTGG AGACCACTGA TGTCAATGGC AAAACTCTCG AGGGGCCGGT GCCTCTGGAA 1140
GTCATTGTGA TTGATCAGAA TGACAACCGA CCGATCTTTC GGGAAGGCCC CTACATCGGC 1200
CACGTCATGG AAGGGTCACC CACAGGCACC ACAGTGATGC GGATGACAGC CTTTGATGCA 1260
GATGACCCAG CCACCGATAA TGCCCTCCTG CGGTATAATA TCCGTCAACA GACGCCTGAC 1320
AAGCCATCTC CCAACATGTT CTACATCGAT CCTGAGAAAG GAGACATTGT CACTGTTGTG 1380
TCACCTGCGC TGCTGGACCG AGAGACTCTG GAAAATCCCA AGTATGAACT GATCATCGAG 1440
GCTCAAGATA TGGCTGGACT GGATGTTGGA TTAACAGGCA CGGCCACAGC CACGATCATG 1500
ATCGATGACA AAAATGATCA CTCACCAAAA TTCACCAAGA AAGAGTTTCA AGCCACAGTC 1560
GAGGAAGGAG CTGTGGGAGT TATTGTCAAT TTGACAGTTG AAGATAAGGA TGACCCCACC 1620
ACAGGTGCAT GGAGGGCTGC CTACACCATC ATCAACGGAA ACCCCGGGCA GAGCTTTGAA 1680
ATCCACACCA ACCCTCAAAC CAACGAAGGG ATGCTTTCTG TTGTCAAACC ATTGGACTAT 1740
GAAATTTCTG CCTTCCACAC CCTGCTGATC AAAGTGGAAA ATGAAGACCC ACTCGTACCC 1800
GACGTCTCCT ACGGCCCCAG CTCCACAGCC ACCGTCCACA TCACTGTCCT GGATGTCAAC 1860
GAGGGCCCAG TCTTCTACCC AGACCCCATG ATGGTGACCA GGCAGGAGGA CCTCTCTGTG 1920
GGCAGCGTGC TGCTGACAGT GAATGCCACG GACCCCGACT CCCTGCAGCA TCAAACCATC 1980
AGGTATTCTG TTTACAAGGA CCCAGCAGGT TGGCTGAATA TTAACCCCAT CAATGGGACT 2040
GTTGACACCA CAGCTGTGCT GGACCGTGAG TCCCCATTTG TCGACAACAG CGTGTACACT 2100
GCTCTCTTCC TGGCAATTGA CAGTGGCAAC CCTCCCGCTA CGGGCACTGG GACTTTGCTG 2160
ATAACCCTGG AGGACGTGAA TGACAATGCC CCGTTCATTT ACCCCACAGT AGCTGAAGTC 2220
TGTGATGATG CCAAAAACCT CAGTGTAGTC ATTTTGGGAG CATCAGATAA GGATCTTCAC 2280
CCGAATACAG ATCCTTTCAA ATTTGAAATC CACAAACAAG CTGTTCCTGA TAAAGTCTGG 2340
AAGATCTCCA AGATCAACAA TACACACGCC CTGGTAAGCC TTCTTCAAAA TCTGAACAAA 2400
GCAAACTACA ACCTGCCCAT CATGGTGACA GATTCAGGGA AACCACCCAT GACGAATATC 2460
ACAGATCTCA GGGTACAAGT GTGCTCCTGC AGGAATTCCA AAGTGGACTG CAACGCGGCG 2520
GGGGCCCTGC GCTTCAGCCT GCCCTCAGTC CTGCTCCTCA GCCTCTTCAG CTTAGCTTGT 2580
CTGTGAGAAC TCCTGACGTC TGAAGCTTGA CTCCCAAGTT TCCATAGCAA CAGGAAAAAA 2640
AAAAAATCTA TCCAAATCTG AAGATTGCGG TTTACAGCTA TCGAACTTCG 2690

(2) INFORMATION FOR SEQ ID NO:62:

(i) SEQUENCE CHARACTERISTICS:
(A) LENGTH: 713 amino acids
(B) TYPE: amino acid
(D) TOPOLOGY: linear

(ii) MOLECULE TYPE: protein

(xi) SEQUENCE DESCRIPTION: SEQ ID NO:62:

Met Gln Pro Arg Thr Pro Leu Val Leu Cys Val Leu Leu Ser Gln Val
1               5                   10                  15

Leu Leu Leu Thr Ser Ala Glu Asp Leu Asp Cys Thr Pro Gly Phe Gln
            20                  25                  30

Gln Lys Val Phe His Ile Asn Gln Pro Ala Glu Phe Ile Glu Asp Gln
        35                  40                  45

Ser Ile Leu Asn Leu Thr Phe Ser Asp Cys Lys Gly Asn Asp Lys Leu
    50                  55                  60

Arg Tyr Glu Val Ser Ser Pro Tyr Phe Lys Val Asn Ser Asp Gly Gly
65                  70                  75                  80

Leu Val Ala Leu Arg Asn Ile Thr Ala Val Gly Lys Thr Leu Phe Val
                85                  90                  95

His Ala Arg Thr Pro His Ala Glu Asp Met Ala Glu Leu Val Ile Val
            100                 105                 110

Gly Gly Lys Asp Ile Gln Gly Ser Leu Gln Asp Ile Phe Lys Phe Ala
        115                 120                 125

Arg Thr Ser Pro Val Pro Arg Gln Lys Arg Ser Ile Val Val Ser Pro
    130                 135                 140

Ile Leu Ile Pro Glu Asn Gln Arg Gln Pro Phe Pro Arg Asp Val Gly
145                 150                 155                 160

Lys Val Val Asp Ser Asp Arg Pro Glu Arg Ser Lys Phe Arg Leu Thr
                165                 170                 175

Gly Lys Gly Val Asp Gln Glu Pro Lys Gly Ile Phe Arg Ile Asn Glu
            180                 185                 190

Asn Thr Gly Ser Val Ser Val Thr Arg Thr Leu Asp Arg Glu Val Ile
        195                 200                 205

Ala Val Tyr Gln Leu Phe Val Glu Thr Thr Asp Val Asn Gly Lys Thr
    210                 215                 220

Leu Glu Gly Pro Val Pro Leu Glu Val Ile Val Ile Asp Gln Asn Asp
225                 230                 235                 240

Asn Arg Pro Ile Phe Arg Glu Gly Pro Tyr Ile Gly His Val Met Glu
                245                 250                 255

Gly Ser Pro Thr Gly Thr Thr Val Met Arg Met Thr Ala Phe Asp Ala
            260                 265                 270

Asp Asp Pro Ala Thr Asp Asn Ala Leu Leu Arg Tyr Asn Ile Arg Gln
        275                 280                 285

Gln Thr Pro Asp Lys Pro Ser Pro Asn Met Phe Tyr Ile Asp Pro Glu
    290                 295                 300

Lys Gly Asp Ile Val Thr Val Val Ser Pro Ala Leu Leu Asp Arg Glu
305                 310                 315                 320

Thr Leu Glu Asn Pro Lys Tyr Glu Leu Ile Ile Glu Ala Gln Asp Met
                325                 330                 335

Ala Gly Leu Asp Val Gly Leu Thr Gly Thr Ala Thr Ala Thr Ile Met
            340                 345                 350

Ile Asp Asp Lys Asn Asp His Ser Pro Lys Phe Thr Lys Lys Glu Phe
        355                 360                 365

Gln Ala Thr Val Glu Glu Gly Ala Val Gly Val Ile Val Asn Leu Thr
    370                 375                 380

Val Glu Asp Lys Asp Asp Pro Thr Thr Gly Ala Trp Arg Ala Ala Tyr
385                 390                 395                 400

Thr Ile Ile Asn Gly Asn Pro Gly Gln Ser Phe Glu Ile His Thr Asn
                405                 410                 415

Pro Gln Thr Asn Glu Gly Met Leu Ser Val Val Lys Pro Leu Asp Tyr
            420                 425                 430

Glu Ile Ser Ala Phe His Thr Leu Leu Ile Lys Val Glu Asn Glu Asp
        435                 440                 445

Pro Leu Val Pro Asp Val Ser Tyr Gly Pro Ser Ser Thr Ala Thr Val
    450                 455                 460

His Ile Thr Val Leu Asp Val Asn Glu Gly Pro Val Phe Tyr Pro Asp
465                 470                 475                 480

Pro Met Met Val Thr Arg Gln Glu Asp Leu Ser Val Gly Ser Val Leu
                485                 490                 495

Leu Thr Val Asn Ala Thr Asp Pro Asp Ser Leu Gln His Gln Thr Ile
            500                 505                 510

Arg Tyr Ser Val Tyr Lys Asp Pro Ala Gly Trp Leu Asn Ile Asn Pro
        515                 520                 525

Ile Asn Gly Thr Val Asp Thr Thr Ala Val Leu Asp Arg Glu Ser Pro
    530                 535                 540

Phe Val Asp Asn Ser Val Tyr Thr Ala Leu Phe Leu Ala Ile Asp Ser
545                 550                 555                 560

Gly Asn Pro Pro Ala Thr Gly Thr Gly Thr Leu Leu Ile Thr Leu Glu
                565                 570                 575

Asp Val Asn Asp Asn Ala Pro Phe Ile Tyr Pro Thr Val Ala Glu Val
            580                 585                 590

Cys Asp Asp Ala Lys Asn Leu Ser Val Val Ile Leu Gly Ala Ser Asp
        595                 600                 605

Lys Asp Leu His Pro Asn Thr Asp Pro Phe Lys Phe Glu Ile His Lys
    610                 615                 620

Gln Ala Val Pro Asp Lys Val Trp Lys Ile Ser Lys Ile Asn Asn Thr
625                 630                 635                 640

His Ala Leu Val Ser Leu Leu Gln Asn Leu Asn Lys Ala Asn Tyr Asn
                645                 650                 655

Leu Pro Ile Met Val Thr Asp Ser Gly Lys Pro Pro Met Thr Asn Ile
            660                 665                 670

Thr Asp Leu Arg Val Gln Val Cys Ser Cys Arg Asn Ser Lys Val Asp
      675                 680                 685

Cys Asn Ala Ala Gly Ala Leu Arg Phe Ser Leu Pro Ser Val Ile Leu
    690                 695                 700

Leu Ser Leu Phe Ser Leu Ala Cys Leu
705                 710